# EUROPEAN PATENT SPECIFICATION

(11) **EP 3 204 516 B1**
(45) Date of publication and mention of the grant of the patent: **26.04.2023**
(21) Application number: 15848684.5
(22) Date of filing: 05.10.2015
(51) Int. Cl.: C12Q 1/68, G01N 33/574, C07K 16/28

(54) **ANGIOPOIETIN-2 BIOMARKERS PREDICTIVE OF ANTI-IMMUNE CHECKPOINT RESPONSE**
PRÄDIKTIVE ANGIOPOIETIN-2-BIOMARKER FÜR ANTI-IMMUN-CHECKPOINT-REAKTION
BIOMARQUEURS À BASE D'ANGIOPOÏÉTINE -2 UTILISÉS POUR LA PRÉDICTION DE LA RÉPONSE DE POINT DE CONTRÔLE ANTI-IMMUNITAIRE

(30) Priority: 06.10.2014 US 201462060230 P; 14.09.2015 US 201562218277 P; 15.09.2015 US 201562218624 P
(43) Date of publication of application: 16.08.2017
(73) Proprietor: Dana-Farber Cancer Institute, Inc., Boston, MA 02115-5450 (US)
(72) Inventor: HODI,F. Stephen, Framingham, MA 01701 (US); ZHOU, Jun, Waltham, MA 02452 (US); WU, Xinqi, Chestnut Hill, MA 02467 (US)
(74) Representative: Hoefer & Partner Patentanwälte mbB
(86) International application number: PCT/US2015/053933
(87) International publication number: WO 2016/057367

(56) References cited:
- WO-A1-2013/106765
- WO-A1-2013/148288
- WO-A1-2014/185540
- WO-A2-2008/143979
- WO-A2-2014/078468
- S.-S. CHAE ET AL: "Angiopoietin-2 Interferes with Anti-VEGFR2-Induced Vessel Normalization and Survival Benefit in Mice Bearing Gliomas", CLINICAL CANCER RESEARCH, vol. 16, no. 14, 15 July 2010 (2010-07-15) , pages 3618-3627, XP055441515, US ISSN: 1078-0432, DOI: 10.1158/1078-0432.CCR-09-3073
- DREW M. PARDOLL: "The blockade of immune checkpoints in cancer immunotherapy", NATURE REVIEWS. CANCER, vol. 12, no. 4, 1 April 2012 (2012-04-01), pages 252-264, XP055415943, GB ISSN: 1474-175X, DOI: 10.1038/nrc3239

## Description

### Cross-Reference to Related Applications

This application claims the benefit of U.S. Provisional Application No. 62/218,624, filed on 15 September 2015; U.S. Provisional Application No. 62/218,277, filed on 14 September 2015; and U.S. Provisional Application No. 62/060,230, filed on 6 October 2014.

### Background of the Invention

Cancer immune therapy is a rapidly developing field that has yielded impressive and promising breakthroughs. For example, the blockade of the immune checkpoint molecule, CTLA-4, has been proven to increase overall survival of metastatic melanoma patients and ipilimumab, an anti-CTLA-4 antibody, has been approved by the FDA as a standard therapy for late-stage melanoma. Malignant melanoma is a neoplastic disease with increasing incidence, poor prognosis, and limited treatment options, particularly in advanced tumors. The 5-year survival rate for advanced melanoma patients with distant metastases is still as low as 5% to 10%. However, only a subset of patients achieve a durable clinical response. While the combination of ipilimumab with anti-VEGF (e.g., bevacizumab) or PD-1 blockade increases clinical efficacy and response rate of ipilimumab, the best response rate thus far observed has been approximately 50% using ipilimumab in combination with PD-1 blockade.

Reliable biomarkers that can predict response or resistance to immune therapies (*e.g.,* blockade of immune checkpoint molecules, such as CTLA-4) blockade are therefore critical for stratifying patient populations and selecting patients who will or will not benefit from such immune therapies. However, such biomarkers are not currently known. Accordingly, there is a great need to identify such biomarkers useful for diagnostic, prognostic, and therapeutic purposes.

### Summary of the Invention

The present invention is based, at least in part, on the discovery that circulating Ang-2 is a highly specific early biomarker for prediction of clinical outcomes (*e.g.,* poor clinical outcomes such as progressive disease and shortened survival) in cancer patients treated with anti-immune checkpoint therapies, such as those comprising an anti-CTLA-4 therapeutic (*e.g.,* ipilimumab alone, ipilimumab in combination with bevacizumab, and the like). Increased circulating Ang-2 is a mechanism for resistance to anti-cancer immunotherapy and adding anti-Ang-2 therapeutics is believed to improve the efficacy of anti-cancer immunotherapies.

In one aspect of the disclosure, a method of identifying the likelihood of a cancer in a subject to be responsive to an anti-immune checkpoint therapy, the method comprising:
a) obtaining or providing a patient sample from a patient having cancer; b) measuring the amount or activity of at least one biomarker listed in Table 1 in the subject sample; and c) comparing said amount or activity of the at least one biomarker listed in Table 1 in a control sample, wherein a significantly increased amount or activity of the at least one biomarker listed in Table 1 in the subject sample relative to the control sample identifies the cancer as being less likely to be responsive to the anti-immune checkpoint therapy and wherein a decreased amount or activity of the at least one biomarker listed in Table 1 in the subject sample relative to the control sample identifies the cancer as being more likely to be responsive to the anti-immune checkpoint therapy, is provided.

In another aspect of the disclosure, a method of identifying the likelihood of a cancer in a subject to be responsive to anti-immune checkpoint therapy, the method comprising: a) obtaining or providing a patient sample from a patient having cancer, wherein the sample comprises nucleic acid molecules from the subject; b) determining the copy number of at least one biomarker listed in Table 1 in the sample; and c) comparing said copy number to that of a control sample, wherein an increased copy number of the biomarker in the sample relative to the control sample identifies the cancer as being less likely to be responsive to the anti-immune checkpoint therapy and wherein a decreased copy number of the biomarker in the sample relative to the control sample identifies the cancer as being more likely to be responsive to the anti-immune checkpoint therapy, is provided.

The methods of the present disclosure are characterized by many embodiments and each such embodiment can be applied to any method described herein. For example, in one embodiment, the method further comprises recommending, prescribing, or administering anti-immune checkpoint therapy if the cancer is determined likely to be responsive to anti-immune checkpoint therapy or administering anti-cancer therapy other than anti-immune checkpoint therapy if the cancer is determined be less likely to be responsive to anti-immune checkpoint therapy. In another embodiment, the anti-cancer therapy is selected from the group consisting of targeted therapy, chemotherapy, radiation therapy, and/or hormonal therapy. In still another embodiment, the control sample is determined from a cancerous or non-cancerous sample from either the patient or a member of the same species to which the patient belongs. In yet another embodiment, the control sample is a cancerous or non-cancerous sample from the patient obtained from an earlier point in time than the patient sample, optionally wherein the control sample is obtained before the patient has received anti-immune checkpoint therapy and the patient sample is obtained after the patient has received anti-immune checkpoint therapy. In another embodiment, the control sample comprises cells or does not comprise cells. In still another embodiment, the control sample comprises cancer cells known to be responsive or non-responsive to the anti-immune checkpoint therapy.

In still another aspect of the disclosure, a method of assessing the efficacy of an agent for treating a cancer in a subject that is unlikely to be responsive to anti-immune checkpoint therapy, comprising: a) detecting in a first subject sample and maintained in the presence of the agent the amount or activity of at least one biomarker listed in Table 1; b) detecting the amount or activity of the at least one biomarker listed in Table 1 in a second subject sample and maintained in the absence of the test compound; and c) comparing the amount or activity of the at least one biomarker listed in Table 1 from steps a) and b), wherein a significantly increased amount or activity of the at least one biomarker listed in Table 1 in the first subject sample relative to at least one subsequent subject sample, indicates that the agent treats the cancer in the subject, is provided.

In yet another aspect of the disclosure, a method of assessing the efficacy of an agent for treating a cancer in a subject or prognosing progression of a cancer in a subject, comprising: a) detecting in a subject sample at a first point in time the amount or activity of at least one biomarker listed in Table 1; b) repeating step a) during at least one subsequent point in time after administration of the agent; and c) comparing the expression and/or activity detected in steps a) and b), wherein a significantly increased amount or activity of the at least one biomarker listed in Table 1 in the first subject sample relative to at least one subsequent subject sample, indicates that the cancer is unlikely to progress or that the agent treats the cancer in the subject, is provided.

As stated above, the methods of the present disclosure are characterized by many embodiments and each such embodiment can be applied to any method described herein. In one embodiment, the subject has undergone treatment, completed treatment, and/or is in remission for the cancer between the first point in time and the subsequent point in time. In another embodiment, the first and/or at least one subsequent sample is selected from the group consisting of *ex vivo* and *in vivo* samples. In still another embodiment, the first and/or at least one subsequent sample is obtained from an animal model of the cancer. In yet another embodiment, the first and/or at least one subsequent sample is a portion of a single sample or pooled samples obtained from the subject.

In another aspect of the disclosure, a cell-based assay for screening for agents that have a cytotoxic or cytostatic effect on a cancer cell that is unresponsive to anti-immune checkpoint therapy comprising, contacting the cancer cell with a test agent, and determining the ability of the test agent to decrease the amount or activity of at least one biomarker listed in Table 1 is provided. In one embodiment, the step of contacting occurs *in vivo, ex vivo,* or *in vitro.*

As stated above, the methods and/or assays of the present disclosure are characterized by many embodiments and each such embodiment can be applied to any method described herein. In one embodiment, the subject sample and/or the control sample has not been contacted with any melanoma treatment or inhibitor of an immune checkpoint. In another embodiment, the subject has not been administered any melanoma treatment or inhibitor of an immune checkpoint. In still another embodiment, the method or assay further comprises recommending, prescribing, or administering at least one additional anti-cancer therapeutic agent, optionally wherein the at least one additional anti-cancer therapeutic agent is bevacizumab and/or an anti-Ang-2 therapeutic agent. In yet another embodiment, the subject sample is selected from the group consisting of serum, whole blood, plasma, urine, cells, cell lines, and biopsies. In another embodiment, the amount of the at least one biomarker listed in Table 1 is detected using a reagent which specifically binds with the protein. In still another embodiment, the reagent is selected from the group consisting of an antibody, an antibody derivative, and an antibody fragment. In yet another embodiment, the at least one biomarker listed in Table 1 is assessed by detecting the presence in the sample of a transcribed polynucleotide or portion thereof. In another embodiment, the transcribed polynucleotide is an mRNA or a cDNA. In still another embodiment, the step of detecting further comprises amplifying the transcribed polynucleotide. In yet another embodiment, the transcribed polynucleotide is detected by identifying a nucleic acid that anneals with the biomarker nucleic acid, or a portion thereof, under stringent hybridization conditions. In another embodiment, the at least one biomarker listed in Table 1 is human Ang-2, or a fragment thereof. In still another embodiment, the anti-immune checkpoint therapy comprises at least one antibody selected from the group consisting of anti-CTLA-4 antibodies, anti-PD-1 antibodies, anti-PD-L1 antibodies, anti-PD-L2 antibodies, and combinations thereof. In yet another embodiment, the anti-immune checkpoint therapy comprises ipilimumab. In another embodiment, the likelihood of the cancer in the subject to be responsive to anti-immune checkpoint therapy is the likelihood of at least one criteria selected from the group consisting of cellular proliferation, tumor burden, m-stage, metastasis, progressive disease, clinical benefit rate, survival until mortality, pathological complete response, semi-quantitative measures of pathologic response, clinical complete remission, clinical partial remission, clinical stable disease, recurrence-free survival, metastasis free survival, disease free survival, circulating tumor cell decrease, circulating marker response, and RECIST criteria. In still another embodiment, the cancer is a solid tumor. In yet another embodiment, the cancer is a cancer responsive to immune checkpoint blockade, such as melanoma, non-small cell lung cancer (SCLC), or renal cell cancer. In another embodiment, the melanoma is metastatic melanoma. In still another embodiment, the subject is a mammal, such as an animal model of cancer or a human.

### Brief Description of Figures

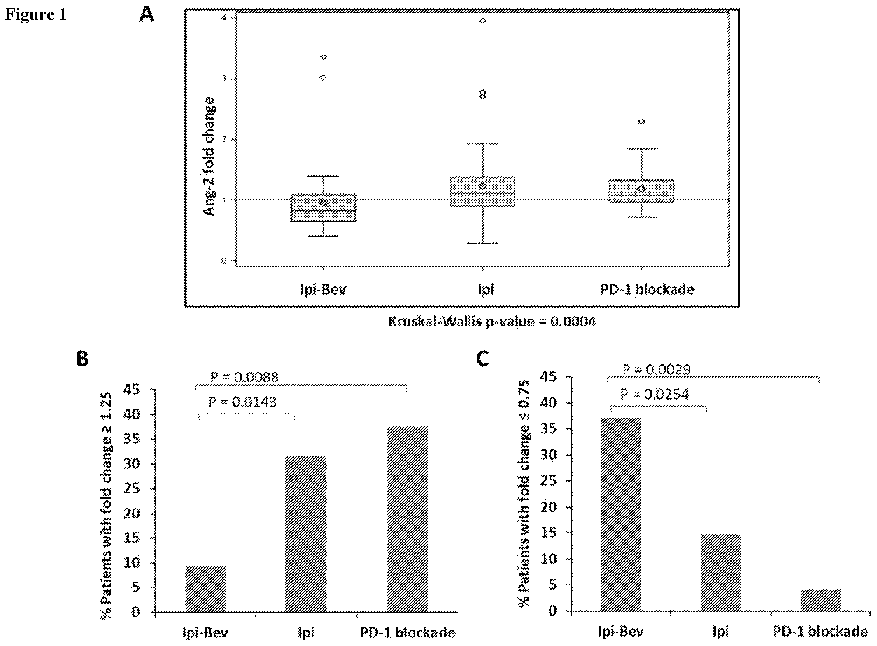

**Figure 1** includes 3 panels, identified as panels A, B, and C, which show that PD-1 blockade and lpi increase circulating Ang-2 levels in significant proportions of patients, while Ipi-Bev decreases circulating Ang-2 levels in significant proportions of patients. Panel A shows that Ipi-Bev-treated patients displayed smaller fold changes than Ipi- and PD-1 blockade-treated patients. Panel B shows the proportion of patients that displayed an increase (*e.g.,* fold change ≥ 1.25) in serum Ang-2. Panel C shows the proportion of patients that displayed a decrease (*e.g.,* fold changes ≤ 0.75) in serum Ang-2 level.

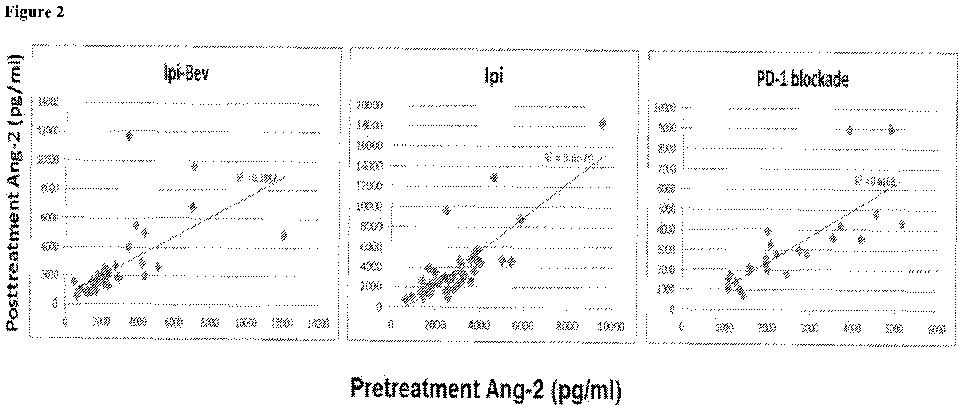

**Figure 2** shows that post-treatment circulating Ang-2 levels is correlated with pre-treatment levels in patients treated with the depicted immune checkpoint therapy.

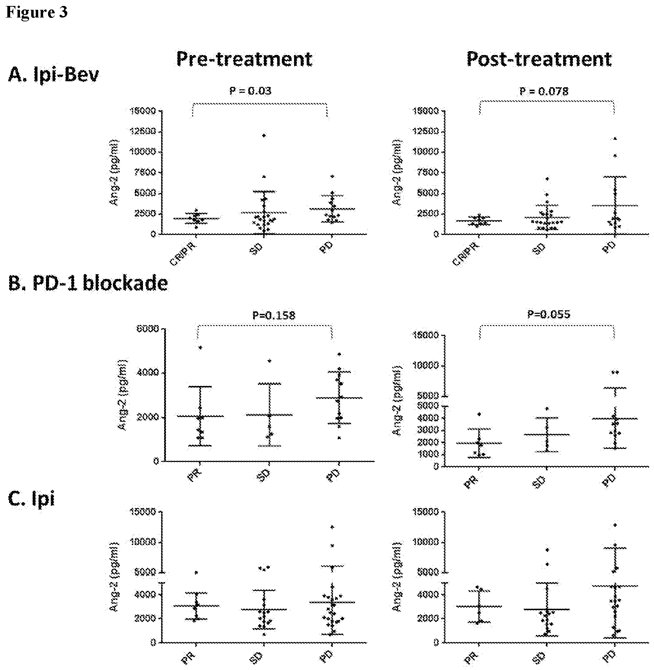

**Figure 3** includes 3 panels, identified as panels A, B, and C, which show the results of pre-treatment and post-treatment circulating Ang-2 in CR/PR, SD and PD patients treated with immune checkpoint therapy (*i.e.,* Ipi-Bev-treated patients in Panel A; PD-1 blockade-treated patients in Panel B; and Ipi-treated patients in Panel C).

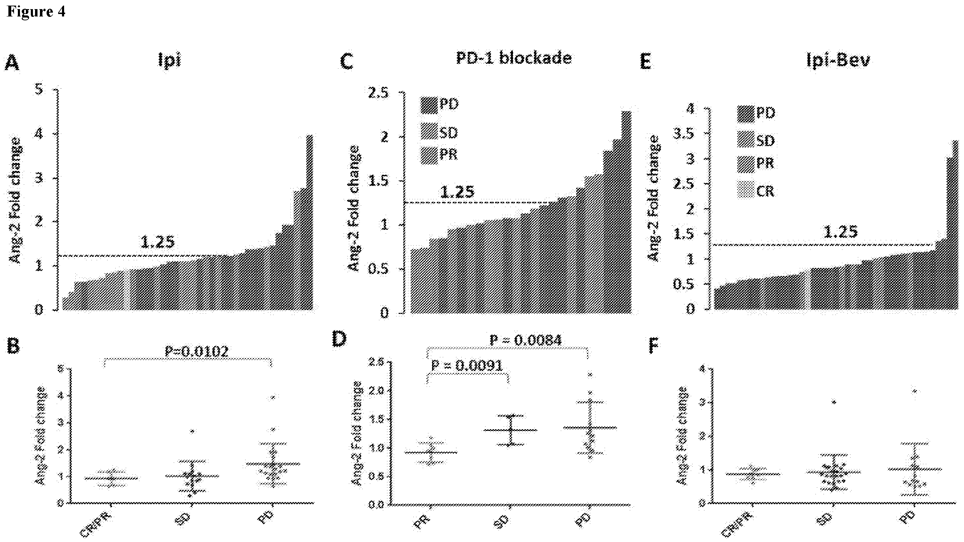

**Figure 4** includes 6 panels, identified as panels A, B, C, D, E, and F, which show the results of clinical response in comparison to circulating Ang-2 fold change in patients treated with immune checkpoint therapy (*i.e.,* Ipi-treated patients in Panels A-B; PD-1 blockade-treated patients in Panels C-D; and lpi-Bev-treated patients in Panels E-F. Patients with a fold change of 1.25 and larger had PD or less frequently SD. Only one Ipi patient with a fold change of 1.26 achieved PR.

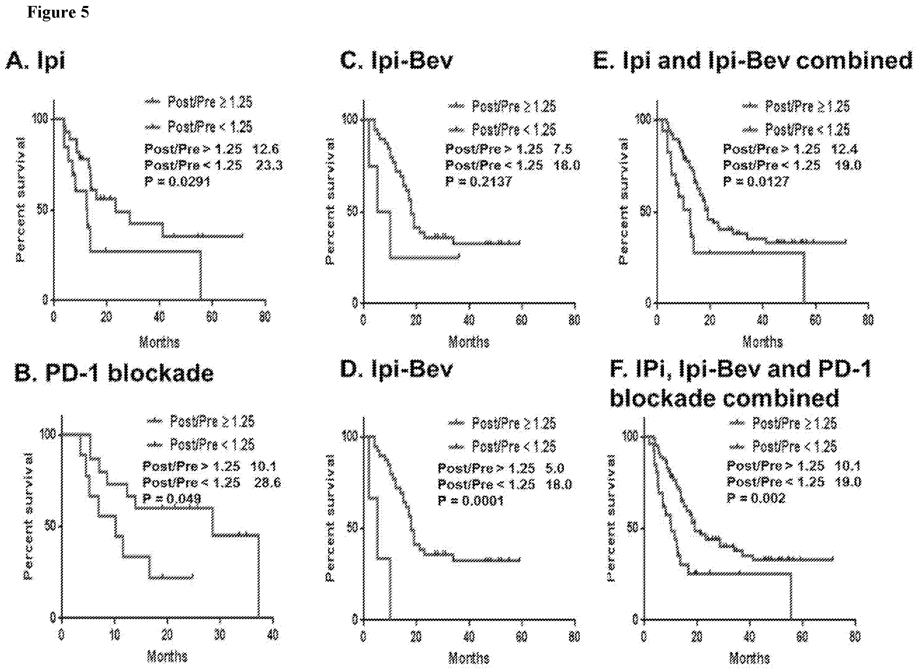

**Figure 5** includes 6 panels, identified as panels A, B, C, D, E, and F, which show Kaplan-Meier survival curves based on circulating serum Ang-2 fold changes (post-/pre-treatment ratios) in patients treated with immune checkpoint therapy (*i.e.,* Ipi-treated patients in Panel A; PD-1 blockade-treated patients in Panel B; Ipi-Bev-treated patients in Panel C; Ipi-Bev-treated patients, with those patients displaying potent antibody response to Ang-2 excluded, in Panel D; combination of Ipi-treated and Ipi-Bev-treated patients in Panel E; and combination of !pi,-treated, Ipi-Bev-treated, and PD-1 blockade-treated patients in Panel F). Large fold changes (≥ 1.25) were associated with reduced overall survival. Median survivals in months are indicated.

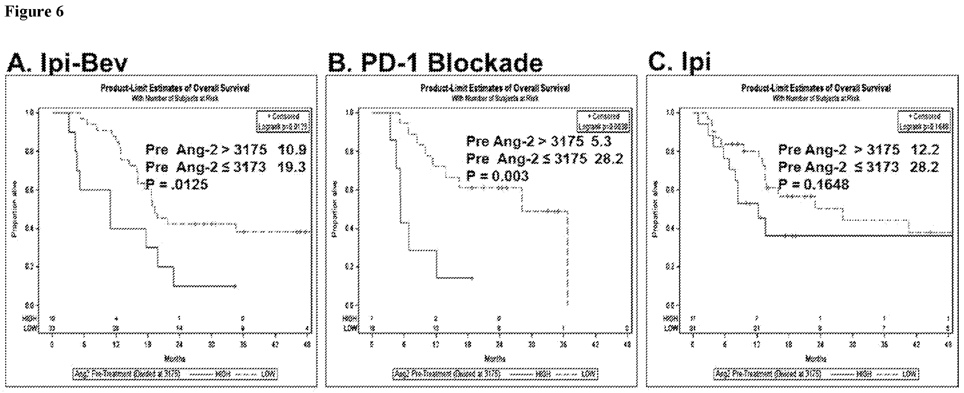

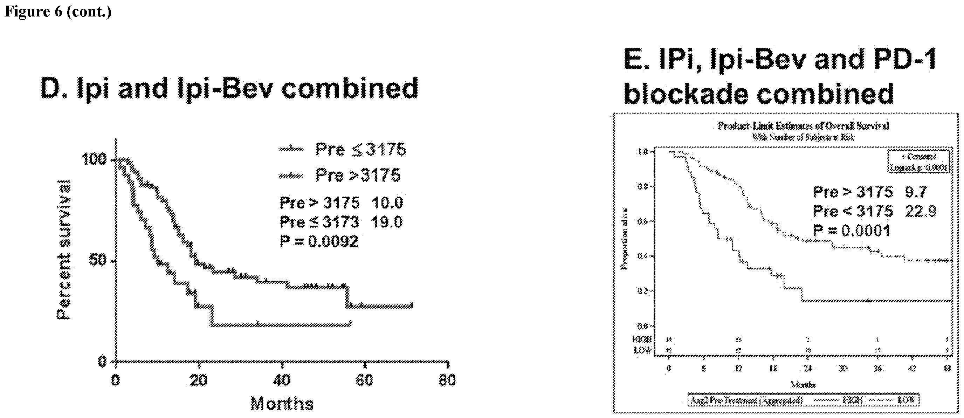

**Figure 6** includes 5 panels, identified as panels A, B, C, D, and E, which show Kaplan-Meier survival curves based on pre-treatment circulating serum Ang-2 levels in patients treated with immune checkpoint therapy (*i.e.,* Ipi-Bev-treated patients in Panel A; PD-1 blockade-treated patients in Panel B; Ipi-treated patients in Panel C; combination of Ipi-treated and Ipi-Bev-treated patients in Panel D; and combination of !pi,-treated, Ipi-Bev-treated, and PD-1 blockade-treated patients in Panel E). High pre-treatment circulating Ang-2 (*e.g.,* > 3175 pg/ml) was associated with reduced overall survival. Median survivals in months are indicated.

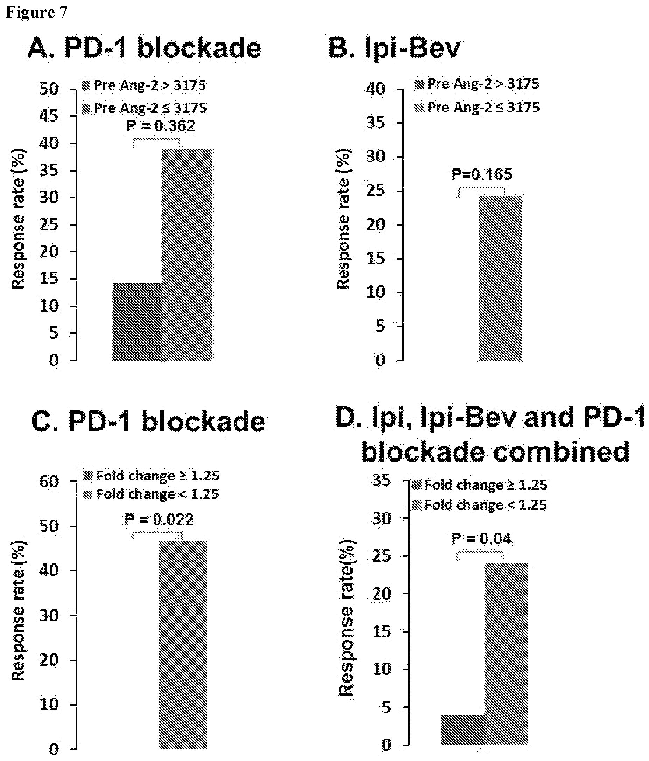

**Figure 7** includes 4 panels, identified as panels A, B, C, and D, which show the association of high pre-treatment circulating Ang-2 and increase in circulating Ang-2 with reduced response rate. Panels A and B show that high pre-treatment circulating Ang-2 was associated with a trend toward to reduced response rate in PD-1 blockade (Panel A) and Ipi-Bev (Panel B) patients. Panels C and D show that an increase in circulating Ang-2 was significantly associated with reduced response rate in PD-1 blockade (Panel C) and combination of Ipi, Ipi-Bev, and PD-1 blockade patients (Panel D).

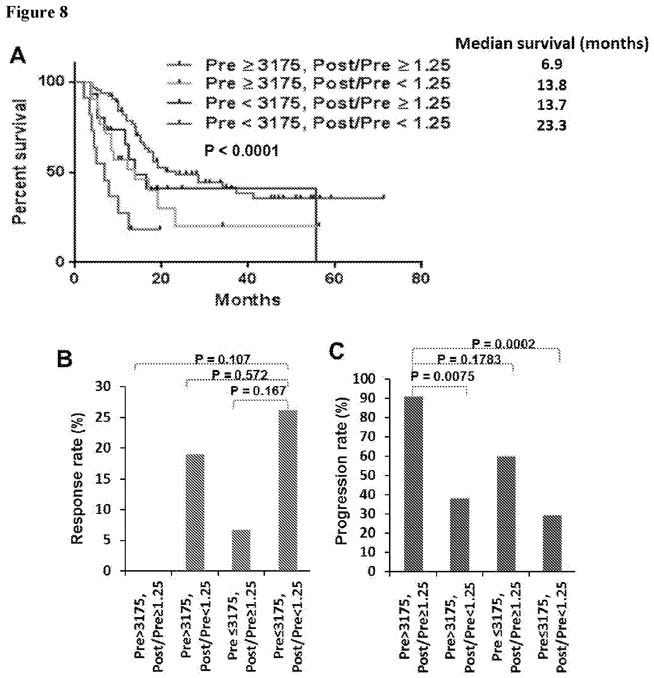

**Figure 8** includes 3 panels, identified as panels A, B, and C, which show that high pre-treatment serum Ang-2 level followed by large fold change is a strong predictor for poor OS and progression disease in Ipi, Ipi-Bev and PD-1 blockade patients combined. Panel A shows Kaplan-Meier survival curves based on pretreatment Ang-2 levels and fold changes. Panel B shows the response rate according to pre-treatment Ang-2 levels and fold changes. None of the patients with high pre-treatment Ang-2 levels and large fold changes achieved clinical response. Panel C shows the progression rate by pre-treatment Ang-2 levels and fold changes. Of note, 90% of the patients with high pre-treatment Ang-2 levels and large fold changes had progressive disease. These patients had significantly higher progression rate than those with small fold changes.

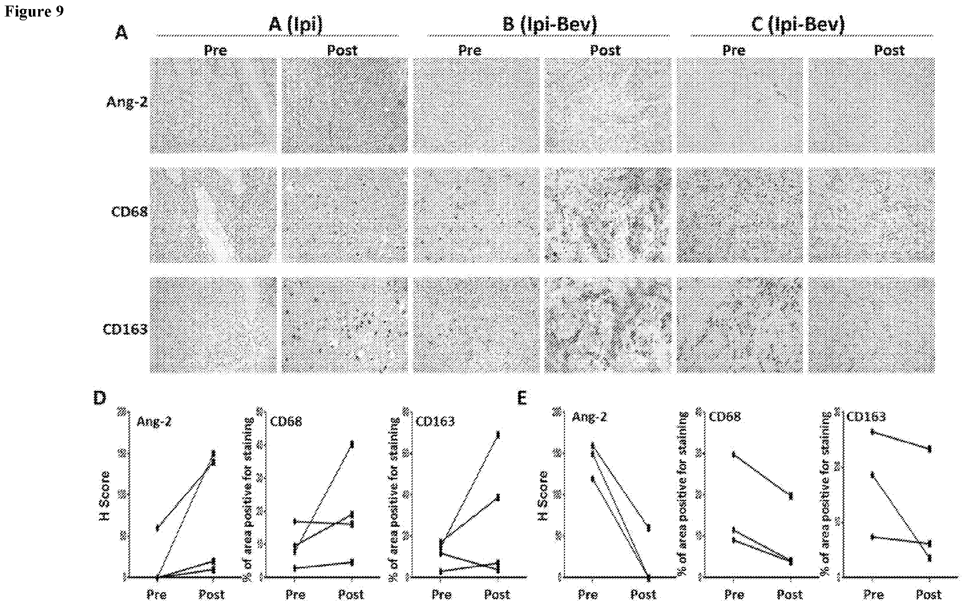

**Figure 9** includes 5 panels, identified as panels A, B, C, D, and E, which show that lpi and Ipi-Bev alter tumor Ang-2 expression and macrophage infiltration. Paired and sequential pre- and post-treatment tumor biopsies of melanoma patients on Ipi or Ipi-Bev were stained with anti-human Ang-2, CD68, and CD163 antibody, respectively. Panel A shows that robust upregulation of Ang-2 expression in the post-treatment tumor of an Ipi patient was accompanied by increased infiltration of CD68⁺ and CD163⁺ macrophages. Panel B shows that Ang-2 upregulation in the post-treatment tumor endothelia of an Ipi-Bev patient was accompanied by increased infiltration of CD68⁺ and CD163⁺ macrophages. Panel C shows that Ang-2 downregulation in the post-treatment tumor vasculature of an Ipi-Bev patient was accompanied by decreased infiltration of CD68⁺ and CD163⁺ macrophages. Panels D and E show quantitative analysis of Ang-2 expression and macrophage infiltration in tumors with increased (Panel D) or decreased (Panel E) tumor vascular Ang-2 expression. The expression of Ang-2 was presented according to H-Score and macrophage infiltration was expressed as the percent area positive for staining as proportional to cell number.

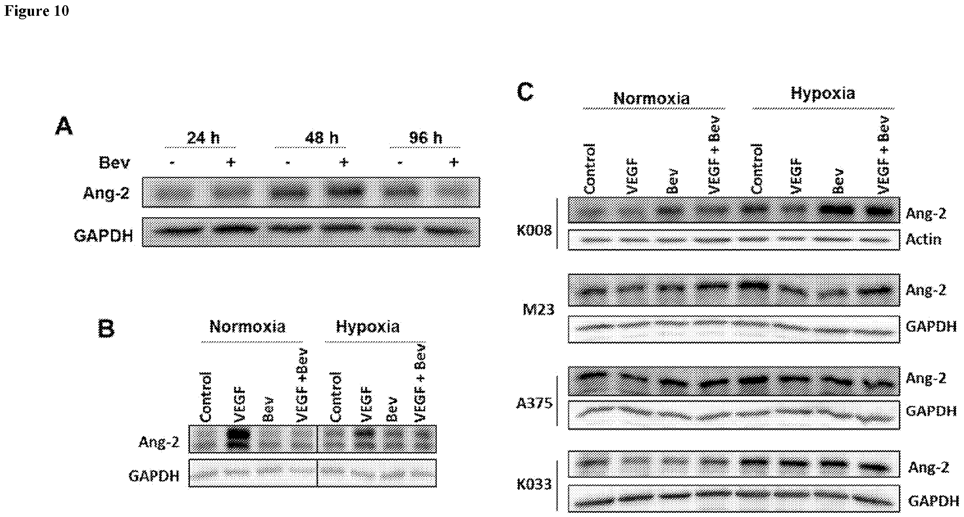

**Figure 10** includes 3 panels, identified as panels A, B, and C, which show results of regulation of Ang-2 expression in tumor associated endothelial cells (TEC) and melanoma cells. Panel A shows the results of Bev-mediated reduced Ang-2 expression in TEC after 96 hours of treatment. Panel B shows that VEGF promoted Ang-2 expression and that Bev blocked VEGF-induced Ang-2 expression in TEC. Panel C shows that hypoxia enhanced Ang-2 expression in melanoma cells.

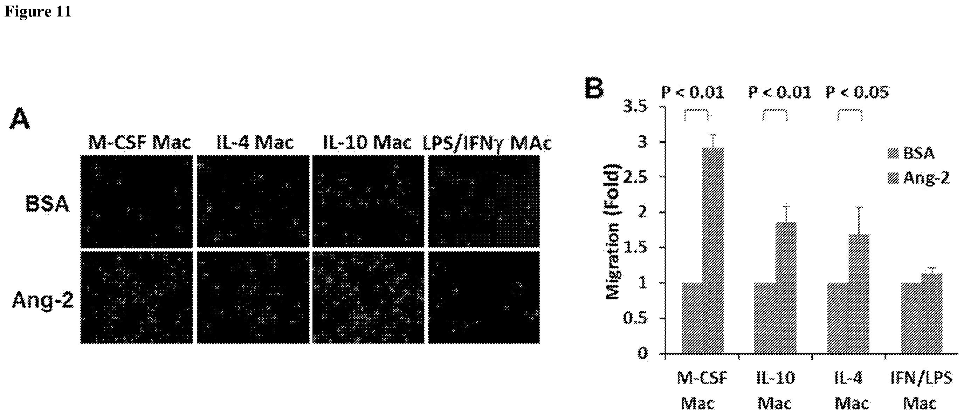

**Figure 11** includes 2 panels, identified as panels A and B, which show that Ang-2 promotes migration of M2-like monocyte derived macrophages. Representative images (Panel A) and bar graphs (Panel B) of migration are shown. Migration was driven with 300 ng/ml recombinant human Ang-2. Data are presented as mean ± SD of 3-4 independent experiments shown in Panel B. Ang-2 promoted migration of M-CSF, IL-10 or IL-4 activated M2-like, but not IFNγ/LPS activated M1-like macrophages.

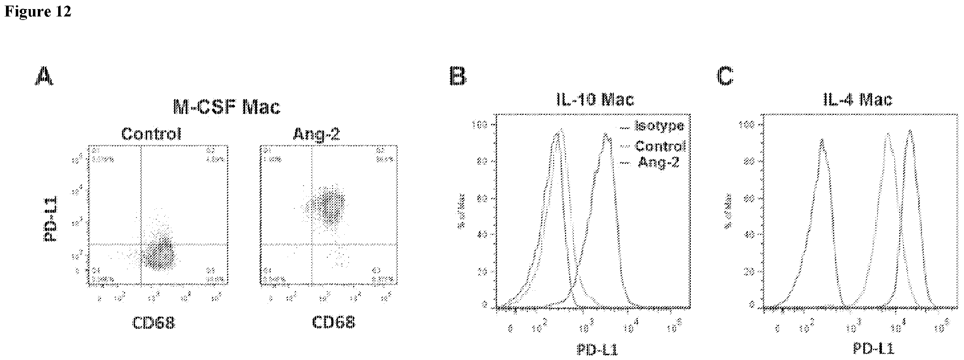

Figure 12 includes 3 panels, identified as panels A, B, and C, which show that Ang-2 induces PD-L1 expression on macrophages. Macrophages were generated by treatment of monocytes with M-CSF for 6 days and then activated with M-CSF (100 ng/ml), IL-4 (20 ng/ml), or IL-10 (20 ng/ml) for 2 days in the presence or absence of recombinant Ang-2 (300 ng/ml). Macrophages were collected and stained with PE-conjugated PD-L1 and then FITC-conjugated CD68 antibody after permeabilization/fixation. Cells were gated first on SSC/FSC and analyzed for PD-L1⁺ and CD68⁺ cells (Panel A) or gated on CD68⁺ cells and then analyzed for PD-L1 expression (Panels B and C). The numbers of M-CSF-activated macrophages (Panel A), IL-10-activated macrophages (Panel B), and IL-4-activated macrophages (Panel C) are shown.

For any figure showing a bar histogram, curve, or other data associated with a legend, the bars, curve, or other data presented from left to right for each indication correspond directly and in order to the boxes from top to bottom of the legend.

### Detailed Description of the Invention

In one aspect the invention is directed to a method of identifying the likelihood of a cancer in a subject to be responsive to an anti-immune checkpoint therapy, wherein the anti-immune checkpoint therapy comprises a therapy targeting CTLA-4 or PD-1, the method comprising:
a) using a patient sample from a patient having cancer;
b) measuring the amount or activity of human Ang2 in the subject sample; and
c) comparing said amount or activity of human Ang2 in a control sample,
wherein a significantly increased amount or activity of human Ang2 in the subject sample relative to the control sample identifies the cancer as being less likely to be responsive to the anti-immune checkpoint therapy and wherein a decreased amount or activity of human Ang2 in the subject sample relative to the control sample identifies the cancer as being more likely to be responsive to the anti-immune checkpoint therapy.

In another aspect the invention is directed to a method of identifying the likelihood of a cancer in a subject to be responsive to anti-immune checkpoint therapy, wherein the anti-immune checkpoint therapy comprises a therapy targeting CTLA-4 or PD-1, the method comprising:
a) using a patient sample from a patient having cancer, wherein the sample comprises nucleic acid molecules from the subject;
b) determining the copy number of human Ang2 in the sample; and
c) comparing said copy number to that of a control sample, wherein an increased copy number of human Ang2 in the sample relative to the control sample identifies the cancer as being less likely to be responsive to the anti-immune checkpoint therapy and wherein a decreased copy number of human Ang2 in the sample relative to the control sample identifies the cancer as being more likely to be responsive to the anti-immune checkpoint therapy.

In another aspect the invention is directed to a method of assessing the efficacy of an anti-immune checkpoint agent for treating a cancer in a subject that is unlikely to be responsive to anti-immune checkpoint therapy, wherein the anti-immune checkpoint therapy comprises a therapy targeting CTLA-4 or PD-1, comprising:
a) detecting in a first subject sample and maintained in the presence of the agent the amount or activity of human Ang2;
b) detecting the amount or activity of human Ang2 in a second subject sample and maintained in the absence of the test compound; and
c) comparing the amount or activity of human Ang2 from steps a) and b), wherein a significantly increased amount or activity of human Ang2 in the first subject sample relative to at least one subsequent subject sample, indicates that the agent treats the cancer in the subject.

In another aspect the invention is directed to a method of assessing the efficacy of an anti-immune checkpoint agent for treating a cancer in a subject or prognosing progression of a cancer in a subject, wherein the anti-immune checkpoint therapy comprises a therapy targeting CTLA-4 or PD-1, comprising:
a) detecting in a subject sample at a first point in time the amount or activity of human Ang2.
b) repeating step a) during at least one subsequent point in time after administration of the agent; and
c) comparing the expression and/or activity detected in steps a) and b), wherein a significantly increased amount or activity of human Ang2_in the first subject sample relative to at least one subsequent subject sample, indicates that the cancer is unlikely to progress or that the agent treats the cancer in the subject,

preferably wherein between the first point in time and the subsequent point in time, the subject has undergone treatment, completed treatment, and/or is in remission for the cancer, or
wherein the first and/or at least one subsequent sample is selected from the group consisting of *ex vivo* and *in vivo* samples, or
wherein the first and/or at least one subsequent sample is obtained from an animal model of the cancer, or
wherein the first and/or at least one subsequent sample is a portion of a single sample or pooled samples obtained from the subject.

It has been determined herein that Ang-2 is a highly specific biomarker for predicted clinical outcome in cancer patients (*e.g.,* metastatic melanoma patients) receiving anti-immune checkpoint-based therapy (*e.g.,* ipilimumab alone or in combination with other anti-cancer therapeutics). Accordingly, the present invention relates, in part, to methods for stratifying patients and predicting response of a cancer in a subject to anti-immune checkpoint therapy based upon a determination and analysis of biomarkers described herein according to amount (*e.g.,* copy number or level of expression) and/or activity, relative to a control. In addition, such analyses can be used in order to provide useful anti-immune checkpoint treatment regimens (*e.g.,* based on predictions of clinical response, subject survival or relapse, timing of adjuvant or neoadjuvant treatment, etc.).

### I. Definitions

The articles "a" and "an" are used herein to refer to one or to more than one (*i.e.* to at least one) of the grammatical object of the article. By way of example, "an element" means one element or more than one element.

The term "altered amount" or "altered level" refers to increased or decreased copy number *(e.g.,* germline and/or somatic) of a biomarker nucleic acid, *e.g.,* increased or decreased expression level in a cancer sample, as compared to the expression level or copy number of the biomarker nucleic acid in a control sample. The term "altered amount" of a biomarker also includes an increased or decreased protein level of a biomarker protein in a sample, *e.g.,* a cancer sample, as compared to the corresponding protein level in a normal, control sample. Furthermore, an altered amount of a biomarker protein may be determined by detecting posttranslational modification such as methylation status of the marker, which may affect the expression or activity of the biomarker protein.

The amount of a biomarker in a subject is "significantly" higher or lower than the normal amount of the biomarker, if the amount of the biomarker is greater or less, respectively, than the normal level by an amount greater than the standard error of the assay employed to assess amount, and preferably at least 20%, 30%, 40%, 50%, 60%, 70%, 80%, 90%, 100%, 150%, 200%, 300%, 350%, 400%, 500%, 600%, 700%, 800%, 900%, 1000% or than that amount. Such "significance" can be assessed from any desired or known point of comparison, such as a particular post-treatment versus pre-treatment biomarker measurement ratio *(e.g.,* 1.05-fold, 1.1-fold, 1.15-fold, 1.2-fold, 1.25-fold, 1.3-fold, 1.35-fold, 1.4-fold, 1.45-fold, 1.5-fold, 1.55-fold, 1.6-fold, 1.65-fold, 1.7-fold, 1.75-fold, 1.8-fold, 1.85-fold, 1.9-fold, 1.95-fold, and the like) or a particular pre-treatment serum biomarker protein measurement (e.g., 2,500 pg/ml, 2,750 pg/ml, 3,000 pg/ml, 3,175 pg/ml, 3,250 pg/ml, 3,500 pg/ml, and the like). The values can be measured at discrete times or at discrete intervals, such as time after treatment (e.g., 1 week, 2 weeks, 3 weeks, 4 weeks, 1 month, 2 months, 3 months, 4 months, 5 months, 6 months, 7 months, 8 months, 9 months, 10 months, 11 months, 1 year, etc.) or any time interval therein (e.g., tracking values every month and/or determining values between months 3.1 and 1 year). Alternatively, the amount of the biomarker in the subject can be considered "significantly" higher or lower than the normal amount if the amount is at least about two, and preferably at least about three, four, or five times, higher or lower, respectively, than the normal amount of the biomarker. Such "significance" can also be applied to any other measured parameter described herein, such as for expression, inhibition, cytotoxicity, cell growth, and the like. Moreover, the comparisons can be used individually or in any combination (e.g., a particular post-treatment versus pre-treatment biomarker measurement ratio in combination with a particular pre-treatment serum biomarker protein measurement).

The term "altered level of expression" of a biomarker refers to an expression level or copy number of the biomarker in a test sample, *e.g.,* a sample derived from a patient suffering from cancer, that is greater or less than the standard error of the assay employed to assess expression or copy number, and is preferably at least twice, and more preferably three, four, five or ten or more times the expression level or copy number of the biomarker in a control sample (e.g., sample from a healthy subjects not having the associated disease) and preferably, the average expression level or copy number of the biomarker in several control samples. The altered level of expression is greater or less than the standard error of the assay employed to assess expression or copy number, and is preferably at least 20%, 30%, 40%, 50%, 60%, 70%, 80%, 90%, 100%, 150%, 200%, 300%, 350%, 400%, 500%, 600%, 700%, 800%, 900%, 1000% or more times the expression level or copy number of the biomarker in a control sample (e.g., sample from a healthy subjects not having the associated disease) and preferably, the average expression level or copy number of the biomarker in several control samples.

The term "altered activity" of a biomarker refers to an activity of the biomarker which is increased or decreased in a disease state, *e.g.,* in a cancer sample, as compared to the activity of the biomarker in a normal, control sample. Altered activity of the biomarker may be the result of, for example, altered expression of the biomarker, altered protein level of the biomarker, altered structure of the biomarker, or, *e.g.,* an altered interaction with other proteins involved in the same or different pathway as the biomarker or altered interaction with transcriptional activators or inhibitors.

The term "altered structure" of a biomarker refers to the presence of mutations or allelic variants within a biomarker nucleic acid or protein, e.g., mutations which affect expression or activity of the biomarker nucleic acid or protein, as compared to the normal or wild-type gene or protein. For example, mutations include, but are not limited to substitutions, deletions, or addition mutations. Mutations may be present in the coding or non-coding region of the biomarker nucleic acid.

The term "Ang-2" refers to angiopoietin-2, which is a ligand of the tyrosine kinase, Tie-2. Ang-2 functions as a vessel-destabilizing molecule and is a critical regulator of blood vessel maturation by antagonizing angiopoietin 1 (ANGPT1) (Maisonpierre et al. (1997) Science 277:55-60; Cheung et al. (1998) Genomics 48:389-391; Witzenbichler et al. (1998) J. Biol. Chem. 273:18514-18521; Sato et al. (1998) Int. Immunol. 10:1217-1227; Tanaka et al. (1999) J. Clin. Invest. 103:341-345). Ang-2 is almost exclusively produced by endothelial cells and facilitates angiogenesis by controlling the Ang-2/Tie-2 signaling pathway. Elevated levels of circulating Ang-2 and higher Ang-2/Ang-1 ratios compared to levels in normal tissues have been reported to be associated with poor prognosis in a number of different tumor types (Kim et al. (2013) BMC Cancer 13:611; Buddingh et al. (2014) J. AM. Coll. Surg. 218:26-32; Avraham et al. (2014) J. Pathol. 232:369-381). Circulating Ang-2 has been recently identified as a biomarker for progression and metastasis in melanoma. The term is intended to include fragments, variants (e.g., allelic variants), and derivatives thereof. Representative human Ang-2 cDNA and human Ang-2 protein sequences are well-known in the art and are publicly available from the National Center for Biotechnology Information (NCBI). For example, three different human Ang-2 isoforms are known. Human Ang-2 transcript variant 1 (NM_001147.2) represents the longest transcript and encodes the longest isoform, isoform a (NP_001138.1; signal peptide = residues 1-18; mature protein = residues 19-495). Human Ang-2 transcript variant 2 (NM_001118887.1) uses an alternate in-frame splice site compared to variant 1 and encodes an isoform b that lacks an internal amino acid compared to isoform a (NP_001112359.1; signal peptide = residues 1-18; mature protein = residues 19-496). Human Ang-2 transcript variant 3 (NM_001118888.1) lacks an alternate in-frame exon compared to variant 1 and encodes an isoform c that has the same N- and C-termini as isoform a, but is shorter compared to isoform a (NP_001112360.1; signal peptide = residues 1-18; mature protein = residues 19-444). Nucleic acid and polypeptide sequences of Ang-2 orthologs in organisms other than humans are well known and include, for example, chimpanzee Ang-2 (XM_001145337.2, XP_001145337.1, XM_001145488.2, and XP_001145488.1), dog Ang-2 (NM_001048126.1 and NP_001041591.1; signal peptide = residues 1-18; mature protein = residues 19-495), cow Ang-2 (NM_001098855.1 and NP_001092325.1; signal peptide = residues 1-18; mature protein = residues 19-496), mouse Ang-2 (NM_007426.4 and NP_031452.2; signal peptide = residues 1-18; mature protein = residues 19-496), rat Ang-2 (NM_134454.1 and NP_604449.1; signal peptide = residues 1-18; mature protein = residues 19-496), and chicken Ang-2 (NM_204817.1 and NP_990148.1). Representative sequences of Ang-2 orthologs are presented below in Table 1. Anti-Ang-2 antibodies suitable for detecting Ang-2 protein are well-known in the art and include, for example, OARA03063 (Aviva Systems Biology), 1OR-7635 (Fitzgerald Industries International), TA319449 (OriGene Technologies), ANG21-A (BIOTREND Chemikalien GmbH), sc-8357 and sc-7015 (Santa Cruz Biotechnology, Inc.), and 10691-R117 and 10691-RPO1 (Sin Biological). In addition, reagents are well-known for detecting Ang-2 expression (see, for example, ab99971 Ang-2 ELISA kit (Abeam), DANG20 Ang-2 ELISA kit (R&D Systems), KHC1641 Ang-2 ELISA kit (Life Technologies), and the like). Moreover, anti-Ang-2 antibodies suitable for therapeutically inactivating Ang-2 are also well-known in the art and include, for example, REGN910 (nesvacumab; Regeneron), LC06 and LC-8 (Thomas et al. (2013) PLoS One 8:e54923), 3.19.3 (Brown et al. (2010) Mol. Cancer Ther. 9:145-156), and antibodies described in Rennel et al. (2011) Microcirculation 18:598-607, U.S. Pat. Publ. 2006/0246071, U.S. Pat. Publ. 2013/0259859, U.S. Pat. Publ. 2010/0159587, U.S. Pat. Publ. 2011/0150895, U.S. Pat. Publ. 2013/0186797, and WO 2013/003606. It is to be noted that the term can further be used to refer to any combination of features described herein regarding Ang-2 molecules. For example, any combination of sequence composition, percentage identify, sequence length, domain structure, functional activity, *etc.* can be used to describe an Ang-2 molecule of the present invention.

Unless otherwise specified here within, the terms "antibody" and "antibodies" broadly encompass naturally-occurring forms of antibodies (e.g. IgG, IgA, IgM, IgE) and recombinant antibodies such as single-chain antibodies, chimeric and humanized antibodies and multi-specific antibodies, as well as fragments and derivatives of all of the foregoing, which fragments and derivatives have at least an antigenic binding site. Antibody derivatives may comprise a protein or chemical moiety conjugated to an antibody.

The term "antibody" as used herein also includes an "antigen-binding portion" of an antibody (or simply "antibody portion"). The term "antigen-binding portion", as used herein, refers to one or more fragments of an antibody that retain the ability to specifically bind to an antigen *(e.g.,* a biomarker polypeptide or fragment thereof). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent polypeptides (known as single chain Fv (scFv); see *e.g.,* Bird et al. (1988) Science 242:423-426; and Huston et al. (1988) Proc. Natl. Acad. Sci. USA 85:5879-5883; and Osbourn et al. 1998, Nature Biotechnology 16: 778). Such single chain antibodies are also intended to be encompassed within the term "antigen-binding portion" of an antibody. Any VH and VL sequences of specific scFv can be linked to human immunoglobulin constant region cDNA or genomic sequences, in order to generate expression vectors encoding complete IgG polypeptides or other isotypes. VH and VL can also be used in the generation of Fab, Fv or other fragments of immunoglobulins using either protein chemistry or recombinant DNA technology. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger, P., et al. (1993) Proc. Natl. Acad. Sci. USA 90:6444-6448; Poljak, R. J., et al. (1994) Structure 2:1121-1123).

Still further, an antibody or antigen-binding portion thereof may be part of larger immunoadhesion polypeptides, formed by covalent or noncovalent association of the antibody or antibody portion with one or more other proteins or peptides. Examples of such immunoadhesion polypeptides include use of the streptavidin core region to make a tetrameric scFv polypeptide (Kipriyanov, S.M., et al. (1995) Human Antibodies and Hybridomas 6:93-101) and use of a cysteine residue, biomarker peptide and a C-terminal polyhistidine tag to make bivalent and biotinylated scFv polypeptides (Kipriyanov, S.M., et al. (1994) Mol. Immunol. 31:1047-1058). Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion polypeptides can be obtained using standard recombinant DNA techniques, as described herein.

Antibodies may be polyclonal or monoclonal; xenogeneic, allogeneic, or syngeneic; or modified forms thereof (e.g. humanized, chimeric, etc.). Antibodies may also be fully human. Preferably, antibodies of the present invention bind specifically or substantially specifically to a biomarker polypeptide or fragment thereof. The terms "monoclonal antibodies" and "monoclonal antibody composition", as used herein, refer to a population of antibody polypeptides that contain only one species of an antigen binding site capable of immunoreacting with a particular epitope of an antigen, whereas the term "polyclonal antibodies" and "polyclonal antibody composition" refer to a population of antibody polypeptides that contain multiple species of antigen binding sites capable of interacting with a particular antigen. A monoclonal antibody composition typically displays a single binding affinity for a particular antigen with which it immunoreacts.

Antibodies may also be "humanized", which is intended to include antibodies made by a non-human cell having variable and constant regions which have been altered to more closely resemble antibodies that would be made by a human cell. For example, by altering the non-human antibody amino acid sequence to incorporate amino acids found in human germline immunoglobulin sequences. The humanized antibodies of the present invention may include amino acid residues not encoded by human germline immunoglobulin sequences (e.g., mutations introduced by random or site-specific mutagenesis *in vitro* or by somatic mutation *in vivo),* for example in the CDRs. The term "humanized antibody", as used herein, also includes antibodies in which CDR sequences derived from the germline of another mammalian species, such as a mouse, have been grafted onto human framework sequences.

The term "assigned score" refers to the numerical value designated for each of the biomarkers after being measured in a patient sample. The assigned score correlates to the absence, presence or inferred amount of the biomarker in the sample. The assigned score can be generated manually (e.g., by visual inspection) or with the aid of instrumentation for image acquisition and analysis. In certain embodiments, the assigned score is determined by a qualitative assessment, for example, detection of a fluorescent readout on a graded scale, or quantitative assessment. In one embodiment, an "aggregate score," which refers to the combination of assigned scores from a plurality of measured biomarkers, is determined. In one embodiment the aggregate score is a summation of assigned scores. In another embodiment, combination of assigned scores involves performing mathematical operations on the assigned scores before combining them into an aggregate score. In certain, embodiments, the aggregate score is also referred to herein as the "predictive score."

The term "biomarker" refers to a measurable entity of the present invention that has been determined to be predictive of anti-immune checkpoint therapy effects on a cancer. Biomarkers can include, without limitation, nucleic acids and proteins, including those shown in Table 1, the Examples, and the Figures.

A "blocking" antibody or an antibody "antagonist" is one which inhibits or reduces at least one biological activity of the antigen(s) it binds. In certain embodiments, the blocking antibodies or antagonist antibodies or fragments thereof described herein substantially or completely inhibit a given biological activity of the antigen(s).

The term "body fluid" refers to fluids that are excreted or secreted from the body as well as fluids that are normally not (e.g. amniotic fluid, aqueous humor, bile, blood and blood plasma, cerebrospinal fluid, cerumen and earwax, cowper's fluid or pre-ejaculatory fluid, chyle, chyme, stool, female ejaculate, interstitial fluid, intracellular fluid, lymph, menses, breast milk, mucus, pleural fluid, pus, saliva, sebum, semen, serum, sweat, synovial fluid, tears, urine, vaginal lubrication, vitreous humor, vomit).

The terms "cancer" or "tumor" or "hyperproliferative" refer to the presence of cells possessing characteristics typical of cancer-causing cells, such as uncontrolled proliferation, immortality, metastatic potential, rapid growth and proliferation rate, and certain characteristic morphological features. In some embodiments, such cells exhibit such characteristics in part or in full due to the expression and activity of immune checkpoint proteins, such as PD-1, PD-L1, and/or CTLA-4. Cancer cells are often in the form of a tumor, but such cells may exist alone within an animal, or may be a non-tumorigenic cancer cell, such as a leukemia cell. As used herein, the term "cancer" includes premalignant as well as malignant cancers. Cancers include, but are not limited to, B cell cancer, e.g., multiple myeloma, Waldenström's macroglobulinemia, the heavy chain diseases, such as, for example, alpha chain disease, gamma chain disease, and mu chain disease, benign monoclonal gammopathy, and immunocytic amyloidosis, melanomas, breast cancer, lung cancer, bronchus cancer, colorectal cancer, prostate cancer, pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematologic tissues, and the like. Other non-limiting examples of types of cancers applicable to the methods encompassed by the present invention include human sarcomas and carcinomas, e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, colorectal cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, liver cancer, choriocarcinoma, seminoma, embryonal carcinoma, Wilms' tumor, cervical cancer, bone cancer, brain tumor, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, e.g., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease. In some embodiments, cancers are epithlelial in nature and include but are not limited to, bladder cancer, breast cancer, cervical cancer, colon cancer, gynecologic cancers, renal cancer, laryngeal cancer, lung cancer, oral cancer, head and neck cancer, ovarian cancer, pancreatic cancer, prostate cancer, or skin cancer. In other embodiments, the cancer is breast cancer, prostate cancer, lung cancer, or colon cancer. In still other embodiments, the epithelial cancer is non-small-cell lung cancer, nonpapillary renal cell carcinoma, cervical carcinoma, ovarian carcinoma (e.g., serous ovarian carcinoma), or breast carcinoma. The epithelial cancers may be characterized in various other ways including, but not limited to, serous, endometrioid, mucinous, clear cell, Brenner, or undifferentiated.

In certain embodiments, the cancer encompasses melanoma. The term "melanoma" generally refers to cancers derived from melanocytes. Although melanocytes are predominantly located in skin, they are also found in other parts of the body, including the eye and bowel. Although cutaneous melanoma is most common, melanoma can originate from any melanocyte in the body. Though melanoma is less than five percent of the skin cancers, it is the seventh most common malignancy in the U.S. and is responsible for most of the skin cancer related deaths. The incidence has increased dramatically in the last several decades due to altered sun exposure habits of the population. several hereditary risk factors are also known. Other important risk factors are the number of pigment nevi, the number dysplastic nevi, and skin type. An increased risk is coupled to many nevi, both benign and dysplastic, and fair skin. Familial history of malignant melanomas is a risk factor, and approximately 8-12% of malignant melanoma cases are familial. Additional details are well known, such as described in US Pat. Pubsl. 2012-0269764 and 2013-0237445.

Malignant melanomas are clinically recognized based on the ABCD(E) system, where A stands for asymmetry, B for border irregularity, C for color variation, D for diameter >5 mm, and E for evolving. Further, an excision biopsy can be performed in order to corroborate a diagnosis using microscopic evaluation. Infiltrative malignant melanoma is traditionally divided into four principal histopathological subgroups: superficial spreading melanoma (SSM), nodular malignant melanoma (NMM), lentigo maligna melanoma (LMM), and acral lentiginous melanoma (ALM). Other rare types also exists, such as desmoplastic malignant melanoma. A substantial subset of malignant melanomas appear to arise from melanocytic nevi and features of dysplastic nevi are often found in the vicinity of infiltrative melanomas. Melanoma is thought to arise through stages of progression from normal melanocytes or nevus cells through a dysplastic nevus stage and further to an in situ stage before becoming invasive. Some of the subtypes evolve through different phases of tumor progression, which are called radial growth phase (RGP) and vertical growth phase (VGP).

In a preferred embodiment, a melanoma subtype is melanoma resistant to treatment with inhibitors of BRAF and/or MEK. For example, the methods described herein are useful for diagnosing and/or prognosing melanoma subtypes that are resistant to treatment with inhibitors of BRAF and/or MEK. Inhibitors of BRAF and/or MEK, especially of mutant versions implicated in cancer (e.g., BRAF^{V600E}) are well-known in the art.

BRAF is a member of the Raf kinase family of serine/threonine-specific protein kinases. This protein plays a role in regulating the MAP kinase/ERKs signaling pathway, which affects cell division, differentiation, and secretion. BRAF transduces cellular regulatory signals from Ras to MEK *in vivo.* BRAF is also referred to as v-raf murine sarcoma viral oncogene homolog B1. BRAF mutants are a mutated form of BRAF that has increased basal kinase activity relative to the basal kinase activity of wild type BRAF is also an activated form of BRAF. More than 30 mutations of the BRAF gene that are associated with human cancers have been identified. The frequency of BRAF mutations in melanomas and nevi are 80%. In 90% of the cases, a Glu for Val substitution at position 600 (referred to as V600E) in the activation segment has been found in human cancers. This mutation is observed in papillary thyroid cancer, colorectal cancer and melanoma. Other mutations which have been found are R462I, I463S, G464E, G464V, G466A, G466E, G466V, G469A, G469E, N581S, E585K, D594V, F595L, G596R, L597V, T599I, V600D, V600K, V600R, K601E or A728V. Most of these mutations are clustered to two regions: the glycine-rich P loop of the N lobe and the activation segment and flanking regions. A mutated form of BRAF that induces focus formation more efficiently than wild type BRAF is also an activated form of BRAF. As used herein, the term "inhibitor of BRAF" refers to a compound or agent, such as a small molecule, that inhibits, decreases, lowers, or reduces the activity of BRAF or a mutant version thereof. Examples of inhibitors of BRAF include, but are not limited to, vemurafenib (PLX-4032; also known as RG7204, RO5185426, and vemurafenib, C23H18ClF2N3O3S), PLX 4720 (C17H14CIF2N3O3S), sorafenib (C21H16ClF3N4O3), GSK2118436, and the like. These and other inhibitors of BRAF, as well as non-limited examples of their methods of manufacture, are described in, for example, PCT Publication Nos. WO 2007/002325, WO 2007/002433, WO 2009/047505, WO 03/086467; WO 2009/143024, WO 2010/104945, WO 2010/104973, WO 2010/111527 and WO 2009/152087; U.S. Pat. Nos. 6,187,799 and 7,329,670; and U.S. Patent Application Publication Nos. 2005/0176740 and 2009/0286783).

MEK1 is a known as dual specificity mitogen-activated protein kinase 1, which is an enzyme that in human is encoded by the MAP2K1 gene. Mutations of MEK1 involved in cancer are known and include, for example, mutation selected from 59delK and P387S or Q56P or C121S or P124L or F129L, and a MAP2K1 gene having a 175-177 AAG deletion or C1159T. As used herein, the term "inhibitor of MEK" refers to a compound or agent, such as a small molecule, that inhibits, decreases, lowers, or reduces the activity of MEK or a mutant version thereof. Examples of inhibitors of MEK include, but are not limited to, AZD6244 (6-(4-Bromo-2-chloro-phenylamino)-7-fluoro-3-methyl-3H-benzoimida- zole-5-carboxylic acid (2-hydroxy-ethoxy)-amide; selumetinib; Structure IV), and U0126 (1,4-diamino-2,3-dicyano-1,4-bis [2-aminophenylthio]butadiene; ARRY-142886; Structure V). Further non-limiting examples of MEK inhibitors include PD0325901, AZD2171, GDC-0973/XL-518, PD98059, PD184352, GSK1120212, RDEA436, RDEA119/BAY869766, AS703026, BIX 02188, BIX 02189, CI-1040 (PD184352), PD0325901, and PD98059. These and other inhibitors of MEK, as well as non-limiting examples of their methods of manufacture, are described in, for example, U.S. Pat. Nos. 5,525,625; 6,251,943; 7,820,664; 6,809,106; 7,759,518; 7,485,643; 7,576,072; 7,923,456; 7,732,616; 7,271,178; 7,429,667; 6,649,640; 6,495,582; 7,001,905; US Patent Publication No. US2010/0331334, US2009/0143389, US2008/0280957, US2007/0049591, US2011/0118298, International Patent Application Publication No. WO98/43960, WO99/01421, WO99/01426, WO00/41505, WO00/42002, WO00/42003, WO00/41994, WO00/42022, WO00/42029, WO00/68201, WO01/68619, WO02/06213 and WO03/077914.

Malignant melanomas are staged according to the American Joint Committee on Cancer (AJCC) TNM-classification system, where Clark level is considered in T-classification. The T stage describes the local extent of the primary tumor, i.e., how far the tumor has invaded and imposed growth into surrounding tissues, whereas the N stage and M stage describe how the tumor has developed metastases, with the N stage describing spread of tumor to lymph nodes and the M stage describing growth of tumor in other distant organs. Early stages include: T0-1, N0, M0, representing localized tumors with negative lymph nodes. More advanced stages include: T2-4, N0, M0, localized tumors with more widespread growth and T1-4, N1-3, M0, tumors that have metastasized to lymph nodes and T1-4, N1-3, M1, tumors with a metastasis detected in a distant organ.

Stages I and II represent no metastatic disease and for stage I (T1a/b-2a,N0,M0) prognosis is very good. The 5-year survival for stage I disease is 90-95%, for stage II (T2b-4-b,N0,M0) the corresponding survival rate ranges from 80 to 45%. Stages III (T1a-4-b,N1a-3,M0) and IV (T(all),N(all),M1a-c) represent spread disease, and for these stages 5-year survival rates range from 70 to 24%, and from 19 to 7%, respectively. "Clark's level" is a measure of the layers of skin involved in a melanoma and is a melanoma prognostic factor. For example, level I involves the epidermis. Level II involves the epidermis and upper dermis. Level III involves the epidermis, upper dermis, and lower dermis. Level IV involves the epidermis, upper dermis, lower dermis, and subcutis. When the primary tumor has a thickness of >1 mm, ulceration, or Clark level IV-V, sentinel node biopsy (SNB) is typically performed. SNB is performed by identifying the first draining lymph node/s (i.e., the SN) from the tumor. This is normally done by injection of radiolabelled colloid particles in the area around the tumor, followed by injection of Vital Blue dye. Rather than dissection of all regional lymph nodes, which was the earlier standard procedure, only the sentinel nodes are generally removed and carefully examined. Following complete lymph node dissection is only performed in confirmed positive cases.

In addition to staging and diagnosis, factors like T-stage, Clark level, SNB status, Breslow's depth, ulceration, and the like can be used as endpoints and/or surrogates for analyses according to the present invention. For example, patients who are diagnosed at an advanced stage with metastases generally have a poor prognosis. For patients diagnosed with a localized disease, the thickness of the tumor measured in mm (Breslow) and ulceration can be endpoints for prognosis. Breslow's depth is determined by using an ocular micrometer at a right angle to the skin. The depth from the granular layer of the epidermis to the deepest point of invasion to which tumor cells have invaded the skin is directly measured. Clark level is important for thin lesions (<1 mm). Other prognostic factors include age, anatomic site of the primary tumor and gender. The sentinel node (SN) status can also be a prognostic factor, especially since the 5-year survival of SN-negative patients has been shown to be as high as 90%. Similarly, overall survival (OS) can be used as a standard primary endpoint. OS takes in to account time to death, irrespective of cause, e.g. if the death is due to cancer or not. Loss to follow-up is censored and regional recurrence, distant metastases, second primary malignant melanomas and second other primary cancers are ignored. Other surrogate endpoints for survival can be used, as described further herein, such as disease-free survival (DFS), which includes time to any event related to the same cancer, i.e. all cancer recurrences and deaths from the same cancer are events.

In addition to endpoints, certain diagnostic and prognostic markers can be analyzed in conjunction with the methods described herein. For example, lactate dehydrogenase (LDH) can be measured as a marker for disease progression. Patients with distant metastases and elevated LDH levels belong to stage IV M1c. Another serum biomarker of interest is S100B. High S100B levels are associated with disease progression, and a decrease in the S100B level is an indicator of treatment response. Melanoma-inhibiting activity (MIA) is yet another serum biomarker that has been evaluated regarding its prognostic value. Studies have shown that elevated MIA levels are rare in stage I and II disease, whereas in stage III or IV, elevation in MIA levels can be seen in 60-100% of cases. Addition useful biomarkers include RGS1 (associated with reduced relapse-free survival (RFS)), osteopontin (associated with both reduced RFS and disease-specific survival (DSS), and predictive of SLN metastases), HER3 (associated with reduced survival), and NCOA3 (associated with poor RFS and DSS, and predictive of SLN metastases). In addition, HMB-45, Ki-67 (MIB1), MITF and MART-1/Melan-A or combinations of any described marker may be used for staining (Ivan & Prieto, 2010, Future Oncol. 6(7), 1163-1175; Linos et al., 2011, Biomarkers Med. 5(3) 333-360). In a literature review Rothberg et al. report that melanoma cell adhesion molecule (MCAM)/MUC18, matrix metalloproteinase-2, Ki-67, proliferating cell nuclear antigen (PCNA) and p16/INK4A are predictive of either all-cause mortality or melanoma specific mortality (Rothberg et al., 2009 J. Nat. Cane. Inst. 101(7) 452-474).

Currently, the typical primary treatment of malignant melanoma is radical surgery. Even though survival rates are high after excision of the primary tumor, melanomas tend to metastasize relatively early, and for patients with metastatic melanoma the prognosis is poor, with a 5-year survival rate of less than 10%. Radical removal of distant metastases with surgery can be an option and systemic chemotherapy can be applied, but response rates are normally low (in most cases less than 20%), and most treatment regiments fail to prolong overall survival. The first FDA-approved chemotherapeutic agent for treatment of metastatic melanoma was dacarbazine (DTIC), which can give response rates of approximately 20%, but where less than 5% may be complete responses. Temozolamid is an analog of DTIC that has the advantage of oral administration, and which have been shown to give a similar response as DTIC. Other chemotherapeutic agents, for example different nitrosureas, cisplatin, carboplatin, and vinca alkaloids, have been used, but without any increase in response rates. Since chemotherapy is an inefficient treatment method, immunotherapy agents have also been proposed. Most studied are interferon-alpha and interleukin-2. As single agents they have not been shown to give a better response than conventional treatment, but in combination with chemotherapeutic agents higher response rates have been reported. For patients with resected stage IIB or III melanoma, some studies have shown that adjuvant interferon alfa has led to longer disease free survival. For first- or second-line stage III and IV melanoma systemic treatments include: carboplatin, cisplatin, dacarbazine, interferon alfa, high-dose interleukin-2, paclitaxel, temozolomide, vinblastine or combinations thereof (NCCN Guidelines, ME-D, MS-9-13). Recently, the FDA approved Zelboraf^{™} (vemurafenib, also known as INN, PLX4032, RG7204 or R05185426) for unresectable or metastatic melanoma with the BRAF V600E mutation (Bollag et al. (2010) Nature 467:596-599 and Chapman et al. (2011) New Eng. J. Med. 364:2507-2516). Another recently approved drug for unresectable or metastatic melanoma is Yervoy^{®} (ipilimumab) an antibody which binds to cytotoxic T-lymphocyte-associated antigen 4 (CTLA-4) (Hodi et al. (2010) New Eng. J. Med. 363:711-723). Others recently reported that patients with KIT receptor activating mutations or over-expression responded to Gleevac^{®} (imatinib mesylate) (Carvajal et al. (2011) JAMA 305:2327-2334). In addition, radiation treatment may be given as an adjuvant after removal of lymphatic metastases, but malignant melanomas are relatively radioresistant. Radiation treatment might also be used as palliative treatment. Melanoma oncologists have also noted that BRAF mutations are common in both primary and metastatic melanomas and that these mutations are reported to be present in 50-70% of all melanomas. This has led to an interest in B-raf inhibitors, such as Sorafenib, as therapeutic agents.

The term "coding region" refers to regions of a nucleotide sequence comprising codons which are translated into amino acid residues, whereas the term "noncoding region" refers to regions of a nucleotide sequence that are not translated into amino acids (*e.g*., 5' and 3' untranslated regions).

The term "complementary" refers to the broad concept of sequence complementarity between regions of two nucleic acid strands or between two regions of the same nucleic acid strand. It is known that an adenine residue of a first nucleic acid region is capable of forming specific hydrogen bonds ("base pairing") with a residue of a second nucleic acid region which is antiparallel to the first region if the residue is thymine or uracil. Similarly, it is known that a cytosine residue of a first nucleic acid strand is capable of base pairing with a residue of a second nucleic acid strand which is antiparallel to the first strand if the residue is guanine. A first region of a nucleic acid is complementary to a second region of the same or a different nucleic acid if, when the two regions are arranged in an antiparallel fashion, at least one nucleotide residue of the first region is capable of base pairing with a residue of the second region. Preferably, the first region comprises a first portion and the second region comprises a second portion, whereby, when the first and second portions are arranged in an antiparallel fashion, at least about 50%, and preferably at least about 75%, at least about 90%, or at least about 95% of the nucleotide residues of the first portion are capable of base pairing with nucleotide residues in the second portion. More preferably, all nucleotide residues of the first portion are capable of base pairing with nucleotide residues in the second portion.

The terms "conjoint therapy" and "combination therapy," as used herein, refer to the administration of two or more therapeutic substances, e.g., an anti-immune checkpoint therapy or inhibitor of an immune checkpoint, and another inhibitor of Ang-2/Tie-2. The inhibitor of Ang-2/Tie-2 may be administered concomitant with, prior to, or following the administration of an inhibitor of an immune checkpoint.

The term "control" refers to any reference standard suitable to provide a comparison to the expression products in the test sample. In one embodiment, the control comprises obtaining a "control sample" from which expression product levels are detected and compared to the expression product levels from the test sample. Such a control sample may comprise any suitable sample, including but not limited to a sample from a control cancer patient (can be stored sample or previous sample measurement) with a known outcome; normal tissue or cells isolated from a subject, such as a normal patient or the cancer patient, cultured primary cells/tissues isolated from a subject such as a normal subject or the cancer patient, adjacent normal cells/tissues obtained from the same organ or body location of the cancer patient, a tissue or cell sample isolated from a normal subject, or a primary cells/tissues obtained from a depository. In another preferred embodiment, the control may comprise a reference standard expression product level from any suitable source, including but not limited to housekeeping genes, an expression product level range from normal tissue or other previously analyzed control sample), a previously determined expression product level range within a test sample from a group of patients, or a set of patients with a certain outcome (for example, survival for one, two, three, four years, etc.) or receiving a certain treatment (for example, standard of care cancer therapy). It will be understood by those of skill in the art that such control samples and reference standard expression product levels can be used in combination as controls in the methods of the present invention. In one embodiment, the control may comprise normal or non-cancerous cell/tissue sample. In another preferred embodiment, the control may comprise an expression level for a set of patients, such as a set of cancer patients, or for a set of cancer patients receiving a certain treatment, or for a set of patients with one outcome versus another outcome. In the former case, the specific expression product level of each patient can be assigned to a percentile level of expression, or expressed as either higher or lower than the mean or average of the reference standard expression level. In another preferred embodiment, the control may comprise normal cells, cells from patients treated with combination chemotherapy, and cells from patients having benign cancer. In another embodiment, the control may also comprise a measured value for example, average level of expression of a particular gene in a population compared to the level of expression of a housekeeping gene in the same population. Such a population may comprise normal subjects, cancer patients who have not undergone any treatment (i.e., treatment naive), cancer patients undergoing standard of care therapy, or patients having benign cancer. In another preferred embodiment, the control comprises a ratio transformation of expression product levels, including but not limited to determining a ratio of expression product levels of two genes in the test sample and comparing it to any suitable ratio of the same two genes in a reference standard; determining expression product levels of the two or more genes in the test sample and determining a difference in expression product levels in any suitable control; and determining expression product levels of the two or more genes in the test sample, normalizing their expression to expression of housekeeping genes in the test sample, and comparing to any suitable control. In particularly preferred embodiments, the control comprises a control sample which is of the same lineage and/or type as the test sample. In another embodiment, the control may comprise expression product levels grouped as percentiles within or based on a set of patient samples, such as all patients with cancer. In one embodiment a control expression product level is established wherein higher or lower levels of expression product relative to, for instance, a particular percentile, are used as the basis for predicting outcome. In another preferred embodiment, a control expression product level is established using expression product levels from cancer control patients with a known outcome, and the expression product levels from the test sample are compared to the control expression product level as the basis for predicting outcome. As demonstrated by the data below, the methods of the present invention are not limited to use of a specific cut-point in comparing the level of expression product in the test sample to the control.

The "copy number" of a biomarker nucleic acid refers to the number of DNA sequences in a cell (e.g., germline and/or somatic) encoding a particular gene product. Generally, for a given gene, a mammal has two copies of each gene. The copy number can be increased, however, by gene amplification or duplication, or reduced by deletion. For example, germline copy number changes include changes at one or more genomic loci, wherein said one or more genomic loci are not accounted for by the number of copies in the normal complement of germline copies in a control (e.g., the normal copy number in germline DNA for the same species as that from which the specific germline DNA and corresponding copy number were determined). Somatic copy number changes include changes at one or more genomic loci, wherein said one or more genomic loci are not accounted for by the number of copies in germline DNA of a control (e.g., copy number in germline DNA for the same subject as that from which the somatic DNA and corresponding copy number were determined).

The "normal" copy number (e.g., germline and/or somatic) of a biomarker nucleic acid or "normal" level of expression of a biomarker nucleic acid or protein is the activity/level of expression or copy number in a biological sample, *e.g.,* a sample containing tissue, whole blood, serum, plasma, buccal scrape, saliva, cerebrospinal fluid, urine, stool, and bone marrow, from a subject, *e.g.,* a human, not afflicted with cancer, or from a corresponding non-cancerous tissue in the same subject who has cancer.

As used herein, the term "costimulate" with reference to activated immune cells includes the ability of a costimulatory molecule to provide a second, non-activating receptor mediated signal (a "costimulatory signal") that induces proliferation or effector function. For example, a costimulatory signal can result in cytokine secretion, e.g., in a T cell that has received a T cell-receptor-mediated signal. Immune cells that have received a cell-receptor mediated signal, e.g., via an activating receptor are referred to herein as "activated immune cells."

The term "determining a suitable treatment regimen for the subject" is taken to mean the determination of a treatment regimen (*i.e.,* a single therapy or a combination of different therapies that are used for the prevention and/or treatment of the cancer in the subject) for a subject that is started, modified and/or ended based or essentially based or at least partially based on the results of the analysis according to the present invention. One example is determining whether to provide targeted therapy against a cancer to provide immunotherapy that generally increases immune responses against the cancer (e.g., anti-immune checkpoint therapy). Another example is starting an adjuvant therapy after surgery whose purpose is to decrease the risk of recurrence, another would be to modify the dosage of a particular chemotherapy. The determination can, in addition to the results of the analysis according to the present invention, be based on personal characteristics of the subject to be treated. In most cases, the actual determination of the suitable treatment regimen for the subject will be performed by the attending physician or doctor.

The term "diagnosing cancer" includes the use of the methods, systems, and code of the present invention to determine the presence or absence of a cancer or subtype thereof in an individual. The term also includes methods, systems, and code for assessing the level of disease activity in an individual.

A molecule is "fixed" or "affixed" to a substrate if it is covalently or non-covalently associated with the substrate such that the substrate can be rinsed with a fluid (e.g. standard saline citrate, pH 7.4) without a substantial fraction of the molecule dissociating from the substrate.

The term "expression signature" or "signature" refers to a group of two or more coordinately expressed biomarkers. For example, the genes, proteins, metabolites, and the like making up this signature may be expressed in a specific cell lineage, stage of differentiation, or during a particular biological response. The biomarkers can reflect biological aspects of the tumors in which they are expressed, such as the cell of origin of the cancer, the nature of the non-malignant cells in the biopsy, and the oncogenic mechanisms responsible for the cancer. Expression data and gene expression levels can be stored on computer readable media, *e.g.,* the computer readable medium used in conjunction with a microarray or chip reading device. Such expression data can be manipulated to generate expression signatures.

"Homologous" as used herein, refers to nucleotide sequence similarity between two regions of the same nucleic acid strand or between regions of two different nucleic acid strands. When a nucleotide residue position in both regions is occupied by the same nucleotide residue, then the regions are homologous at that position. A first region is homologous to a second region if at least one nucleotide residue position of each region is occupied by the same residue. Homology between two regions is expressed in terms of the proportion of nucleotide residue positions of the two regions that are occupied by the same nucleotide residue. By way of example, a region having the nucleotide sequence 5'-ATTGCC-3' and a region having the nucleotide sequence 5'-TATGGC-3' share 50% homology. Preferably, the first region comprises a first portion and the second region comprises a second portion, whereby, at least about 50%, and preferably at least about 75%, at least about 90%, or at least about 95% of the nucleotide residue positions of each of the portions are occupied by the same nucleotide residue. More preferably, all nucleotide residue positions of each of the portions are occupied by the same nucleotide residue.

The term "immune cell" refers to cells that play a role in the immune response. Immune cells are of hematopoietic origin, and include lymphocytes, such as B cells and T cells; natural killer cells; myeloid cells, such as monocytes, macrophages, eosinophils, mast cells, basophils, and granulocytes.

The term "immune checkpoint" refers to a group of molecules on the cell surface of CD4+ and/or CD8+ T cells that fine-tune immune responses by down-modulating or inhibiting an anti-tumor immune response. Immune checkpoint proteins are well known in the art and include, without limitation, CTLA-4, PD-1, VISTA, B7-H2, B7-H3, PD-L1, B7-H4, B7-H6, 2B4, ICOS, HVEM, PD-L2, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, and A2aR (see, for example, WO 2012/177624). The term further encompasses biologically active protein fragment, as well as nucleic acids encoding full-length immune checkpoint proteins and biologically active protein fragments thereof. In some embodiment, the term further encompasses any fragment according to homology descriptions provided herein.

"Anti-immune checkpoint therapy" refers to the use of agents that inhibit immune checkpoint nucleic acids and/or proteins. Inhibition of one or more immune checkpoints can block or otherwise neutralize inhibitory signaling to thereby upregulate an immune response in order to more efficaciously treat cancer. Exemplary agents useful for inhibiting immune checkpoints include antibodies, small molecules, peptides, peptidomimetics, natural ligands, and derivatives of natural ligands, that can either bind and/or inactivate or inhibit immune checkpoint proteins, or fragments thereof; as well as RNA interference, antisense, nucleic acid aptamers, etc. that can downregulate the expression and/or activity of immune checkpoint nucleic acids, or fragments thereof. Exemplary agents for upregulating an immune response include antibodies against one or more immune checkpoint proteins block the interaction between the proteins and its natural receptor(s); a non-activating form of one or more immune checkpoint proteins (e.g., a dominant negative polypeptide); small molecules or peptides that block the interaction between one or more immune checkpoint proteins and its natural receptor(s); fusion proteins (*e.g*. the extracellular portion of an immune checkpoint inhibition protein fused to the Fc portion of an antibody or immunoglobulin) that bind to its natural receptor(s); nucleic acid molecules that block immune checkpoint nucleic acid transcription or translation; and the like. Such agents can directly block the interaction between the one or more immune checkpoints and its natural receptor(s) (e.g., antibodies) to prevent inhibitory signaling and upregulate an immune response. Alternatively, agents can indirectly block the interaction between one or more immune checkpoint proteins and its natural receptor(s) to prevent inhibitory signaling and upregulate an immune response. For example, a soluble version of an immune checkpoint protein ligand such as a stabilized extracellular domain can binding to its receptor to indirectly reduce the effective concentration of the receptor to bind to an appropriate ligand. In one embodiment, anti-PD-1 antibodies, anti-PD-L1 antibodies, and anti-CTLA-4 antibodies, either alone or in combination, are used to inhibit immune checkpoints.

"Ipilimumab" is a representative example of an anti-immune checkpoint therapy. Ipilimumab (previously MDX-010; Medarex Inc., marketed by Bristol-Myers Squibb as YERVOY^{™}) is a fully human anti-human CTLA-4 monoclonal antibody that blocks the binding of CTLA-4 to CD80 and CD86 expressed on antigen presenting cells, thereby, blocking the negative down-regulation of the immune responses elicited by the interaction of these molecules (see, for example, WO 2013/169971, U.S. Pat. Publ. 2002/0086014, and U.S. Pat. Publ. 2003/0086930.

The term "immune response" includes T cell mediated and/or B cell mediated immune responses. Exemplary immune responses include T cell responses, *e.g.,* cytokine production and cellular cytotoxicity. In addition, the term immune response includes immune responses that are indirectly effected by T cell activation, *e.g.,* antibody production (humoral responses) and activation of cytokine responsive cells, *e.g.,* macrophages.

The term "immunotherapeutic agent" can include any molecule, peptide, antibody or other agent which can stimulate a host immune system to generate an immune response to a tumor or cancer in the subject. Various immunotherapeutic agents are useful in the compositions and methods described herein.

The term "inhibit" includes the decrease, limitation, or blockage, of, for example a particular action, function, or interaction. In some embodiments, cancer is "inhibited" if at least one symptom of the cancer is alleviated, terminated, slowed, or prevented. As used herein, cancer is also "inhibited" if recurrence or metastasis of the cancer is reduced, slowed, delayed, or prevented.

The term "interaction", when referring to an interaction between two molecules, refers to the physical contact (e.g., binding) of the molecules with one another. Generally, such an interaction results in an activity (which produces a biological effect) of one or both of said molecules.

An "isolated protein" refers to a protein that is substantially free of other proteins, cellular material, separation medium, and culture medium when isolated from cells or produced by recombinant DNA techniques, or chemical precursors or other chemicals when chemically synthesized. An "isolated" or "purified" protein or biologically active portion thereof is substantially free of cellular material or other contaminating proteins from the cell or tissue source from which the antibody, polypeptide, peptide or fusion protein is derived, or substantially free from chemical precursors or other chemicals when chemically synthesized. The language "substantially free of cellular material" includes preparations of a biomarker polypeptide or fragment thereof, in which the protein is separated from cellular components of the cells from which it is isolated or recombinantly produced. In one embodiment, the language "substantially free of cellular material" includes preparations of a biomarker protein or fragment thereof, having less than about 30% (by dry weight) of non-biomarker protein (also referred to herein as a "contaminating protein"), more preferably less than about 20% of non-biomarker protein, still more preferably less than about 10% of non-biomarker protein, and most preferably less than about 5% non-biomarker protein. When antibody, polypeptide, peptide or fusion protein or fragment thereof, e.g., a biologically active fragment thereof, is recombinantly produced, it is also preferably substantially free of culture medium, *i.e.,* culture medium represents less than about 20%, more preferably less than about 10%, and most preferably less than about 5% of the volume of the protein preparation.

A "kit" is any manufacture (e.g. a package or container) comprising at least one reagent, *e.g.* a probe or small molecule, for specifically detecting and/or affecting the expression of a marker of the present invention. The kit may be promoted, distributed, or sold as a unit for performing the methods of the present invention. The kit may comprise one or more reagents necessary to express a composition useful in the methods of the present invention. In certain embodiments, the kit may further comprise a reference standard, *e.g.,* a nucleic acid encoding a protein that does not affect or regulate signaling pathways controlling cell growth, division, migration, survival or apoptosis. One skilled in the art can envision many such control proteins, including, but not limited to, common molecular tags *(e.g.,* green fluorescent protein and beta-galactosidase), proteins not classified in any of pathway encompassing cell growth, division, migration, survival or apoptosis by GeneOntology reference, or ubiquitous housekeeping proteins. Reagents in the kit may be provided in individual containers or as mixtures of two or more reagents in a single container. In addition, instructional materials which describe the use of the compositions within the kit can be included.

The term "neoadjuvant therapy" refers to a treatment given before the primary treatment. Examples of neoadjuvant therapy can include chemotherapy, radiation therapy, and hormone therapy. For example, in treating breast cancer, neoadjuvant therapy can allows patients with large breast cancer to undergo breast-conserving surgery.

The "normal" level of expression of a biomarker is the level of expression of the biomarker in cells of a subject, e.g., a human patient, not afflicted with a cancer. An "over-expression" or "significantly higher level of expression" of a biomarker refers to an expression level in a test sample that is greater than the standard error of the assay employed to assess expression, and is preferably at least 10%, and more preferably 1.2, 1.3, 1.4, 1.5, 1.6, 1.7, 1.8, 1.9, 2.0, 2.1, 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more higher than the expression activity or level of the biomarker in a control sample (e.g., sample from a healthy subject not having the biomarker associated disease) and preferably, the average expression level of the biomarker in several control samples. A "significantly lower level of expression" of a biomarker refers to an expression level in a test sample that is at least 10%, and more preferably 1.2, 1.3, 1.4, 1.5, 1.6, 1.7, 1.8, 1.9, 2.0, 2.1, 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more lower than the expression level of the biomarker in a control sample (e.g., sample from a healthy subject not having the biomarker associated disease) and preferably, the average expression level of the biomarker in several control samples.

An "over-expression" or "significantly higher level of expression" of a biomarker refers to an expression level in a test sample that is greater than the standard error of the assay employed to assess expression, and is preferably at least 10%, and more preferably 1.2, 1.3, 1.4, 1.5, 1.6, 1.7, 1.8, 1.9, 2.0, 2.1, 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more higher than the expression activity or level of the biomarker in a control sample (e.g., sample from a healthy subject not having the biomarker associated disease) and preferably, the average expression level of the biomarker in several control samples. A "significantly lower level of expression" of a biomarker refers to an expression level in a test sample that is at least 10%, and more preferably 1.2, 1.3, 1.4, 1.5, 1.6, 1.7, 1.8, 1.9, 2.0, 2.1, 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more lower than the expression level of the biomarker in a control sample (e.g., sample from a healthy subject not having the biomarker associated disease) and preferably, the average expression level of the biomarker in several control samples.

The term "pre-determined" biomarker amount and/or activity measurement(s) may be a biomarker amount and/or activity measurement(s) used to, by way of example only, evaluate a subject that may be selected for a particular treatment, evaluate a response to a treatment such as an anti-immune checkpoint inhibitor therapy, and/or evaluate the disease state. A pre-determined biomarker amount and/or activity measurement(s) may be determined in populations of patients with or without cancer. The pre-determined biomarker amount and/or activity measurement(s) can be a single number, equally applicable to every patient, or the pre-determined biomarker amount and/or activity measurement(s) can vary according to specific subpopulations of patients. Age, weight, height, and other factors of a subject may affect the pre-determined biomarker amount and/or activity measurement(s) of the individual. Furthermore, the pre-determined biomarker amount and/or activity can be determined for each subject individually. In one embodiment, the amounts determined and/or compared in a method described herein are based on absolute measurements. In another embodiment, the amounts determined and/or compared in a method described herein are based on relative measurements, such as ratios (e.g., serum biomarker normalized to the expression of a housekeeping or otherwise generally constant biomarker). The pre-determined biomarker amount and/or activity measurement(s) can be any suitable standard. For example, the pre-determined biomarker amount and/or activity measurement(s) can be obtained from the same or a different human for whom a patient selection is being assessed. In one embodiment, the pre-determined biomarker amount and/or activity measurement(s) can be obtained from a previous assessment of the same patient. In such a manner, the progress of the selection of the patient can be monitored over time. In addition, the control can be obtained from an assessment of another human or multiple humans, e.g., selected groups of humans, if the subject is a human. In such a manner, the extent of the selection of the human for whom selection is being assessed can be compared to suitable other humans, *e.g.,* other humans who are in a similar situation to the human of interest, such as those suffering from similar or the same condition(s) and/or of the same ethnic group.

The term "predictive" includes the use of a biomarker nucleic acid and/or protein status, e.g., over- or under- activity, emergence, expression, growth, remission, recurrence or resistance of tumors before, during or after therapy, for determining the likelihood of response of a cancer to anti-immune checkpoint treatment (e.g., therapeutic antibodies against CTLA-4, PD-1, PD-L1, and the like). Such predictive use of the biomarker may be confirmed by, *e.g.,* (1) increased or decreased copy number (e.g., by FISH, FISH plus SKY, single-molecule sequencing, e.g., as described in the art at least at J. Biotechnol., 86:289-301, or qPCR), overexpression or underexpression of a biomarker nucleic acid *(e.g.,* by ISH, Northern Blot, or qPCR), increased or decreased biomarker protein (e.g., by IHC), or increased or decreased activity, e.g., in more than about 5%, 6%, 7%, 8%, 9%, 10%, 11%, 12%, 13%, 14%, 15%, 20%, 25%, 30%, 40%, 50%, 60%, 70%, 80%, 90%, 95%, 100%, or more of assayed human cancers types or cancer samples; (2) its absolute or relatively modulated presence or absence in a biological sample, *e.g.,* a sample containing tissue, whole blood, serum, plasma, buccal scrape, saliva, cerebrospinal fluid, urine, stool, or bone marrow, from a subject, *e.g.* a human, afflicted with cancer; (3) its absolute or relatively modulated presence or absence in clinical subset of patients with cancer (e.g., those responding to a particular anti-immune checkpoint therapy or those developing resistance thereto).

The term "pre-malignant lesions" as described herein refers to a lesion that, while not cancerous, has potential for becoming cancerous. It also includes the term "pre-malignant disorders" or "potentially malignant disorders." In particular this refers to a benign, morphologically and/or histologically altered tissue that has a greater than normal risk of malignant transformation, and a disease or a patient's habit that does not necessarily alter the clinical appearance of local tissue but is associated with a greater than normal risk of precancerous lesion or cancer development in that tissue (leukoplakia, erythroplakia, erytroleukoplakia lichen planus (lichenoid reaction) and any lesion or an area which histological examination showed atypia of cells or dysplasia.

The terms "prevent," "preventing," "prevention," "prophylactic treatment," and the like refer to reducing the probability of developing a disease, disorder, or condition in a subject, who does not have, but is at risk of or susceptible to developing a disease, disorder, or condition.

The term "probe" refers to any molecule which is capable of selectively binding to a specifically intended target molecule, for example, a nucleotide transcript or protein encoded by or corresponding to a biomarker nucleic acid. Probes can be either synthesized by one skilled in the art, or derived from appropriate biological preparations. For purposes of detection of the target molecule, probes may be specifically designed to be labeled, as described herein. Examples of molecules that can be utilized as probes include, but are not limited to, RNA, DNA, proteins, antibodies, and organic molecules.

The term "prognosis" includes a prediction of the probable course and outcome of cancer or the likelihood of recovery from the disease. In some embodiments, the use of statistical algorithms provides a prognosis of cancer in an individual. For example, the prognosis can be surgery, development of a clinical subtype of cancer (e.g., solid tumors, such as lung cancer, melanoma, and renal cell carcinoma), development of one or more clinical factors, development of intestinal cancer, or recovery from the disease.

The term "response to anti-immune checkpoint therapy" relates to any response of the hyperproliferative disorder (e.g., cancer) to an anti-immune checkpoint therapy, such as anti-immune checkpoint therapy, preferably to a change in tumor mass and/or volume after initiation of neoadjuvant or adjuvant chemotherapy. Hyperproliferative disorder response may be assessed , for example for efficacy or in a neoadjuvant or adjuvant situation, where the size of a tumor after systemic intervention can be compared to the initial size and dimensions as measured by CT, PET, mammogram, ultrasound or palpation. Responses may also be assessed by caliper measurement or pathological examination of the tumor after biopsy or surgical resection. Response may be recorded in a quantitative fashion like percentage change in tumor volume or in a qualitative fashion like "pathological complete response" (pCR), "clinical complete remission" (cCR), "clinical partial remission" (cPR), "clinical stable disease" (cSD), "clinical progressive disease" (cPD) or other qualitative criteria. Assessment of hyperproliferative disorder response may be done early after the onset of neoadjuvant or adjuvant therapy, e.g., after a few hours, days, weeks or preferably after a few months. A typical endpoint for response assessment is upon termination of neoadjuvant chemotherapy or upon surgical removal of residual tumor cells and/or the tumor bed. This is typically three months after initiation of neoadjuvant therapy. In some embodiments, clinical efficacy of the therapeutic treatments described herein may be determined by measuring the clinical benefit rate (CBR). The clinical benefit rate is measured by determining the sum of the percentage of patients who are in complete remission (CR), the number of patients who are in partial remission (PR) and the number of patients having stable disease (SD) at a time point at least 6 months out from the end of therapy. The shorthand for this formula is CBR=CR+PR+SD over 6 months. In some embodiments, the CBR for a particular cancer therapeutic regimen is at least 25%, 30%, 35%, 40%, 45%, 50%, 55%, 60%, 65%, 70%, 75%, 80%, 85%, or more. Additional criteria for evaluating the response to cancer therapies are related to "survival," which includes all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point (e.g., time of diagnosis or start of treatment) and end point (e.g., death, recurrence or metastasis). In addition, criteria for efficacy of treatment can be expanded to include response to chemotherapy, probability of survival, probability of metastasis within a given time period, and probability of tumor recurrence. For example, in order to determine appropriate threshold values, a particular cancer therapeutic regimen can be administered to a population of subjects and the outcome can be correlated to biomarker measurements that were determined prior to administration of any cancer therapy. The outcome measurement may be pathologic response to therapy given in the neoadjuvant setting. Alternatively, outcome measures, such as overall survival and disease-free survival can be monitored over a period of time for subjects following cancer therapy for whom biomarker measurement values are known. In certain embodiments, the doses administered are standard doses known in the art for cancer therapeutic agents. The period of time for which subjects are monitored can vary. For example, subjects may be monitored for at least 2, 4, 6, 8, 10, 12, 14, 16, 18, 20, 25, 30, 35, 40, 45, 50, 55, or 60 months. Biomarker measurement threshold values that correlate to outcome of a cancer therapy can be determined using well-known methods in the art, such as those described in the Examples section.

The term "resistance" refers to an acquired or natural resistance of a cancer sample or a mammal to a cancer therapy ( i.e., being nonresponsive to or having reduced or limited response to the therapeutic treatment), such as having a reduced response to a therapeutic treatment by 25% or more, for example, 30%, 40%, 50%, 60%, 70%, 80%, or more, to 2-fold, 3-fold, 4-fold, 5-fold, 10-fold, 15-fold, 20-fold or more. The reduction in response can be measured by comparing with the same cancer sample or mammal before the resistance is acquired, or by comparing with a different cancer sample or a mammal who is known to have no resistance to the therapeutic treatment. A typical acquired resistance to chemotherapy is called "multidrug resistance." The multidrug resistance can be mediated by P-glycoprotein or can be mediated by other mechanisms, or it can occur when a mammal is infected with a multi-drug-resistant microorganism or a combination of microorganisms. The determination of resistance to a therapeutic treatment is routine in the art and within the skill of an ordinarily skilled clinician, for example, can be measured by cell proliferative assays and cell death assays as described herein as "sensitizing." In some embodiments, the term "reverses resistance" means that the use of a second agent in combination with a primary cancer therapy (e.g., chemotherapeutic or radiation therapy) is able to produce a significant decrease in tumor volume at a level of statistical significance (e.g., p<0.05) when compared to tumor volume of untreated tumor in the circumstance where the primary cancer therapy (e.g., chemotherapeutic or radiation therapy) alone is unable to produce a statistically significant decrease in tumor volume compared to tumor volume of untreated tumor. This generally applies to tumor volume measurements made at a time when the untreated tumor is growing log rhythmically.

The terms "response" or "responsiveness" refers to an anti-cancer response, e.g. in the sense of reduction of tumor size or inhibiting tumor growth. The terms can also refer to an improved prognosis, for example, as reflected by an increased time to recurrence, which is the period to first recurrence censoring for second primary cancer as a first event or death without evidence of recurrence, or an increased overall survival, which is the period from treatment to death from any cause. To respond or to have a response means there is a beneficial endpoint attained when exposed to a stimulus. Alternatively, a negative or detrimental symptom is minimized, mitigated or attenuated on exposure to a stimulus. It will be appreciated that evaluating the likelihood that a tumor or subject will exhibit a favorable response is equivalent to evaluating the likelihood that the tumor or subject will not exhibit favorable response (i.e., will exhibit a lack of response or be non-responsive).

An "RNA interfering agent" as used herein, is defined as any agent which interferes with or inhibits expression of a target biomarker gene by RNA interference (RNAi). Such RNA interfering agents include, but are not limited to, nucleic acid molecules including RNA molecules which are homologous to the target biomarker gene of the present invention, or a fragment thereof, short interfering RNA (siRNA), and small molecules which interfere with or inhibit expression of a target biomarker nucleic acid by RNA interference (RNAi).

"RNA interference (RNAi)" is an evolutionally conserved process whereby the expression or introduction of RNA of a sequence that is identical or highly similar to a target biomarker nucleic acid results in the sequence specific degradation or specific post-transcriptional gene silencing (PTGS) of messenger RNA (mRNA) transcribed from that targeted gene (see Coburn, G. and Cullen, B. (2002) J. of Virology 76(18):9225), thereby inhibiting expression of the target biomarker nucleic acid. In one embodiment, the RNA is double stranded RNA (dsRNA). This process has been described in plants, invertebrates, and mammalian cells. In nature, RNAi is initiated by the dsRNA-specific endonuclease Dicer, which promotes processive cleavage of long dsRNA into double-stranded fragments termed siRNAs. siRNAs are incorporated into a protein complex that recognizes and cleaves target mRNAs. RNAi can also be initiated by introducing nucleic acid molecules, e.g., synthetic siRNAs or RNA interfering agents, to inhibit or silence the expression of target biomarker nucleic acids. As used herein, "inhibition of target biomarker nucleic acid expression" or "inhibition of marker gene expression" includes any decrease in expression or protein activity or level of the target biomarker nucleic acid or protein encoded by the target biomarker nucleic acid. The decrease may be of at least 30%, 40%, 50%, 60%, 70%, 80%, 90%, 95% or 99% or more as compared to the expression of a target biomarker nucleic acid or the activity or level of the protein encoded by a target biomarker nucleic acid which has not been targeted by an RNA interfering agent.

The term "sample" used for detecting or determining the presence or level of at least one biomarker is typically whole blood, plasma, serum, saliva, urine, stool (e.g., feces), tears, and any other bodily fluid (e.g., as described above under the definition of "body fluids"), or a tissue sample (e.g., biopsy) such as a small intestine, colon sample, or surgical resection tissue. In certain instances, the method of the present invention further comprises obtaining the sample from the individual prior to detecting or determining the presence or level of at least one marker in the sample.

The term "sensitize" means to alter cancer cells or tumor cells in a way that allows for more effective treatment of the associated cancer with a cancer therapy (e.g., anti-immune checkpoint, chemotherapeutic, and/or radiation therapy). In some embodiments, normal cells are not affected to an extent that causes the normal cells to be unduly injured by the anti-immune checkpoint therapy. An increased sensitivity or a reduced sensitivity to a therapeutic treatment is measured according to a known method in the art for the particular treatment and methods described herein below, including, but not limited to, cell proliferative assays (Tanigawa N, Kern D H, Kikasa Y, Morton D L, Cancer Res 1982; 42: 2159-2164), cell death assays (Weisenthal L M, Shoemaker R H, Marsden J A, Dill P L, Baker J A, Moran E M, Cancer Res 1984; 94: 161-173; Weisenthal L M, Lippman M E, Cancer Treat Rep 1985; 69: 615-632; Weisenthal L M, In: Kaspers G J L, Pieters R, Twentyman P R, Weisenthal L M, Veerman A J P, eds. Drug Resistance in Leukemia and Lymphoma. Langhorne, P A: Harwood Academic Publishers, 1993: 415-432; Weisenthal L M, Contrib Gynecol Obstet 1994; 19: 82-90). The sensitivity or resistance may also be measured in animal by measuring the tumor size reduction over a period of time, for example, 6 month for human and 4-6 weeks for mouse. A composition or a method sensitizes response to a therapeutic treatment if the increase in treatment sensitivity or the reduction in resistance is 25% or more, for example, 30%, 40%, 50%, 60%, 70%, 80%, or more, to 2-fold, 3-fold, 4-fold, 5-fold, 10-fold, 15-fold, 20-fold or more, compared to treatment sensitivity or resistance in the absence of such composition or method. The determination of sensitivity or resistance to a therapeutic treatment is routine in the art and within the skill of an ordinarily skilled clinician. It is to be understood that any method described herein for enhancing the efficacy of a cancer therapy can be equally applied to methods for sensitizing hyperproliferative or otherwise cancerous cells (e.g., resistant cells) to the cancer therapy.

The term "synergistic effect" refers to the combined effect of two or more anti-immune checkpoint agents can be greater than the sum of the separate effects of the anticancer agents alone.

"Short interfering RNA" (siRNA), also referred to herein as "small interfering RNA" is defined as an agent which functions to inhibit expression of a target biomarker nucleic acid, *e.g.,* by RNAi. An siRNA may be chemically synthesized, may be produced by *in vitro* transcription, or may be produced within a host cell. In one embodiment, siRNA is a double stranded RNA (dsRNA) molecule of about 15 to about 40 nucleotides in length, preferably about 15 to about 28 nucleotides, more preferably about 19 to about 25 nucleotides in length, and more preferably about 19, 20, 21, or 22 nucleotides in length, and may contain a 3' and/or 5' overhang on each strand having a length of about 0, 1, 2, 3, 4, or 5 nucleotides. The length of the overhang is independent between the two strands, *i.e.,* the length of the overhang on one strand is not dependent on the length of the overhang on the second strand. Preferably the siRNA is capable of promoting RNA interference through degradation or specific post-transcriptional gene silencing (PTGS) of the target messenger RNA (mRNA).

In another embodiment, an siRNA is a small hairpin (also called stem loop) RNA (shRNA). In one embodiment, these shRNAs are composed of a short (e.g., 19-25 nucleotide) antisense strand, followed by a 5-9 nucleotide loop, and the analogous sense strand. Alternatively, the sense strand may precede the nucleotide loop structure and the antisense strand may follow. These shRNAs may be contained in plasmids, retroviruses, and lentiviruses and expressed from, for example, the pol III U6 promoter, or another promoter (see, *e.g.,* Stewart, et al. (2003) RNA Apr;9(4):493-501).

RNA interfering agents, e.g., siRNA molecules, may be administered to a patient having or at risk for having cancer, to inhibit expression of a biomarker gene which is overexpressed in cancer and thereby treat, prevent, or inhibit cancer in the subject.

The term "subject" refers to any healthy animal, mammal or human, or any animal, mammal or human afflicted with a cancer, *e.g.,* lung, ovarian, pancreatic, liver, breast, prostate, and colon carcinomas, as well as melanoma and multiple myeloma. The term "subject" is interchangeable with "patient."

The term "survival" includes all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point (*e.g.* time of diagnosis or start of treatment) and end point (*e.g.* death, recurrence or metastasis). In addition, criteria for efficacy of treatment can be expanded to include response to chemotherapy, probability of survival, probability of metastasis within a given time period, and probability of tumor recurrence.

The term "therapeutic effect" refers to a local or systemic effect in animals, particularly mammals, and more particularly humans, caused by a pharmacologically active substance. The term thus means any substance intended for use in the diagnosis, cure, mitigation, treatment or prevention of disease or in the enhancement of desirable physical or mental development and conditions in an animal or human. The phrase "therapeutically-effective amount" means that amount of such a substance that produces some desired local or systemic effect at a reasonable benefit/risk ratio applicable to any treatment. In certain embodiments, a therapeutically effective amount of a compound will depend on its therapeutic index, solubility, and the like. For example, certain compounds discovered by the methods of the present invention may be administered in a sufficient amount to produce a reasonable benefit/risk ratio applicable to such treatment.

The terms "therapeutically-effective amount" and "effective amount" as used herein means that amount of a compound, material, or composition comprising a compound of the present invention which is effective for producing some desired therapeutic effect in at least a sub-population of cells in an animal at a reasonable benefit/risk ratio applicable to any medical treatment. Toxicity and therapeutic efficacy of subject compounds may be determined by standard pharmaceutical procedures in cell cultures or experimental animals, *e.g.,* for determining the LD₅₀ and the ED₅₀. Compositions that exhibit large therapeutic indices are preferred. In some embodiments, the LD₅₀ (lethal dosage) can be measured and can be, for example, at least 10%, 20%, 30%, 40%, 50%, 60%, 70%, 80%, 90%, 100%, 200%, 300%, 400%, 500%, 600%, 700%, 800%, 900%, 1000% or more reduced for the agent relative to no administration of the agent. Similarly, the ED₅₀ (*i.e.,* the concentration which achieves a half-maximal inhibition of symptoms) can be measured and can be, for example, at least 10%, 20%, 30%, 40%, 50%, 60%, 70%, 80%, 90%, 100%, 200%, 300%, 400%, 500%, 600%, 700%, 800%, 900%, 1000% or more increased for the agent relative to no administration of the agent. Also, Similarly, the IC₅₀ (*i.e.,* the concentration which achieves half-maximal cytotoxic or cytostatic effect on cancer cells) can be measured and can be, for example, at least 10%, 20%, 30%, 40%, 50%, 60%, 70%, 80%, 90%, 100%, 200%, 300%, 400%, 500%, 600%, 700%, 800%, 900%, 1000% or more increased for the agent relative to no administration of the agent. In some embodiments, cancer cell growth in an assay can be inhibited by at least about 10%, 15%, 20%, 25%, 30%, 35%, 40%, 45%, 50%, 55%, 60%, 65%, 70%, 75%, 80%, 85%, 90%, 95%, or even 100%. In another embodiment, at least about a 10% , 15%, 20%, 25%, 30%, 35%, 40%, 45%, 50%, 55%, 60%, 65%, 70%, 75%, 80%, 85%, 90%, 95%, or even 100% decrease in a solid malignancy can be achieved.

A "transcribed polynucleotide" or "nucleotide transcript" is a polynucleotide (e.g. an mRNA, hnRNA, a cDNA, or an analog of such RNA or cDNA) which is complementary to or homologous with all or a portion of a mature mRNA made by transcription of a biomarker nucleic acid and normal post-transcriptional processing (e.g. splicing), if any, of the RNA transcript, and reverse transcription of the RNA transcript.

As used herein, the term "unresponsiveness" includes refractivity of immune cells to stimulation, e.g., stimulation via an activating receptor or a cytokine. Unresponsiveness can occur, e.g., because of exposure to immunosuppressants or exposure to high doses of antigen. As used herein, the term "anergy" or "tolerance" includes refractivity to activating receptor-mediated stimulation. Such refractivity is generally antigen-specific and persists after exposure to the tolerizing antigen has ceased. For example, anergy in T cells (as opposed to unresponsiveness) is characterized by lack of cytokine production, e.g., IL-2. T cell anergy occurs when T cells are exposed to antigen and receive a first signal (a T cell receptor or CD-3 mediated signal) in the absence of a second signal (a costimulatory signal). Under these conditions, reexposure of the cells to the same antigen (even if reexposure occurs in the presence of a costimulatory polypeptide) results in failure to produce cytokines and, thus, failure to proliferate. Anergic T cells can, however, proliferate if cultured with cytokines (e.g., IL-2). For example, T cell anergy can also be observed by the lack of IL-2 production by T lymphocytes as measured by ELISA or by a proliferation assay using an indicator cell line. Alternatively, a reporter gene construct can be used. For example, anergic T cells fail to initiate IL-2 gene transcription induced by a heterologous promoter under the control of the 5' IL-2 gene enhancer or by a multimer of the AP1 sequence that can be found within the enhancer (Kang et al. (1992) Science 257:1134).

There is a known and definite correspondence between the amino acid sequence of a particular protein and the nucleotide sequences that can code for the protein, as defined by the genetic code (shown below). Likewise, there is a known and definite correspondence between the nucleotide sequence of a particular nucleic acid and the amino acid sequence encoded by that nucleic acid, as defined by the genetic code.

### GENETIC CODE

- Alanine (Ala, A): GCA, GCC, GCG, GCT
- Arginine (Arg, R): AGA, ACG, CGA, CGC, CGG, CGT
- Asparagine (Asn, N): AAC, AAT
- Aspartic acid (Asp, D): GAC, GAT
- Cysteine (Cys, C): TGC, TGT
- Glutamic acid (Glu, E): GAA, GAG
- Glutamine (Gln, Q): CAA, CAG
- Glycine (Gly, G): GGA, GGC, GGG, GGT
- Histidine (His, H): CAC, CAT
- Isoleucine (Ile, I): ATA, ATC, ATT
- Leucine (Leu, L): CTA, CTC, CTG, CTT, TTA, TTG
- Lysine (Lys, K): AAA, AAG
- Methionine (Met, M): ATG
- Phenylalanine (Phe, F): TTC, TTT
- Proline (Pro, P): CCA, CCC, CCG, CCT
- Serine (Ser, S): AGC, AGT, TCA, TCC, TCG, TCT
- Threonine (Thr, T): ACA, ACC, ACG, ACT
- Tryptophan (Trp, W): TGG
- Tyrosine (Tyr, Y): TAC, TAT
- Valine (Val, V): GTA, GTC, GTG, GTT
- Termination signal (end): TAA, TAG, TGA

An important and well known feature of the genetic code is its redundancy, whereby, for most of the amino acids used to make proteins, more than one coding nucleotide triplet may be employed (illustrated above). Therefore, a number of different nucleotide sequences may code for a given amino acid sequence. Such nucleotide sequences are considered functionally equivalent since they result in the production of the same amino acid sequence in all organisms (although certain organisms may translate some sequences more efficiently than they do others). Moreover, occasionally, a methylated variant of a purine or pyrimidine may be found in a given nucleotide sequence. Such methylations do not affect the coding relationship between the trinucleotide codon and the corresponding amino acid.

In view of the foregoing, the nucleotide sequence of a DNA or RNA encoding a biomarker nucleic acid (or any portion thereof) can be used to derive the polypeptide amino acid sequence, using the genetic code to translate the DNA or RNA into an amino acid sequence. Likewise, for polypeptide amino acid sequence, corresponding nucleotide sequences that can encode the polypeptide can be deduced from the genetic code (which, because of its redundancy, will produce multiple nucleic acid sequences for any given amino acid sequence). Thus, description and/or disclosure herein of a nucleotide sequence which encodes a polypeptide should be considered to also include description and/or disclosure of the amino acid sequence encoded by the nucleotide sequence. Similarly, description and/or disclosure of a polypeptide amino acid sequence herein should be considered to also include description and/or disclosure of all possible nucleotide sequences that can encode the amino acid sequence.

Finally, nucleic acid and amino acid sequence information for the loci and biomarkers of the present invention (e.g., biomarkers listed in Table 1) are well known in the art and readily available on publicly available databases, such as the National Center for Biotechnology Information (NCBI). For example, exemplary nucleic acid and amino acid sequences derived from publicly available sequence databases are provided below.

**Table 1**

| | |
|---|---|
| SEQ ID NO: 1 | Human Ang-2 transcript variant 1 cDNA Sequence |
| | 1 atgtggcaga ttgttttctt tactctgagc tgtgatcttg tcttggccgc agcctataac 61 aactttcgga agagcatgga cagcatagga aagaagcaat atcaggtcca gcatgggtcc 121 tgcagctaca ctttcctcct gccagagatg gacaactgcc gctcttcctc cagcccctac 181 gtgtccaatg ctgtgcagag ggacgcgccg ctcgaatacg atgactcggt gcagaggctg 241 caagtgctgg agaacatcat ggaaaacaac actcagtggc taatgaagct tgagaattat 301 atccaggaca acatgaagaa agaaatggta gagatacagc agaatgcagt acagaaccag 361 acggctgtga tgatagaaat agggacaaac ctgttgaacc aaacagcgga gcaaacgcgg 421 aagttaactg atgtggaagc ccaagtatta aatcagacca cgagacttga acttcagctc 481 ttggaacact ccctctcgac aaacaaattg gaaaaacaga ttttggacca gaccagtgaa 541 ataaacaaat tgcaagataa gaacagtttc ctagaaaaga aggtgctagc tatggaagac 601 aagcacatca tccaactaca gtcaataaaa gaagagaaag atcagctaca ggtgttagta 661 tccaagcaaa attccatcat tgaagaacta gaaaaaaaaa tagtgactgc cacggtgaat 721 aattcagttc ttcagaagca gcaacatgat ctcatggaga cagttaataa cttactgact 781 atgatgtcca catcaaactc agctaaggac cccactgttg ctaaagaaga acaaatcagc 841 ttcagagact gtgctgaagt attcaaatca ggacacacca cgaatggcat ctacacgtta 901 acattcccta attctacaga agagatcaag gcctactgtg acatggaagc tggaggaggc 961 gggtggacaa ttattcagcg acgtgaggat ggcagcgttg attttcagag gacttggaaa 1021 gaatataaag tgggatttgg taacccttca ggagaatatt ggctgggaaa tgagtttgtt 1081 tcgcaactga ctaatcagca acgctatgtg cttaaaatac accttaaaga ctgggaaggg 1141 aatgaggctt actcattgta tgaacatttc tatctctcaa gtgaagaact caattatagg 1201 attcacctta aaggacttac agggacagcc ggcaaaataa gcagcatcag ccaaccagga 1261 aatgatttta gcacaaagga tggagacaac gacaaatgta tttgcaaatg ttcacaaatg 1321 ctaacaggag gctggtggtt tgatgcatgt ggtccttcca acttgaacgg aatgtactat 1381 ccacagaggc agaacacaaa taagttcaac ggcattaaat ggtactactg gaaaggctca 1441 ggctattcgc tcaaggccac aaccatgatg atccgaccag cagatttcta a |
| SEQ ID NO: 2 | Human Ang-2 isoform a Amino Acid Sequence |
| | 1 mwqivfftls cdlvlaaayn nfrksmdsig kkqyqvqhgs csytfllpem dncrsssspy 61 vsnavqrdap leyddsvqrl qvlenimenn tqwlmkleny iqdnmkkemv eiqqnavqnq 121 tavmieigtn llnqtaeqtr kltdveaqvl nqttrlelql lehslstnkl ekqildqtse 181 inklqdknsf lekkvlamed khiiqlqsik eekdqlqvlv skqnsiieel ekkivtatvn 241 nsvlqkqqhd lmetvnnllt mmstsnsakd ptvakeeqis frdcaevfks ghttngiytl 301 tfpnsteeik aycdmeaggg gwtiiqrred gsvdfqrtwk eykvgfgnps geywlgnefv 361 sqltnqqryv lkihlkdweg neayslyehf ylsseelnyr ihlkgltgta gkissisqpg 421 ndfstkdgdn dkcickcsqm ltggwwfdac gpsnlngmyy pqrqntnkfn gikwyywkgs 481 gyslkattmm irpadf |
| SEQ ID NO: 3 | Human Anq-2 transcript variant 2 cDNA Sequence |
| | 1 atgtggcaga ttgttttctt tactctgagc tgtgatcttg tcttggccgc agcctataac 61 aactttcgga agagcatgga cagcatagga aagaagcaat atcaggtcca gcatgggtcc 121 tgcagctaca ctttcctcct gccagagatg gacaactgcc gctcttcctc cagcccctac 181 gtgtccaatg ctgtgcagag ggacgcgccg ctcgaatacg atgactcggt gcagaggctg 241 caagtgctgg agaacatcat ggaaaacaac actcagtggc taatgaagct tgagaattat 301 atccaggaca acatgaagaa agaaatggta gagatacagc agaatgcagt acagaaccag 361 acggctgtga tgatagaaat agggacaaac ctgttgaacc aaacagcgga gcaaacgcgg 421 aagttaactg atgtggaagc ccaagtatta aatcagacca cgagacttga acttcagctc 481 ttggaacact ccctctcgac aaacaaattg gaaaaacaga ttttggacca gaccagtgaa 541 ataaacaaat tgcaagataa gaacagtttc ctagaaaaga aggtgctagc tatggaagac 601 aagcacatca tccaactaca gtcaataaaa gaagagaaag atcagctaca ggtgttagta 661 tccaagcaaa attccatcat tgaagaacta gaaaaaaaaa tagtgactgc cacggtgaat 721 aattcagttc ttcagaagca gcaacatgat ctcatggaga cagttaataa cttactgact 781 atgatgtcca catcaaactc taaggacccc actgttgcta aagaagaaca aatcagcttc 841 agagactgtg ctgaagtatt caaatcagga cacaccacga atggcatcta cacgttaaca 901 ttccctaatt ctacagaaga gatcaaggcc tactgtgaca tggaagctgg aggaggcggg 961 tggacaatta ttcagcgacg tgaggatggc agcgttgatt ttcagaggac ttggaaagaa 1021 tataaagtgg gatttggtaa cccttcagga gaatattggc tgggaaatga gtttgtttcg 1081 caactgacta atcagcaacg ctatgtgctt aaaatacacc ttaaagactg ggaagggaat 1141 gaggcttact cattgtatga acatttctat ctctcaagtg aagaactcaa ttataggatt 1201 caccttaaag gacttacagg gacagccggc aaaataagca gcatcagcca accaggaaat 1261 gattttagca caaaggatgg agacaacgac aaatgtattt gcaaatgttc acaaatgcta 1321 acaggaggct ggtggtttga tgcatgtggt ccttccaact tgaacggaat gtactatcca 1381 cagaggcaga acacaaataa gttcaacggc attaaatggt actactggaa aggctcaggc 1441 tattcgctca aggccacaac catgatgatc cgaccagcag atttctaa |
| SEQ ID NO: 4 | Human Ang-2 isoform b Amino Acid Sequence |
| | 1 mwqivfftls cdlvlaaayn nfrksmdsig kkqyqvqhgs csytfllpem dncrsssspy 61 vsnavqrdap leyddsvqrl qvlenimenn tqwlmkleny iqdnmkkemv eiqqnavqnq 121 tavmieigtn llnqtaeqtr kltdveaqvl nqttrlelql lehslstnkl ekqildqtse 181 inklqdknsf lekkvlamed khiiqlqsik eekdqlqvlv skqnsiieel ekkivtatvn 241 nsvlqkqqhd lmetvnnllt mmstsnskdp tvakeeqisf rdcaevfksg httngiytlt 301 fpnsteeika ycdmeagggg wtiiqrredg svdfqrtwke ykvgfgnpsg eywlgnefvs 361 qltnqqryvl kihlkdwegn eayslyehfy lsseelnyri hlkgltgtag kissisqpgn 421 dfstkdgdnd kcickcsqml tggwwfdacg psnlngmyyp qrqntnkfng ikwyywkgsg 481 yslkattmmi rpadf |
| SEQ ID NO: 5 | Human Anq-2 transcript variant 3 cDNA Sequence |
| | 1 atgtggcaga ttgttttctt tactctgagc tgtgatcttg tcttggccgc agcctataac 61 aactttcgga agagcatgga cagcatagga aagaagcaat atcaggtcca gcatgggtcc 121 tgcagctaca ctttcctcct gccagagatg gacaactgcc gctcttcctc cagcccctac 181 gtgtccaatg ctgtgcagag ggacgcgccg ctcgaatacg atgactcggt gcagaggctg 241 caagtgctgg agaacatcat ggaaaacaac actcagtggc taatgaaggt attaaatcag 301 accacgagac ttgaacttca gctcttggaa cactccctct cgacaaacaa attggaaaaa 361 cagattttgg accagaccag tgaaataaac aaattgcaag ataagaacag tttcctagaa 421 aagaaggtgc tagctatgga agacaagcac atcatccaac tacagtcaat aaaagaagag 481 aaagatcagc tacaggtgtt agtatccaag caaaattcca tcattgaaga actagaaaaa 541 aaaatagtga ctgccacggt gaataattca gttcttcaga agcagcaaca tgatctcatg 601 gagacagtta ataacttact gactatgatg tccacatcaa actcagctaa ggaccccact 661 gttgctaaag aagaacaaat cagcttcaga gactgtgctg aagtattcaa atcaggacac 721 accacgaatg gcatctacac gttaacattc cctaattcta cagaagagat caaggcctac 781 tgtgacatgg aagctggagg aggcgggtgg acaattattc agcgacgtga ggatggcagc 841 gttgatttcc agaggacttg gaaagaatat aaagtgggat ttggtaaccc ttcaggagaa 901 tattggctgg gaaatgagtt tgtttcgcaa ctgactaatc agcaacgcta tgtgcttaaa 961 atacacctta aagactggga agggaatgag gcttactcat tgtatgaaca tttctatctc 1021 tcaagtgaag aactcaatta taggattcac cttaaaggac ttacagggac agccggcaaa 1081 ataagcagca tcagccaacc aggaaatgat tttagcacaa aggatggaga caacgacaaa 1141 tgtatttgca aatgttcaca aatgctaaca ggaggctggt ggtttgatgc atgtggtcct 1201 tccaacttga acggaatgta ctatccacag aggcagaaca caaataagtt caacggcatt 1261 aaatggtact actggaaagg ctcaggctat tcgctcaagg ccacaaccat gatgatccga 1321 ccagcagatt tctaa |
| SEQ ID NO: 6 | Human Anq-2 isoform c Amino Acid Sequence |
| | 1 mwqivfftls cdlvlaaayn nfrksmdsig kkqyqvqhgs csytfllpem dncrsssspy 61 vsnavqrdap leyddsvqrl qvlenimenn tqwlmkvlnq ttrlelqlle hslstnklek 121 qildqtsein klqdknsfle kkvlamedkh iiqlqsikee kdqlqvlvsk qnsiieelek 181 kivtatvnns vlqkqqhdlm etvnnlltmm stsnsakdpt vakeeqisfr dcaevfksgh 241 ttngiytltf pnsteeikay cdmeaggggw tiiqrredgs vdfqrtwkey kvgfgnpsge 301 ywlgnefvsq ltnqqryvlk ihlkdwegne ayslyehfyl sseelnyrih lkgltgtagk 361 issisqpgnd fstkdgdndk cickcsqmlt ggwwfdacgp snlngmyypq rqntnkfngi 421 kwyywkgsgy slkattmmir padf |
| SEQ ID NO: 7 | Chimpanzee Anq-2 cDNA Sequence |
| | 1 atgtggcaga ttgttttctt tactctgagc tgtgatcttg tcttggccgc agcctataac 61 aactttcgga agagcatgga cagcatagga aagaagcaat atcaggtcca gcatgggtcc 121 tgcagctaca ctttcctcct gccagagatg gacaactgcc gctcttcctc cagcccctac 181 gtgtccaatg ctgtgcagag ggacgcgccg ctcgaatacg atgactcggt gcagaggctg 241 caagtgctgg agaacatcat ggaaaacaac actcagtggc taatgaaggt attaaatcag 301 accacgagac ttgaacttca gctcttggaa cactccctct cgacaaacaa attggaaaaa 361 cagattttgg accagaccag tgaaataaac aaattgcaag ataagaacag tttcctagaa 421 aagaaggtgc tagctatgga agacaagcac atcatccaac tacagtcaat aaaagaagag 481 aaagatcagc tacaggtgtt agtatccaag caaaattcca tcattgaaga actagaaaaa 541 aaaatagtga ctgccacggt gaataattca gttcttcaaa agcagcaaca tgatctcatg 601 gagacagtta ataacttact gactatgatg tccacatcaa actcagctaa ggaccccact 661 gttgctaaag aagaacaaat cagtttcaga gactgtgctg aagtattcaa atcaggacac 721 accacgaatg gcatctacac gttaacattc cctaattcta cagaagagat caaggcctac 781 tgtgacatgg aagctggagg aggcgggtgg acaattattc agcgacgtga ggatggcagc 841 gttgattttc agaggacttg gaaagaatat aaagtgggat ttggtaaccc ttcaggagaa |
| | 901 tattggctgg gaaatgagtt tgtttcgcaa ctgactaatc agcaacgcta tgtgcttaaa 961 atacacctta aagactggga agggaatgag gcttactcat tgtatgaaca cttctatctc 1021 tcaagtgaag aactcaatta taggattcac cttaaaggac ttacagggac agccggcaaa 1081 ataagcagca tcagccaacc aggaaatgat tttagcacaa aggatgcaga caacgacaaa 1141 tgtatttgca aatgttcaca aatgctaaca ggaggctggt ggtttgatgc atgtggtcct 1201 tccaacttga acggaatgta ctatccacaa aggcagaaca caaataagtt caacggcatt 1261 aaatggtact actggaaagg ctcaggctat tcgctcaagg ccacaaccat gatgatccga 1321 ccagcagatt tctga |
| SEQ ID NO: 8 | Chimpanzee Anq-2 Amino Acid Sequence |
| | 1 mwqivfftls cdlvlaaayn nfrksmdsig kkqyqvqhgs csytfllpem dncrsssspy 61 vsnavqrdap leyddsvqrl qvlenimenn tqwlmkvlnq ttrlelqlle hslstnklek 121 qildqtsein klqdknsfle kkvlamedkh iiqlqsikee kdqlqvlvsk qnsiieelek 181 kivtatvnns vlqkqqhdlm etvnnlltmm stsnsakdpt vakeeqisfr dcaevfksgh 241 ttngiytltf pnsteeikay cdmeaggggw tiiqrredgs vdfqrtwkey kvgfgnpsge 301 ywlgnefvsq ltnqqryvlk ihlkdwegne ayslyehfyl sseelnyrih lkgltgtagk 361 issisqpgnd fstkdadndk cickcsqmlt ggwwfdacgp snlngmyypq rqntnkfngi 421 kwyywkgsgy slkattmmir padf |
| SEQ ID NO: 9 | Doq Anq-2 cDNA Sequence |
| | 1 atgtggcaga ttgttttctt tactctgagc tgtgatcttg tccgagccgc agcctacaac 61 aattttcgga gaagcatgga cagcatcggg agaaggcagt atcaggttca gcacgggtcc 121 tgcagctaca cgttcctcct gccagaaaca gacaactgcc gctcccccgg ctcctacgtg 181 cccaacgccg tgcagaggga cgcgcctcta gattacgacg actcggtgca gcggctccaa 241 gtgttagaga acatcatgga gaataatacg cagtggctaa ttaagcttga gaattacata 301 caggacaaca tgaagaaaga gatggtagag atgcaacaga atgcagtaca gaaccagact 361 gccgtgatga tagaaattgg aacaaaccta ctaaatcaaa cagcagagca aacacggaag 421 ttaacagatg tcgaagcaca agtattaaat cagacaacaa gacttgaact tcagcttctg 481 gaacattccc tttccaccaa caaattggaa aaacagattt tggatcagac cagtgaaata 541 aacaaattgc aagataagaa cagtttcctg gaaaagaaag tactagacat ggaagacaag 601 catatagttc aactacggtc aataaaagaa gagaaagatc agctccaggt gttagtatcc 661 aagcaaaact ccatcattga agaactagaa aaacagttag tgaccgccac agtgaataat 721 tcggttctcc agaagcagca acatgatctg atggagacag ttcatagttt attgactatg 781 atatcaccat caaaatcacc taaggacacc tttgttgcta aggaagagca gatcatttac 841 agagactgtg cagaagtatt caagtcagga ctgacaacca atggcatcta tacgctaaca 901 ttccctaatt ccacagagga gataaaggca tactgcgaca tggaaacctc tggaggtggg 961 tggacggtca ttcagcggcg tgaagatggc agtgttgatt ttcagaggac ttggaaagaa 1021 tataaagtgg gatttgggaa cccttctggc gaacactggc tgggaaatga gtttgttttc 1081 caagtgacca atcaacagcc atatgtgctt aaaatacacc ttaaggactg ggagggaaac 1141 gaggcgtact cgctgtatga acatttctat ctctccggtg aagaactcaa ttacaggatt 1201 caccttaaag gacttacagg gacggccggc aaaataagca gcataagcca accaggaaat 1261 gattttagca caaaggatgc agacaacgac aaatgtattt gcaaatgttc acaaatgctc 1321 acaggaggct ggtggtttga tgcatgcggc ccctccaact tgaacggaat gtactacccg 1381 cagaggcaga acacaaataa gttcaacggc attaagtggt actactggaa aggctcaggc 1441 tactcgctca agggcacaac catgatgatc cgaccggcgg acttctag |
| SEQ ID NO: 10 | Doq Anq-2 Amino Acid Sequence |
| | 1 mwqivfftls cdlvraaayn nfrrsmdsig rrqyqvqhgs csytfllpet dncrspgsyv |
| | 61 pnavqrdapl dyddsvqrlq vlenimennt qwliklenyi qdnmkkemve mqqnavqnqt 121 avmieigtnl lnqtaeqtrk ltdveaqvln qttrlelqll ehslstnkle kqildqtsei 181 nklqdknsfl ekkvldmedk hivqlrsike ekdqlqvlvs kqnsiieele kqlvtatvnn 241 svlqkqqhdl metvhslltm ispskspkdt fvakeeqiiy rdcaevfksg lttngiytlt 301 fpnsteeika ycdmetsggg wtviqrredg svdfqrtwke ykvgfgnpsg ehwlgnefvf 361 qvtnqqpyvl kihlkdwegn eayslyehfy lsgeelnyri hlkgltgtag kissisqpgn 421 dfstkdadnd kcickcsqml tggwwfdacg psnlngmyyp qrqntnkfng ikwyywkgsg 481 yslkgttmmi rpadf |
| SEQ ID NO: 11 | Cow Anq-2 cDNA Sequence |
| | 1 atgtggcagc ttgttttctt gactctgagc tgtgatctgg ccgtagccac agcccacagc 61 ggctctcgga agggcatgga catcgccgct gggaagaaac agtatcaggt tcagcacgga 121 gcctgtagct acacgttcct cctgccggag acggaccact gccgctcgcc ctccagcgcc 181 tacgtgccta acgccgtgca gagggacgcg ccccttgact acgacgactc ggtgcagagg 241 ctgcaggtgc tggagaacat catggagaac aacacacagt ggctcatgaa gcttgagaat 301 tacatccagg acaacatgaa gaaagagatg gtggagatac aacagaatgc agtgcagaac 361 cagaccgctg tgatgataga aatagggacc aacctgctca atcaaaccgc agagcagacc 421 cggaagttaa ccgatgttga agcccaagta ttaaatcaga caacaagact tgaacttcag 481 cttctagaac actctctttc tacaaacaaa ttggaaaaac agattttgga tcagaccagt 541 gaaataagca aactgcaaga taagaacagt ttcctggaaa agaaagttct agatatggaa 601 gacaagcaca tagttcaact tcggtcaatc aaagaagaga aagatcagct ccaggtgtta 661 gtatccaagc aaaattccat cattgaagaa ctggaaaaac aactggtgac ggccacggtg 721 aataattcag ttctccaaaa gcagcaacat gatctgatgg agacagttaa taatttactg 781 actctgatgt caacatcaaa cccctcatac tccttgcttg ccaaggacga acaaatcatc 841 ttcagagact gcggggaggc attcaaatcg ggactgacca ccagcggtgt ctacacattg 901 acgttcccta actccacgga ggagatcaag gcttactgtg acatggaaac gggtggaggt 961 gggtggacag ttattcagcg acgtgaagac ggcagtgttg attttcagag gacttggaaa 1021 gaatataaag tgggattcgg gaacccttca ggtgaacact ggctgggaaa tgagtttgtt 1081 tcgcaggtga cgggtcagaa acgctacgtg ctgaaaatac acctaagaga ctgggaaggg 1141 aatgaggctt actcgctgta cgaccacttc tatctctcga atgaagaact caactacagg 1201 attcacctta aaggcctcac ggggacagcc ggcaaaataa gcagcataag ccagccagga 1261 aatgatttta gcacgaagga tgcagacaac gacaaatgca tttgcaaatg ctcacaaatg 1321 ctcacaggag gctggtggtt tgatgcctgt ggtccttcca acctgaatgg aatgtactac 1381 ccacagaggc agaacacaaa taagttcaac ggtattaagt ggtactactg gaagggctcc 1441 ggctactcac ttaaggccac gaccatgatg atccgtccag cagatttcta a |
| SEQ ID NO: 12 | Cow Anq-2 Amino Acid Sequence |
| | 1 mwqlvfltls cdlavatahs gsrkgmdiaa gkkqyqvqhg acsytfllpe tdhcrspssa 61 yvpnavqrda pldyddsvqr lqvlenimen ntqwlmklen yiqdnmkkem veiqqnavqn 121 qtavmieigt nllnqtaeqt rkltdveaqv lnqttrlelq llehslstnk lekqildqts 181 eisklqdkns flekkvldme dkhivqlrsi keekdqlqvl vskqnsiiee lekqlvtatv 241 nnsvlqkqqh dlmetvnnll tlmstsnpsy sllakdeqii frdcgeafks glttsgvytl 301 tfpnsteeik aycdmetggg gwtviqrred gsvdfqrtwk eykvgfgnps gehwlgnefv 361 sqvtgqkryv lkihlrdweg neayslydhf ylsneelnyr ihlkgltgta gkissisqpg 421 ndfstkdadn dkcickcsqm ltggwwfdac gpsnlngmyy pqrqntnkfn gikwyywkgs 481 gyslkattmm irpadf |
| SEQ ID NO: 13 | Mouse Anq-2 cDNA Sequence |
| | 1 atgtggcaga tcattttcct aacttttggc tgggatcttg tcttggcctc agcctacagt 61 aactttagga agagcgtgga cagcacaggc agaaggcagt accaggtcca gaacggaccc 121 tgcagctaca cgttcctgct gccggagacc gacagctgcc gatcttcctc cagcccctac 181 atgtccaatg ccgtgcagag ggatgcaccc ctcgactacg acgactcagt gcaaaggctg 241 caggtgctgg agaacattct agagaacaac acacagtggc tgatgaagct ggagaattac 301 attcaggaca acatgaagaa ggagatggtg gagatccaac agaatgtggt gcagaaccag 361 acagctgtga tgatagagat tggaaccagc ttgctgaacc agacagcagc acaaactcgg 421 aaactgactg atgtggaagc ccaagtacta aaccagacga caagactcga gctgcagctt 481 ctccaacatt ctatttctac caacaaattg gaaaagcaga ttttggatca gaccagtgaa 541 ataaacaagc tacaaaataa gaacagcttc ctagaacaga aagttctgga catggagggc 601 aagcacagcg agcagctaca gtccatgaag gagcagaagg acgagctcca ggtgctggtg 661 tccaagcaga gctctgtcat tgacgagctg gagaagaagc tggtgacagc cacggtcaac 721 aactcgctcc ttcagaagca gcagcatgac ctaatggaga ccgtcaacag cttgctgacc 781 atgatgtcat cacccaactc caagagctcg gttgctatcc gtaaagaaga gcaaaccacc 841 ttcagagact gtgcggaaat cttcaagtca ggactcacca ccagtggcat ctacacactg 901 accttcccca actccacaga ggagatcaag gcctactgtg acatggacgt gggtggagga 961 gggtggacag tcatccaaca ccgagaagat ggcagtgtgg acttccagag gacgtggaaa 1021 gaatacaaag agggcttcgg gagccctctg ggagagtact ggctgggcaa tgagtttgtc 1081 tcccagctga ccggtcagca ccgctacgtg cttaagatcc agctgaagga ctgggaaggc 1141 aacgaggcgc attcgctgta tgatcacttc tacctcgctg gtgaagagtc caactacagg 1201 attcacctta caggactcac ggggaccgcg ggcaaaataa gtagcatcag ccaaccagga 1261 agtgatttta gcacaaagga ttcggacaat gacaaatgca tctgcaagtg ttcccagatg 1321 ctctcaggag gctggtggtt tgacgcatgt ggtccttcca acttgaatgg acagtactac 1381 ccacaaaaac agaatacaaa taagtttaac ggtatcaagt ggtactactg gaaggggtcc 1441 ggctactcgc tcaaggccac aaccatgatg atccggccag cagatttcta a |
| SEQ ID NO: 14 | Mouse Anq-2 Amino Acid Sequence |
| | 1 mwqiifltfg wdlvlasays nfrksvdstg rrqyqvqngp csytfllpet dscrsssspy 61 msnavqrdap ldyddsvqrl qvlenilenn tqwlmkleny iqdnmkkemv eiqqnvvqnq 121 tavmieigts llnqtaaqtr kltdveaqvl nqttrlelql lqhsistnkl ekqildqtse 181 inklqnknsf leqkvldmeg khseqlqsmk eqkdelqvlv skqssvidel ekklvtatvn 241 nsllqkqqhd lmetvnsllt mmsspnskss vairkeeqtt frdcaeifks glttsgiytl 301 tfpnsteeik aycdmdvggg gwtviqhred gsvdfqrtwk eykegfgspl geywlgnefv 361 sqltgqhryv lkiqlkdweg neahslydhf ylageesnyr ihltgltgta gkissisqpg 421 sdfstkdsdn dkcickcsqm lsggwwfdac gpsnlngqyy pqkqntnkfn gikwyywkgs 481 gyslkattmm irpadf |
| SEQ ID NO: 15 | Rat Anq-2 cDNA Sequence |
| | 1 atgtggcaga ttgttttcct aacttttggc tgtgatcttg tcttggcctc agcctacaat 61 aactttagga agagcgtgga cagcacgggc aggaggcagt accaggtcca gaacggaccc 121 tgcagctaca cattcctgct gccggagacc gacagctgcc gatcttcctc cagcccctac 181 atgtctaacg ccgtgcagag ggatgcaccc ctcgactacg acgactcagt gcaaaggctg 241 caggtgctgg agaacattct agagaacaac acacagtggc tgatgaagct ggagaactac 301 atccaggaca acatgaagaa ggagatggtg gagatccagc agaatgtggt gcagaaccag 361 acggctgtga tgatcgagat tggaaccagt ttgctcaacc agacggcggc acaaactcgg 421 aaactgactg atgtggaagc ccaagtacta aaccagacga caagactcga gttgcagctt 481 cttcaacatt ctatttctac caacaaattg gaaaagcaga ttttggacca gaccagtgaa 541 ataaacaaac tacaagataa gaacagcttc ctagaaaaga aagtgctgga catggaggac 601 aagcacagtg tgcagctgca gtccatgaag gagcagaagg accagcttca ggtgctggtg 661 tccaagcaga gctccgtcat cgatgagctg gagaagaagc tggtgacggc cacagtcaac |
| | 721 aactcagtcc ttcagaagca gcagcatgat ctaatggaga cggtcaacag cttgctgacc 781 atgatgtcat cgcccgacta taagagctct gttgctgtcc ctaaagaaga gaaaaccacc 841 ttcagagact gtgcagaaat cttcaagtca ggactcacca ccagtggcat ctacacgctg 901 accttcccca actccacaga ggaggtcaag gcctactgtg acatggacat gggtggagga 961 gggtggacgg tcatccaaca ccgggaagat ggcagcgttg acttccagag aacgtggaaa 1021 gagtacaaag agggcttcgg gagccctctg ggagagtatt ggctgggcaa cgagtttgtc 1081 tcccagctga ccagtgggca tcgctacgtg ctgaagatcc agctgaagga ctgggaaggc 1141 agcgaggcac actctctgta tgagcacttc tacctgtccg gcgaggagtc caactacagg 1201 attcacctta caggactcac aggcacggcg ggcaaaatca gtagcatcag ccaaccagga 1261 agtgatttta gcacaaagga ttcggacaat gacaaatgca tctgcaagtg ttcccagatg 1321 ctcacaggag gctggtggtt cgacgcatgt ggtccttcca acttgaatgg acagtactac 1381 ccacaaaagc agaatacgaa taagttcaac ggtatcaagt ggtactactg gaaggggtcc 1441 ggttactcgc tcaaggccac gaccatgatg atccggccag cagatttcta a |
| SEQ ID NO: 16 | Rat Anq-2 Amino Acid Sequence |
| | 1 mwqivfltfg cdlvlasayn nfrksvdstg rrqyqvqngp csytfllpet dscrsssspy 61 msnavqrdap ldyddsvqrl qvlenilenn tqwlmkleny iqdnmkkemv eiqqnvvqnq 121 tavmieigts llnqtaaqtr kltdveaqvl nqttrlelql lqhsistnkl ekqildqtse 181 inklqdknsf lekkvldmed khsvqlqsmk eqkdqlqvlv skqssvidel ekklvtatvn 241 nsvlqkqqhd lmetvnsllt mmsspdykss vavpkeektt frdcaeifks glttsgiytl 301 tfpnsteevk aycdmdmggg gwtviqhred gsvdfqrtwk eykegfgspl geywlgnefv 361 sqltsghryv lkiqlkdweg seahslyehf ylsgeesnyr ihltgltgta gkissisqpg 421 sdfstkdsdn dkcickcsqm ltggwwfdac gpsnlngqyy pqkqntnkfn gikwyywkgs 481 gyslkattmm irpadf |
| SEQ ID NO: 17 | Chicken Anq-2 cDNA Sequence |
| | 1 atggcatcac tgatgattca gcttgagaat tacatccaag acagtatgaa gaaagaaatg 61 gtagagatcc agcaaactgc agtgcagaac cagactgcag taatgattga aataggcaca 121 aacttactaa atcaaacagc tgagcagacc cgcaaattaa cagatgttga agcacaagta 181 ctaaaccaga caaccagact tgaacttcag cttttggaac attccctttc aacaaataaa 241 ttagaaagac agatttctgt tcagaccaac gagataacta aattacaaga aaaaaacagc 301 tttctagaaa aaagagttct tgagatggaa gacaagcaca cacttcagct gaagtcaata 361 aaagatgaaa aagatcagct tcaagtccta gtagccagac agaattccat tatagaagaa 421 ctagaaaagc agttagttac agctacggta aacaactccg ttctgcaaaa acagcaacat 481 gatttgatgg agactgttca taacttactt actatgatat ctacaccaaa ctcagctaag 541 aagaacttca tagctaaaga ggagcaaatc agcttcaaag actgtgctga agctttcaaa 601 tctggactca caacaagtgg aacctacacc ttaacatttc ctaactctgc acaggagaag 661 aaggcctact gtgacatgga aagtaatgga ggaggttgga cagttcttca gagacgtgaa 721 ggtggcagcg tggattttca ccggacatgg aaggagtaca agataggatt tggtgatcct 781 gctggggagt actggctagg aaatgagttt gtttctcaac tgactaatca gaagcgctac 841 gttcttaaga taatcctgaa agactgggaa ggaaatgaag cgtacacttt gtatgaccaa 901 ttctatctag caaacgaaga acaaaaatac aggatccacc ttaaaggact tactgggaca 961 gcaggcaaaa taagtagtat aagccaacca ggaaatgatt ttagcacaaa ggatacagac 1021 aatgacaaat gtatttgcaa atgttcacaa atgctaacag gagggtggtg gtttgatgct 1081 tgtggtcctt ctaacctcaa tggaatgtat tatccattac gacagaacaa caacaagttc 1141 aatggtatca agtggtacta ctggaaaggc tcaggatact ctctcaaagc cacgactatg 1201 atgattcgac cagcagattt ctaa |
| SEQ ID NO: 18 | Chicken Anq-2 Amino Acid Sequence |
| | 1 maslmiqlen yiqdsmkkem veiqqtavqn qtavmieigt nllnqtaeqt rkltdveaqv 61 lnqttrlelq llehslstnk lerqisvqtn eitklqekns flekrvleme dkhtlqlksi 121 kdekdqlqvl varqnsiiee lekqlvtatv nnsvlqkqqh dlmetvhnll tmistpnsak 181 knfiakeeqi sfkdcaeafk sglttsgtyt ltfpnsaqek kaycdmesng ggwtvlqrre 241 ggsvdfhrtw keykigfgdp ageywlgnef vsqltnqkry vlkiilkdwe gneaytlydq 301 fylaneeqky rihlkgltgt agkissisqp gndfstkdtd ndkcickcsq mltggwwfda 361 cgpsnlngmy yplrqnnnkf ngikwyywkg sgyslkattm mirpadf |

| | |
|---|---|
| * Included in Table 1 are RNA nucleic acid molecules (e.g., thymines replaced with uredines), nucleic acid molecules encoding orthologs of the encoded proteins, as well as DNA or RNA nucleic acid sequences comprising a nucleic acid sequence having at least 80%, 81%, 82%, 83%, 84%, 85%, 86%, 87%, 88%, 89%, 90%, 91%, 92%, 93%, 94%, 95%, 96%, 97%, 98%, 99%, 99.5%, or more identity across their full length with the nucleic acid sequence of any SEQ ID NO listed in Table 1, or a portion thereof. Such nucleic acid molecules can have a function of the full-length nucleic acid as described further herein. * Included in Table 1 are orthologs of the proteins, as well as polypeptide molecules comprising an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, 99.5%, or more identity across their full length with an amino acid sequence of any SEQ ID NO listed in Table 1, or a portion thereof. Such polypeptides can have a function of the full-length polypeptide as described further herein. * Included in Table 1 is Ang-2, including any Ang-2 cDNA or polypeptide. | |

### II. Subjects

In one embodiment, the subject for whom predicted likelihood of efficacy of an anti-immune checkpoint therapy is determined, is a mammal (e.g., mouse, rat, primate, non-human mammal, domestic animal, such as a dog, cat, cow, horse, and the like), and is preferably a human.

In another embodiment of the methods of the present invention, the subject has not undergone treatment, such as chemotherapy, radiation therapy, targeted therapy, and/or anti-immune checkpoint therapy. In still another embodiment, the subject has undergone treatment, such as chemotherapy, radiation therapy, targeted therapy, and/or anti-immune checkpoint therapy.

In certain embodiments, the subject has had surgery to remove cancerous or precancerous tissue. In other embodiments, the cancerous tissue has not been removed, e.g., the cancerous tissue may be located in an inoperable region of the body, such as in a tissue that is essential for life, or in a region where a surgical procedure would cause considerable risk of harm to the patient.

The methods of the present invention can be used to determine the responsiveness to anti-immune checkpoint therapies of many different cancers in subjects such as those described above. In one embodiment, the cancers are solid tumors, such as lung cancer, melanoma, and/or renal cell carcinoma. In another embodiment, the cancer is an epithelial cancer such as, but not limited to, brain cancer (e.g., glioblastomas) bladder cancer, breast cancer, cervical cancer, colon cancer, gynecologic cancers, renal cancer, laryngeal cancer, lung cancer, oral cancer, head and neck cancer, ovarian cancer, pancreatic cancer, prostate cancer, or skin cancer. In still other embodiments, the cancer is breast cancer, prostate cancer, lung cancer, or colon cancer. In still other embodiments, the epithelial cancer is non-small-cell lung cancer, nonpapillary renal cell carcinoma, cervical carcinoma, ovarian carcinoma (e.g., serous ovarian carcinoma), or breast carcinoma. The epithelial cancers may be characterized in various other ways including, but not limited to, serous, endometrioid, mucinous, clear cell, brenner, or undifferentiated.

### III. Sample Collection, Preparation and Separation

In some embodiments, biomarker amount and/or activity measurement(s) in a sample from a subject is compared to a predetermined control (standard) sample. The sample from the subject is typically from a diseased tissue, such as cancer cells or tissues. The control sample can be from the same subject or from a different subject. The control sample is typically a normal, non-diseased sample. However, in some embodiments, such as for staging of disease or for evaluating the efficacy of treatment, the control sample can be from a diseased tissue. The control sample can be a combination of samples from several different subjects. In some embodiments, the biomarker amount and/or activity measurement(s) from a subject is compared to a pre-determined level. This pre-determined level is typically obtained from normal samples. As described herein, a "pre-determined" biomarker amount and/or activity measurement(s) may be a biomarker amount and/or activity measurement(s) used to, by way of example only, evaluate a subject that may be selected for treatment, evaluate a response to an anti-immune checkpoint therapy, and/or evaluate a response to a combination anti-immune checkpoint therapy. A pre-determined biomarker amount and/or activity measurement(s) may be determined in populations of patients with or without cancer. The pre-determined biomarker amount and/or activity measurement(s) can be a single number, equally applicable to every patient, or the pre-determined biomarker amount and/or activity measurement(s) can vary according to specific subpopulations of patients. Age, weight, height, and other factors of a subject may affect the pre-determined biomarker amount and/or activity measurement(s) of the individual. Furthermore, the pre-determined biomarker amount and/or activity can be determined for each subject individually. In one embodiment, the amounts determined and/or compared in a method described herein are based on absolute measurements.

In another embodiment, the amounts determined and/or compared in a method described herein are based on relative measurements, such as ratios (e.g., biomarker copy numbers, level, and/or activity before a treatment vs. after a treatment, such biomarker measurements relative to a spiked or man-made control, such biomarker measurements relative to the expression of a housekeeping gene, and the like). For example, the relative analysis can be based on the ratio of pre-treatment biomarker measurement as compared to post-treatment biomarker measurement. Pre-treatment biomarker measurement can be made at any time prior to initiation of anti-cancer therapy. Post-treatment biomarker measurement can be made at any time after initiation of anti-cancer therapy. In some embodiments, post-treatment biomarker measurements are made 1, 2, 3, 4, 5, 6, 7, 8, 9, 10, 11, 12, 13, 14, 15, 16, 17, 18, 19, 20 weeks or more after initiation of anti-cancer therapy, and even longer toward indefinitely for continued monitoring. Treatment can comprise anti-cancer therapy, such as a therapeutic regimen comprising ipilimumab alone or in combination with other anti-cancer agents, such as bevacizumab or anti-Ang-2 agents.

The pre-determined biomarker amount and/or activity measurement(s) can be any suitable standard. For example, the pre-determined biomarker amount and/or activity measurement(s) can be obtained from the same or a different human for whom a patient selection is being assessed. In one embodiment, the pre-determined biomarker amount and/or activity measurement(s) can be obtained from a previous assessment of the same patient. In such a manner, the progress of the selection of the patient can be monitored over time. In addition, the control can be obtained from an assessment of another human or multiple humans, *e.g.,* selected groups of humans, if the subject is a human. In such a manner, the extent of the selection of the human for whom selection is being assessed can be compared to suitable other humans, *e.g.,* other humans who are in a similar situation to the human of interest, such as those suffering from similar or the same condition(s) and/or of the same ethnic group.

In some embodiments of the present invention the change of biomarker amount and/or activity measurement(s) from the pre-determined level is about 0.1, 0.2, 0.3, 0.4, 0.5, 0.6, 0.7, 0.8, 0.9, 1.0, 1.5, 2.0, 2.5, 3.0, 3.5, 4.0, 4.5, or 5.0 fold or greater, or any range in between, inclusive. Such cutoff values apply equally when the measurement is based on relative changes, such as based on the ratio of pre-treatment biomarker measurement as compared to post-treatment biomarker measurement.

Biological samples can be collected from a variety of sources from a patient including a body fluid sample, cell sample, or a tissue sample comprising nucleic acids and/or proteins. "Body fluids" refer to fluids that are excreted or secreted from the body as well as fluids that are normally not (e.g., amniotic fluid, aqueous humor, bile, blood and blood plasma, cerebrospinal fluid, cerumen and earwax, cowper's fluid or pre-ej aculatory fluid, chyle, chyme, stool, female ejaculate, interstitial fluid, intracellular fluid, lymph, menses, breast milk, mucus, pleural fluid, pus, saliva, sebum, semen, serum, sweat, synovial fluid, tears, urine, vaginal lubrication, vitreous humor, vomit). In a preferred embodiment, the subject and/or control sample is selected from the group consisting of cells, cell lines, histological slides, paraffin embedded tissues, biopsies, whole blood, nipple aspirate, serum, plasma, buccal scrape, saliva, cerebrospinal fluid, urine, stool, and bone marrow. In one embodiment, the sample is serum, plasma, or urine. IN another embodiment, the sample is serum.

The samples can be collected from individuals repeatedly over a longitudinal period of time *(e.g.,* once or more on the order of days, weeks, months, annually, biannually, etc.). Obtaining numerous samples from an individual over a period of time can be used to verify results from earlier detections and/or to identify an alteration in biological pattern as a result of, for example, disease progression, drug treatment, *etc.* For example, subject samples can be taken and monitored every month, every two months, or combinations of one, two, or three month intervals according to the present invention. In addition, the biomarker amount and/or activity measurements of the subject obtained over time can be conveniently compared with each other, as well as with those of normal controls during the monitoring period, thereby providing the subject's own values, as an internal, or personal, control for long-term monitoring.

Sample preparation and separation can involve any of the procedures, depending on the type of sample collected and/or analysis of biomarker measurement(s). Such procedures include, by way of example only, concentration, dilution, adjustment of pH, removal of high abundance polypeptides (e.g., albumin, gamma globulin, and transferrin, etc.), addition of preservatives and calibrants, addition of protease inhibitors, addition of denaturants, desalting of samples, concentration of sample proteins, extraction and purification of lipids.

The sample preparation can also isolate molecules that are bound in non-covalent complexes to other protein (e.g., carrier proteins). This process may isolate those molecules bound to a specific carrier protein (e.g., albumin), or use a more general process, such as the release of bound molecules from all carrier proteins via protein denaturation, for example using an acid, followed by removal of the carrier proteins.

Removal of undesired proteins (e.g., high abundance, uninformative, or undetectable proteins) from a sample can be achieved using high affinity reagents, high molecular weight filters, ultracentrifugation and/or electrodialysis. High affinity reagents include antibodies or other reagents (e.g., aptamers) that selectively bind to high abundance proteins. Sample preparation could also include ion exchange chromatography, metal ion affinity chromatography, gel filtration, hydrophobic chromatography, chromatofocusing, adsorption chromatography, isoelectric focusing and related techniques. Molecular weight filters include membranes that separate molecules on the basis of size and molecular weight. Such filters may further employ reverse osmosis, nanofiltration, ultrafiltration and microfiltration.

Ultracentrifugation is a method for removing undesired polypeptides from a sample. Ultracentrifugation is the centrifugation of a sample at about 15,000-60,000 rpm while monitoring with an optical system the sedimentation (or lack thereof) of particles. Electrodialysis is a procedure which uses an electromembrane or semipermable membrane in a process in which ions are transported through semi-permeable membranes from one solution to another under the influence of a potential gradient. Since the membranes used in electrodialysis may have the ability to selectively transport ions having positive or negative charge, reject ions of the opposite charge, or to allow species to migrate through a semipermable membrane based on size and charge, it renders electrodialysis useful for concentration, removal, or separation of electrolytes.

Separation and purification in the present invention may include any procedure known in the art, such as capillary electrophoresis (*e.g.,* in capillary or on-chip) or chromatography (*e.g.,* in capillary, column or on a chip). Electrophoresis is a method which can be used to separate ionic molecules under the influence of an electric field. Electrophoresis can be conducted in a gel, capillary, or in a microchannel on a chip. Examples of gels used for electrophoresis include starch, acrylamide, polyethylene oxides, agarose, or combinations thereof. A gel can be modified by its cross-linking, addition of detergents, or denaturants, immobilization of enzymes or antibodies (affinity electrophoresis) or substrates (zymography) and incorporation of a pH gradient. Examples of capillaries used for electrophoresis include capillaries that interface with an electrospray.

Capillary electrophoresis (CE) is preferred for separating complex hydrophilic molecules and highly charged solutes. CE technology can also be implemented on microfluidic chips. Depending on the types of capillary and buffers used, CE can be further segmented into separation techniques such as capillary zone electrophoresis (CZE), capillary isoelectric focusing (ClEF), capillary isotachophoresis (cITP) and capillary electrochromatography (CEC). An embodiment to couple CE techniques to electrospray ionization involves the use of volatile solutions, for example, aqueous mixtures containing a volatile acid and/or base and an organic such as an alcohol or acetonitrile.

Capillary isotachophoresis (cITP) is a technique in which the analytes move through the capillary at a constant speed but are nevertheless separated by their respective mobilities. Capillary zone electrophoresis (CZE), also known as free-solution CE (FSCE), is based on differences in the electrophoretic mobility of the species, determined by the charge on the molecule, and the frictional resistance the molecule encounters during migration which is often directly proportional to the size of the molecule. Capillary isoelectric focusing (CIEF) allows weakly-ionizable amphoteric molecules, to be separated by electrophoresis in a pH gradient. CEC is a hybrid technique between traditional high performance liquid chromatography (HPLC) and CE.

Separation and purification techniques used in the present invention include any chromatography procedures known in the art. Chromatography can be based on the differential adsorption and elution of certain analytes or partitioning of analytes between mobile and stationary phases. Different examples of chromatography include, but not limited to, liquid chromatography (LC), gas chromatography (GC), high performance liquid chromatography (HPLC), etc.

### IV. Biomarker Nucleic Acids and Polypeptides

One aspect of the present invention pertains to the use of isolated nucleic acid molecules that correspond to biomarker nucleic acids that encode a biomarker polypeptide or a portion of such a polypeptide. As used herein, the term "nucleic acid molecule" is intended to include DNA molecules *(e.g.,* cDNA or genomic DNA) and RNA molecules *(e.g.,* mRNA) and analogs of the DNA or RNA generated using nucleotide analogs. The nucleic acid molecule can be single-stranded or double-stranded, but preferably is double-stranded DNA.

An "isolated" nucleic acid molecule is one which is separated from other nucleic acid molecules which are present in the natural source of the nucleic acid molecule. Preferably, an "isolated" nucleic acid molecule is free of sequences (preferably protein-encoding sequences) which naturally flank the nucleic acid (*i.e*., sequences located at the 5' and 3' ends of the nucleic acid) in the genomic DNA of the organism from which the nucleic acid is derived. For example, in various embodiments, the isolated nucleic acid molecule can contain less than about 5 kB, 4 kB, 3 kB, 2 kB, 1 kB, 0.5 kB or 0.1 kB of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material or culture medium when produced by recombinant techniques, or substantially free of chemical precursors or other chemicals when chemically synthesized.

A biomarker nucleic acid molecule of the present invention can be isolated using standard molecular biology techniques and the sequence information in the database records described herein. Using all or a portion of such nucleic acid sequences, nucleic acid molecules of the present invention can be isolated using standard hybridization and cloning techniques *(e.g.,* as described in Sambrook et al., ed., Molecular Cloning: A Laboratory Manual, 2nd ed., Cold Spring Harbor Laboratory Press, Cold Spring Harbor, NY, 1989).

A nucleic acid molecule of the present invention can be amplified using cDNA, mRNA, or genomic DNA as a template and appropriate oligonucleotide primers according to standard PCR amplification techniques. The nucleic acid molecules so amplified can be cloned into an appropriate vector and characterized by DNA sequence analysis. Furthermore, oligonucleotides corresponding to all or a portion of a nucleic acid molecule of the present invention can be prepared by standard synthetic techniques, *e.g.,* using an automated DNA synthesizer.

Moreover, a nucleic acid molecule of the present invention can comprise only a portion of a nucleic acid sequence, wherein the full length nucleic acid sequence comprises a marker of the present invention or which encodes a polypeptide corresponding to a marker of the present invention. Such nucleic acid molecules can be used, for example, as a probe or primer. The probe/primer typically is used as one or more substantially purified oligonucleotides. The oligonucleotide typically comprises a region of nucleotide sequence that hybridizes under stringent conditions to at least about 7, preferably about 15, more preferably about 25, 50, 75, 100, 125, 150, 175, 200, 250, 300, 350, or 400 or more consecutive nucleotides of a biomarker nucleic acid sequence. Probes based on the sequence of a biomarker nucleic acid molecule can be used to detect transcripts or genomic sequences corresponding to one or more markers of the present invention. The probe comprises a label group attached thereto, *e.g.,* a radioisotope, a fluorescent compound, an enzyme, or an enzyme co-factor.

A biomarker nucleic acid molecules that differ, due to degeneracy of the genetic code, from the nucleotide sequence of nucleic acid molecules encoding a protein which corresponds to the biomarker, and thus encode the same protein, are also contemplated.

In addition, it will be appreciated by those skilled in the art that DNA sequence polymorphisms that lead to changes in the amino acid sequence can exist within a population (*e.g.,* the human population). Such genetic polymorphisms can exist among individuals within a population due to natural allelic variation. An allele is one of a group of genes which occur alternatively at a given genetic locus. In addition, it will be appreciated that DNA polymorphisms that affect RNA expression levels can also exist that may affect the overall expression level of that gene (*e.g.,* by affecting regulation or degradation).

The term "allele," which is used interchangeably herein with "allelic variant," refers to alternative forms of a gene or portions thereof. Alleles occupy the same locus or position on homologous chromosomes. When a subject has two identical alleles of a gene, the subject is said to be homozygous for the gene or allele. When a subject has two different alleles of a gene, the subject is said to be heterozygous for the gene or allele. For example, biomarker alleles can differ from each other in a single nucleotide, or several nucleotides, and can include substitutions, deletions, and insertions of nucleotides. An allele of a gene can also be a form of a gene containing one or more mutations.

The term "allelic variant of a polymorphic region of gene" or "allelic variant", used interchangeably herein, refers to an alternative form of a gene having one of several possible nucleotide sequences found in that region of the gene in the population. As used herein, allelic variant is meant to encompass functional allelic variants, non-functional allelic variants, SNPs, mutations and polymorphisms.

The term "single nucleotide polymorphism" (SNP) refers to a polymorphic site occupied by a single nucleotide, which is the site of variation between allelic sequences. The site is usually preceded by and followed by highly conserved sequences of the allele (e.g., sequences that vary in less than 1/100 or 1/1000 members of a population). A SNP usually arises due to substitution of one nucleotide for another at the polymorphic site. SNPs can also arise from a deletion of a nucleotide or an insertion of a nucleotide relative to a reference allele. Typically the polymorphic site is occupied by a base other than the reference base. For example, where the reference allele contains the base "T" (thymidine) at the polymorphic site, the altered allele can contain a "C" (cytidine), "G" (guanine), or "A" (adenine) at the polymorphic site. SNP's may occur in protein-coding nucleic acid sequences, in which case they may give rise to a defective or otherwise variant protein, or genetic disease. Such a SNP may alter the coding sequence of the gene and therefore specify another amino acid (a "missense" SNP) or a SNP may introduce a stop codon (a "nonsense" SNP). When a SNP does not alter the amino acid sequence of a protein, the SNP is called "silent." SNP's may also occur in noncoding regions of the nucleotide sequence. This may result in defective protein expression, e.g., as a result of alternative spicing, or it may have no effect on the function of the protein.

As used herein, the terms "gene" and "recombinant gene" refer to nucleic acid molecules comprising an open reading frame encoding a polypeptide corresponding to a marker of the present invention. Such natural allelic variations can typically result in 1-5% variance in the nucleotide sequence of a given gene. Alternative alleles can be identified by sequencing the gene of interest in a number of different individuals. This can be readily carried out by using hybridization probes to identify the same genetic locus in a variety of individuals. Any and all such nucleotide variations and resulting amino acid polymorphisms or variations that are the result of natural allelic variation and that do not alter the functional activity are intended to be within the scope of the present invention.

In another embodiment, a biomarker nucleic acid molecule is at least 7, 15, 20, 25, 30, 40, 60, 80, 100, 150, 200, 250, 300, 350, 400, 450, 550, 650, 700, 800, 900, 1000, 1100, 1200, 1300, 1400, 1500, 1600, 1700, 1800, 1900, 2000, 2200, 2400, 2600, 2800, 3000, 3500, 4000, 4500, or more nucleotides in length and hybridizes under stringent conditions to a nucleic acid molecule corresponding to a marker of the present invention or to a nucleic acid molecule encoding a protein corresponding to a marker of the present invention. As used herein, the term "hybridizes under stringent conditions" is intended to describe conditions for hybridization and washing under which nucleotide sequences at least 60% (65%, 70%, 75%, 80%, preferably 85%) identical to each other typically remain hybridized to each other. Such stringent conditions are known to those skilled in the art and can be found in sections 6.3.1-6.3.6 of Current Protocols in Molecular Biology, John Wiley & Sons, N.Y. (1989). A preferred, non-limiting example of stringent hybridization conditions are hybridization in 6X sodium chloride/sodium citrate (SSC) at about 45°C, followed by one or more washes in 0.2X SSC, 0.1% SDS at 50-65°C.

In addition to naturally-occurring allelic variants of a nucleic acid molecule of the present invention that can exist in the population, the skilled artisan will further appreciate that sequence changes can be introduced by mutation thereby leading to changes in the amino acid sequence of the encoded protein, without altering the biological activity of the protein encoded thereby. For example, one can make nucleotide substitutions leading to amino acid substitutions at "non-essential" amino acid residues. A "non-essential" amino acid residue is a residue that can be altered from the wild-type sequence without altering the biological activity, whereas an "essential" amino acid residue is required for biological activity. For example, amino acid residues that are not conserved or only semi-conserved among homologs of various species may be non-essential for activity and thus would be likely targets for alteration. Alternatively, amino acid residues that are conserved among the homologs of various species (*e.g.,* murine and human) may be essential for activity and thus would not be likely targets for alteration.

Accordingly, another aspect of the present invention pertains to nucleic acid molecules encoding a polypeptide of the present invention that contain changes in amino acid residues that are not essential for activity. Such polypeptides differ in amino acid sequence from the naturally-occurring proteins which correspond to the markers of the present invention, yet retain biological activity. In one embodiment, a biomarker protein has an amino acid sequence that is at least about 40% identical, 50%, 60%, 70%, 75%, 80%, 83%, 85%, 87.5%, 90%, 91%, 92%, 93%, 94%, 95%, 96%, 97%, 98%, 99% or identical to the amino acid sequence of a biomarker protein described herein.

An isolated nucleic acid molecule encoding a variant protein can be created by introducing one or more nucleotide substitutions, additions or deletions into the nucleotide sequence of nucleic acids of the present invention, such that one or more amino acid residue substitutions, additions, or deletions are introduced into the encoded protein. Mutations can be introduced by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), non-polar side chains (*e.g.,* alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity. Following mutagenesis, the encoded protein can be expressed recombinantly and the activity of the protein can be determined.

In some embodiments, the present invention further contemplates the use of anti-biomarker antisense nucleic acid molecules, *i.e.,* molecules which are complementary to a sense nucleic acid of the present invention, *e.g.,* complementary to the coding strand of a double-stranded cDNA molecule corresponding to a marker of the present invention or complementary to an mRNA sequence corresponding to a marker of the present invention. Accordingly, an antisense nucleic acid molecule of the present invention can hydrogen bond to (*i.e.* anneal with) a sense nucleic acid of the present invention. The antisense nucleic acid can be complementary to an entire coding strand, or to only a portion thereof, *e.g.,* all or part of the protein coding region (or open reading frame). An antisense nucleic acid molecule can also be antisense to all or part of a non-coding region of the coding strand of a nucleotide sequence encoding a polypeptide of the present invention. The non-coding regions ("5' and 3' untranslated regions") are the 5' and 3' sequences which flank the coding region and are not translated into amino acids.

An antisense oligonucleotide can be, for example, about 5, 10, 15, 20, 25, 30, 35, 40, 45, or 50 or more nucleotides in length. An antisense nucleic acid can be constructed using chemical synthesis and enzymatic ligation reactions using procedures known in the art. For example, an antisense nucleic acid (*e.g.,* an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g.,* phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been sub-cloned in an antisense orientation (*i.e*., RNA transcribed from the inserted nucleic acid will be of an antisense orientation to a target nucleic acid of interest, described further in the following subsection).

The antisense nucleic acid molecules of the present invention are typically administered to a subject or generated *in situ* such that they hybridize with or bind to cellular mRNA and/or genomic DNA encoding a polypeptide corresponding to a selected marker of the present invention to thereby inhibit expression of the marker, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. Examples of a route of administration of antisense nucleic acid molecules of the present invention includes direct injection at a tissue site or infusion of the antisense nucleic acid into a blood- or bone marrow-associated body fluid. Alternatively, antisense nucleic acid molecules can be modified to target selected cells and then administered systemically. For example, for systemic administration, antisense molecules can be modified such that they specifically bind to receptors or antigens expressed on a selected cell surface, *e.g.*, by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

An antisense nucleic acid molecule of the present invention can be an α-anomeric nucleic acid molecule. An α-anomeric nucleic acid molecule forms specific double-stranded hybrids with complementary RNA in which, contrary to the usual α-units, the strands run parallel to each other (Gaultier et al., 1987, Nucleic Acids Res. 15:6625-6641). The antisense nucleic acid molecule can also comprise a 2'-o-methylribonucleotide (Inoue et al., 1987, Nucleic Acids Res. 15:6131-6148) or a chimeric RNA-DNA analogue (Inoue et al., 1987, FEBS Lett. 215:327-330).

The present invention also encompasses ribozymes. Ribozymes are catalytic RNA molecules with ribonuclease activity which are capable of cleaving a single-stranded nucleic acid, such as an mRNA, to which they have a complementary region. Thus, ribozymes (e.g., hammerhead ribozymes as described in Haselhoff and Gerlach, 1988, Nature 334:585-591) can be used to catalytically cleave mRNA transcripts to thereby inhibit translation of the protein encoded by the mRNA. A ribozyme having specificity for a nucleic acid molecule encoding a polypeptide corresponding to a marker of the present invention can be designed based upon the nucleotide sequence of a cDNA corresponding to the marker. For example, a derivative of a *Tetrahymena* L-19 IVS RNA can be constructed in which the nucleotide sequence of the active site is complementary to the nucleotide sequence to be cleaved (see Cech et al. U.S. Patent No. 4,987,071; and Cech et al. U.S. Patent No. 5,116,742). Alternatively, an mRNA encoding a polypeptide of the present invention can be used to select a catalytic RNA having a specific ribonuclease activity from a pool of RNA molecules (see, *e.g.,* Bartel and Szostak, 1993, Science 261:1411-1418).

The present invention also encompasses nucleic acid molecules which form triple helical structures. For example, expression of a biomarker protein can be inhibited by targeting nucleotide sequences complementary to the regulatory region of the gene encoding the polypeptide (*e.g.,* the promoter and/or enhancer) to form triple helical structures that prevent transcription of the gene in target cells. See generally Helene (1991) Anticancer Drug Des. 6(6):569-84; Helene (1992) Ann. N.Y. Acad. Sci. 660:27-36; and Maher (1992) Bioassays 14(12):807-15.

In various embodiments, the nucleic acid molecules of the present invention can be modified at the base moiety, sugar moiety or phosphate backbone to improve, e.g., the stability, hybridization, or solubility of the molecule. For example, the deoxyribose phosphate backbone of the nucleic acid molecules can be modified to generate peptide nucleic acid molecules (see Hyrup et al., 1996, Bioorganic & Medicinal Chemistry 4(1): 5-23). As used herein, the terms "peptide nucleic acids" or "PNAs" refer to nucleic acid mimics, e.g., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup *et al.* (1996), *supra;* Perry-O'Keefe et al. (1996) Proc. Natl. Acad. Sci. USA 93:14670-675.

PNAs can be used in therapeutic and diagnostic applications. For example, PNAs can be used as antisense or antigene agents for sequence-specific modulation of gene expression by, e.g., inducing transcription or translation arrest or inhibiting replication. PNAs can also be used, *e.g.,* in the analysis of single base pair mutations in a gene by, *e.g.,* PNA directed PCR clamping; as artificial restriction enzymes when used in combination with other enzymes, *e*.*g*., S1 nucleases (Hyrup (1996), *supra*; or as probes or primers for DNA sequence and hybridization (Hyrup, 1996, *supra*; Perry-O'Keefe et al., 1996, Proc. Natl. Acad. Sci. USA 93:14670-675).

In another embodiment, PNAs can be modified, *e.g.,* to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras can be generated which can combine the advantageous properties of PNA and DNA. Such chimeras allow DNA recognition enzymes, *e.g.,* RNASE H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup, 1996, *supra*). The synthesis of PNA-DNA chimeras can be performed as described in Hyrup (1996), *supra,* and Finn et al. (1996) Nucleic Acids Res. 24(17):3357-63. For example, a DNA chain can be synthesized on a solid support using standard phosphoramidite coupling chemistry and modified nucleoside analogs. Compounds such as 5'-(4-methoxytrityl)amino-5'-deoxy-thymidine phosphoramidite can be used as a link between the PNA and the 5' end of DNA (Mag et al., 1989, Nucleic Acids Res. 17:5973-88). PNA monomers are then coupled in a step-wise manner to produce a chimeric molecule with a 5' PNA segment and a 3' DNA segment (Finn et al., 1996, Nucleic Acids Res. 24(17):3357-63). Alternatively, chimeric molecules can be synthesized with a 5' DNA segment and a 3' PNA segment (Peterser et al., 1975, Bioorganic Med. Chem. Lett. 5:1119-11124).

In other embodiments, the oligonucleotide can include other appended groups such as peptides *(e.g.,* for targeting host cell receptors *in vivo*), or agents facilitating transport across the cell membrane (see, *e.g.,* Letsinger et al., 1989, Proc. Natl. Acad. Sci. USA 86:6553-6556; Lemaitre et al., 1987, Proc. Natl. Acad. Sci. USA 84:648-652; PCT Publication No. WO 88/09810) or the blood-brain barrier (see, *e.g.,* PCT Publication No. WO 89/10134). In addition, oligonucleotides can be modified with hybridization-triggered cleavage agents (see, *e.g.,* Krol et al., 1988, Bio/Techniques 6:958-976) or intercalating agents (see, *e.g.,* Zon, 1988, Pharm. Res. 5:539-549). To this end, the oligonucleotide can be conjugated to another molecule, *e.g.,* a peptide, hybridization triggered cross-linking agent, transport agent, hybridization-triggered cleavage agent, *etc.*

Another aspect of the present invention pertains to the use of biomarker proteins and biologically active portions thereof. In one embodiment, the native polypeptide corresponding to a marker can be isolated from cells or tissue sources by an appropriate purification scheme using standard protein purification techniques. In another embodiment, polypeptides corresponding to a marker of the present invention are produced by recombinant DNA techniques. Alternative to recombinant expression, a polypeptide corresponding to a marker of the present invention can be synthesized chemically using standard peptide synthesis techniques.

An "isolated" or "purified" protein or biologically active portion thereof is substantially free of cellular material or other contaminating proteins from the cell or tissue source from which the protein is derived, or substantially free of chemical precursors or other chemicals when chemically synthesized. The language "substantially free of cellular material" includes preparations of protein in which the protein is separated from cellular components of the cells from which it is isolated or recombinantly produced. Thus, protein that is substantially free of cellular material includes preparations of protein having less than about 30%, 20%, 10%, or 5% (by dry weight) of heterologous protein (also referred to herein as a "contaminating protein"). When the protein or biologically active portion thereof is recombinantly produced, it is also preferably substantially free of culture medium, i.e., culture medium represents less than about 20%, 10%, or 5% of the volume of the protein preparation. When the protein is produced by chemical synthesis, it is preferably substantially free of chemical precursors or other chemicals, i.e., it is separated from chemical precursors or other chemicals which are involved in the synthesis of the protein. Accordingly such preparations of the protein have less than about 30%, 20%, 10%, 5% (by dry weight) of chemical precursors or compounds other than the polypeptide of interest.

Biologically active portions of a biomarker polypeptide include polypeptides comprising amino acid sequences sufficiently identical to or derived from a biomarker protein amino acid sequence described herein, but which includes fewer amino acids than the full length protein, and exhibit at least one activity of the corresponding full-length protein. Typically, biologically active portions comprise a domain or motif with at least one activity of the corresponding protein. A biologically active portion of a protein of the present invention can be a polypeptide which is, for example, 10, 25, 50, 100 or more amino acids in length. Moreover, other biologically active portions, in which other regions of the protein are deleted, can be prepared by recombinant techniques and evaluated for one or more of the functional activities of the native form of a polypeptide of the present invention.

Preferred polypeptides have an amino acid sequence of a biomarker protein encoded by a nucleic acid molecule described herein. Other useful proteins are substantially identical *(e.g.,* at least about 40%, preferably 50%, 60%, 70%, 75%, 80%, 83%, 85%, 88%, 90%, 91%, 92%, 93%, 94%, 95%, 96%, 97%, 98%, or 99%) to one of these sequences and retain the functional activity of the protein of the corresponding naturally-occurring protein yet differ in amino acid sequence due to natural allelic variation or mutagenesis.

To determine the percent identity of two amino acid sequences or of two nucleic acids, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (*i.e.,* % identity = # of identical positions/total # of positions (*e*.*g*., overlapping positions) x100). In one embodiment the two sequences are the same length.

The determination of percent identity between two sequences can be accomplished using a mathematical algorithm. A preferred, non-limiting example of a mathematical algorithm utilized for the comparison of two sequences is the algorithm of Karlin and Altschul (1990) Proc. Natl. Acad. Sci. USA 87:2264-2268, modified as in Karlin and Altschul (1993) Proc. Natl. Acad. Sci. USA 90:5873-5877. Such an algorithm is incorporated into the NBLAST and XBLAST programs of Altschul, et al. (1990) J. Mol. Biol. 215:403-410. BLAST nucleotide searches can be performed with the NBLAST program, score = 100, wordlength = 12 to obtain nucleotide sequences homologous to a nucleic acid molecules of the present invention. BLAST protein searches can be performed with the XBLAST program, score = 50, wordlength = 3 to obtain amino acid sequences homologous to a protein molecules of the present invention. To obtain gapped alignments for comparison purposes, Gapped BLAST can be utilized as described in Altschul et al. (1997) Nucleic Acids Res. 25:3389-3402. Alternatively, PSI-Blast can be used to perform an iterated search which detects distant relationships between molecules. When utilizing BLAST, Gapped BLAST, and PSI-Blast programs, the default parameters of the respective programs (e.g., XBLAST and NBLAST) can be used. See http://www.ncbi.nlm.nih.gov. Another preferred, non-limiting example of a mathematical algorithm utilized for the comparison of sequences is the algorithm of Myers and Miller, (1988) Comput Appl Biosci, 4:11-7. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algorithm for identifying regions of local sequence similarity and alignment is the FASTA algorithm as described in Pearson and Lipman (1988) Proc. Natl. Acad. Sci. USA 85:2444-2448. When using the FASTA algorithm for comparing nucleotide or amino acid sequences, a PAM120 weight residue table can, for example, be used with a k-tuple value of 2.

The percent identity between two sequences can be determined using techniques similar to those described above, with or without allowing gaps. In calculating percent identity, only exact matches are counted.

The present invention also provides chimeric or fusion proteins corresponding to a biomarker protein. As used herein, a "chimeric protein" or "fusion protein" comprises all or part (preferably a biologically active part) of a polypeptide corresponding to a marker of the present invention operably linked to a heterologous polypeptide (i.e., a polypeptide other than the polypeptide corresponding to the marker). Within the fusion protein, the term "operably linked" is intended to indicate that the polypeptide of the present invention and the heterologous polypeptide are fused in-frame to each other. The heterologous polypeptide can be fused to the amino-terminus or the carboxyl-terminus of the polypeptide of the present invention.

One useful fusion protein is a GST fusion protein in which a polypeptide corresponding to a marker of the present invention is fused to the carboxyl terminus of GST sequences. Such fusion proteins can facilitate the purification of a recombinant polypeptide of the present invention.

In another embodiment, the fusion protein contains a heterologous signal sequence, immunoglobulin fusion protein, toxin, or other useful protein sequence. Chimeric and fusion proteins of the present invention can be produced by standard recombinant DNA techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and re-amplified to generate a chimeric gene sequence (see, *e.g.,* Ausubel *et al.*, *supra*). Moreover, many expression vectors are commercially available that already encode a fusion moiety (*e.g.,* a GST polypeptide). A nucleic acid encoding a polypeptide of the present invention can be cloned into such an expression vector such that the fusion moiety is linked in-frame to the polypeptide of the present invention.

A signal sequence can be used to facilitate secretion and isolation of the secreted protein or other proteins of interest. Signal sequences are typically characterized by a core of hydrophobic amino acids which are generally cleaved from the mature protein during secretion in one or more cleavage events. Such signal peptides contain processing sites that allow cleavage of the signal sequence from the mature proteins as they pass through the secretory pathway. Thus, the present invention pertains to the described polypeptides having a signal sequence, as well as to polypeptides from which the signal sequence has been proteolytically cleaved (*i.e.,* the cleavage products). In one embodiment, a nucleic acid sequence encoding a signal sequence can be operably linked in an expression vector to a protein of interest, such as a protein which is ordinarily not secreted or is otherwise difficult to isolate. The signal sequence directs secretion of the protein, such as from a eukaryotic host into which the expression vector is transformed, and the signal sequence is subsequently or concurrently cleaved. The protein can then be readily purified from the extracellular medium by art recognized methods. Alternatively, the signal sequence can be linked to the protein of interest using a sequence which facilitates purification, such as with a GST domain.

The present invention also pertains to variants of the biomarker polypeptides described herein. Such variants have an altered amino acid sequence which can function as either agonists (mimetics) or as antagonists. Variants can be generated by mutagenesis, e.g., discrete point mutation or truncation. An agonist can retain substantially the same, or a subset, of the biological activities of the naturally occurring form of the protein. An antagonist of a protein can inhibit one or more of the activities of the naturally occurring form of the protein by, for example, competitively binding to a downstream or upstream member of a cellular signaling cascade which includes the protein of interest. Thus, specific biological effects can be elicited by treatment with a variant of limited function. Treatment of a subject with a variant having a subset of the biological activities of the naturally occurring form of the protein can have fewer side effects in a subject relative to treatment with the naturally occurring form of the protein.

Variants of a biomarker protein which function as either agonists (mimetics) or as antagonists can be identified by screening combinatorial libraries of mutants, *e.g.,* truncation mutants, of the protein of the present invention for agonist or antagonist activity. In one embodiment, a variegated library of variants is generated by combinatorial mutagenesis at the nucleic acid level and is encoded by a variegated gene library. A variegated library of variants can be produced by, for example, enzymatically ligating a mixture of synthetic oligonucleotides into gene sequences such that a degenerate set of potential protein sequences is expressible as individual polypeptides, or alternatively, as a set of larger fusion proteins (*e.g.,* for phage display). There are a variety of methods which can be used to produce libraries of potential variants of the polypeptides of the present invention from a degenerate oligonucleotide sequence. Methods for synthesizing degenerate oligonucleotides are known in the art (see, *e.g.,* Narang, 1983, Tetrahedron 39:3; Itakura et al., 1984, Annu. Rev. Biochem. 53 :323; Itakura et al., 1984, Science 198:1056; Ike et al., 1983 Nucleic Acid Res. 11:477).

In addition, libraries of fragments of the coding sequence of a polypeptide corresponding to a marker of the present invention can be used to generate a variegated population of polypeptides for screening and subsequent selection of variants. For example, a library of coding sequence fragments can be generated by treating a double stranded PCR fragment of the coding sequence of interest with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes amino terminal and internal fragments of various sizes of the protein of interest.

Several techniques are known in the art for screening gene products of combinatorial libraries made by point mutations or truncation, and for screening cDNA libraries for gene products having a selected property. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify variants of a protein of the present invention (Arkin and Yourvan, 1992, Proc. Natl. Acad. Sci. USA 89:7811-7815; Delgrave et al., 1993, Protein Engineering 6(3):327- 331).

The production and use of biomarker nucleic acid and/or biomarker polypeptide molecules described herein can be facilitated by using standard recombinant techniques. In some embodiments, such techniques use vectors, preferably expression vectors, containing a nucleic acid encoding a biomarker polypeptide or a portion of such a polypeptide. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g.,* bacterial vectors having a bacterial origin of replication and episomal mammalian vectors). Other vectors (*e.g.,* non-episomal mammalian vectors) are integrated into the genome of a host cell upon introduction into the host cell, and thereby are replicated along with the host genome. Moreover, certain vectors, namely expression vectors, are capable of directing the expression of genes to which they are operably linked. In general, expression vectors of utility in recombinant DNA techniques are often in the form of plasmids (vectors). However, the present invention is intended to include such other forms of expression vectors, such as viral vectors (*e*.*g*., replication defective retroviruses, adenoviruses and adeno-associated viruses), which serve equivalent functions.

The recombinant expression vectors of the present invention comprise a nucleic acid of the present invention in a form suitable for expression of the nucleic acid in a host cell. This means that the recombinant expression vectors include one or more regulatory sequences, selected on the basis of the host cells to be used for expression, which is operably linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence *(e.g.,* in an *in vitro* transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel, Methods in Enzymology: Gene Expression Technology vol.185, Academic Press, San Diego, CA (1991). Regulatory sequences include those which direct constitutive expression of a nucleotide sequence in many types of host cell and those which direct expression of the nucleotide sequence only in certain host cells (*e.g.,* tissue-specific regulatory sequences). It will be appreciated by those skilled in the art that the design of the expression vector can depend on such factors as the choice of the host cell to be transformed, the level of expression of protein desired, and the like. The expression vectors of the present invention can be introduced into host cells to thereby produce proteins or peptides, including fusion proteins or peptides, encoded by nucleic acids as described herein.

The recombinant expression vectors for use in the present invention can be designed for expression of a polypeptide corresponding to a marker of the present invention in prokaryotic (*e.g., E. coli)* or eukaryotic cells *(e.g.,* insect cells {using baculovirus expression vectors}, yeast cells or mammalian cells). Suitable host cells are discussed further in Goeddel, *supra.* Alternatively, the recombinant expression vector can be transcribed and translated *in vitro,* for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988, Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Examples of suitable inducible non-fusion *E. coli* expression vectors include pTrc (Amann et al., 1988, Gene 69:301-315) and pET 11d (Studier *et al.*, p. 60-89, In Gene Expression Technology: Methods in Enzymology vol.185, Academic Press, San Diego, CA, 1991). Target biomarker nucleic acid expression from the pTrc vector relies on host RNA polymerase transcription from a hybrid trp-lac fusion promoter. Target biomarker nucleic acid expression from the pET 11d vector relies on transcription from a T7 gn10-lac fusion promoter mediated by a co-expressed viral RNA polymerase (T7 gn1). This viral polymerase is supplied by host strains BL21 (DE3) or HMS174(DE3) from a resident prophage harboring a T7 gn1 gene under the transcriptional control of the lacUV 5 promoter.

One strategy to maximize recombinant protein expression in *E. coli* is to express the protein in a host bacterium with an impaired capacity to proteolytically cleave the recombinant protein (Gottesman, p. 119-128, In Gene Expression Technology: Methods in Enzymology vol. 185, Academic Press, San Diego, CA, 1990. Another strategy is to alter the nucleic acid sequence of the nucleic acid to be inserted into an expression vector so that the individual codons for each amino acid are those preferentially utilized in *E. coli* (Wada et al., 1992, Nucleic Acids Res. 20:2111-2118). Such alteration of nucleic acid sequences of the present invention can be carried out by standard DNA synthesis techniques.

In another embodiment, the expression vector is a yeast expression vector. Examples of vectors for expression in yeast *S. cerevisiae* include pYepSec1 (Baldari et al., 1987, EMBO J. 6:229-234), pMFa (Kurjan and Herskowitz, 1982, Cell 30:933-943), pJRY88 (Schultz et al., 1987, Gene 54:113-123), pYES2 (Invitrogen Corporation, San Diego, CA), and pPicZ (Invitrogen Corp, San Diego, CA).

Alternatively, the expression vector is a baculovirus expression vector. Baculovirus vectors available for expression of proteins in cultured insect cells *(e.g.,* Sf 9 cells) include the pAc series (Smith et al., 1983, Mol. Cell Biol. 3:2156-2165) and the pVL series (Lucklow and Summers, 1989, Virology 170:31-39).

In yet another embodiment, a nucleic acid of the present invention is expressed in mammalian cells using a mammalian expression vector. Examples of mammalian expression vectors include pCDM8 (Seed, 1987, Nature 329:840) and pMT2PC (Kaufman et al., 1987, EMBO J. 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook *et al.*, *supra.*

In another embodiment, the recombinant mammalian expression vector is capable of directing expression of the nucleic acid preferentially in a particular cell type (*e.g.,* tissue-specific regulatory elements are used to express the nucleic acid). Tissue-specific regulatory elements are known in the art. Non-limiting examples of suitable tissue-specific promoters include the albumin promoter (liver-specific; Pinkert et al., 1987, Genes Dev. 1:268-277), lymphoid-specific promoters (Calame and Eaton, 1988, Adv. Immunol. 43:235-275), in particular promoters of T cell receptors (Winoto and Baltimore, 1989, EMBO J. 8:729-733) and immunoglobulins (Banerji et al., 1983, Cell 33:729-740; Queen and Baltimore, 1983, Cell 33:741-748), neuron-specific promoters *(e.g.,* the neurofilament promoter; Byrne and Ruddle, 1989, Proc. Natl. Acad. Sci. USA 86:5473-5477), pancreas-specific promoters (Edlund et al., 1985, Science 230:912-916), and mammary gland-specific promoters (*e.g.,* milk whey promoter; U.S. Patent No. 4,873,316 and European Application Publication No. 264,166). Developmentally-regulated promoters are also encompassed, for example the murine hox promoters (Kessel and Gruss, 1990, Science 249:374-379) and the α-fetoprotein promoter (Camper and Tilghman, 1989, Genes Dev. 3:537-546).

The present invention further provides a recombinant expression vector comprising a DNA molecule cloned into the expression vector in an antisense orientation. That is, the DNA molecule is operably linked to a regulatory sequence in a manner which allows for expression (by transcription of the DNA molecule) of an RNA molecule which is antisense to the mRNA encoding a polypeptide of the present invention. Regulatory sequences operably linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue-specific or cell type specific expression of antisense RNA. The antisense expression vector can be in the form of a recombinant plasmid, phagemid, or attenuated virus in which antisense nucleic acids are produced under the control of a high efficiency regulatory region, the activity of which can be determined by the cell type into which the vector is introduced. For a discussion of the regulation of gene expression using antisense genes (see Weintraub et al., 1986, Trends in Genetics, Vol. 1(1)).

Another aspect of the present invention pertains to host cells into which a recombinant expression vector of the present invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic (*e.g., E. coli*) or eukaryotic cell *(e.g.,* insect cells, yeast or mammalian cells).

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid into a host cell, including calcium phosphate or calcium chloride coprecipitation, DEAE-dextran-mediated transfection, lipofection, or electroporation. Suitable methods for transforming or transfecting host cells can be found in Sambrook, *et al.* (*supra*), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker *(e.g.,* for resistance to antibiotics) is generally introduced into the host cells along with the gene of interest. Preferred selectable markers include those which confer resistance to drugs, such as G418, hygromycin and methotrexate. Cells stably transfected with the introduced nucleic acid can be identified by drug selection (e.g., cells that have incorporated the selectable marker gene will survive, while the other cells die).

### V. Analyzing Biomarker Nucleic Acids and Polypeptides

Biomarker nucleic acids and/or biomarker polypeptides can be analyzed according to the methods described herein and techniques known to the skilled artisan to identify such genetic or expression alterations useful for the present invention including, but not limited to, 1) an alteration in the level of a biomarker transcript or polypeptide, 2) a deletion or addition of one or more nucleotides from a biomarker gene, 4) a substitution of one or more nucleotides of a biomarker gene, 5) aberrant modification of a biomarker gene, such as an expression regulatory region, and the like.

### a. Methods for Detection of Copy Number

Methods of evaluating the copy number of a biomarker nucleic acid are well known to those of skill in the art. The presence or absence of chromosomal gain or loss can be evaluated simply by a determination of copy number of the regions or markers identified herein.

In one embodiment, a biological sample is tested for the presence of copy number changes in genomic loci containing the genomic marker. A copy number of at least 3, 4, 5, 6, 7, 8, 9, or 10 is predictive of poorer outcome of anti-immune checkpoint treatment.

Methods of evaluating the copy number of a biomarker locus include, but are not limited to, hybridization-based assays. Hybridization-based assays include, but are not limited to, traditional "direct probe" methods, such as Southern blots, *in situ* hybridization (*e.g.,* FISH and FISH plus SKY) methods, and "comparative probe" methods, such as comparative genomic hybridization (CGH), *e.g.,* cDNA-based or oligonucleotide-based CGH. The methods can be used in a wide variety of formats including, but not limited to, substrate (*e.g.* membrane or glass) bound methods or array-based approaches.

In one embodiment, evaluating the biomarker gene copy number in a sample involves a Southern Blot. In a Southern Blot, the genomic DNA (typically fragmented and separated on an electrophoretic gel) is hybridized to a probe specific for the target region. Comparison of the intensity of the hybridization signal from the probe for the target region with control probe signal from analysis of normal genomic DNA (*e.g.,* a non-amplified portion of the same or related cell, tissue, organ, etc.) provides an estimate of the relative copy number of the target nucleic acid. Alternatively, a Northern blot may be utilized for evaluating the copy number of encoding nucleic acid in a sample. In a Northern blot, mRNA is hybridized to a probe specific for the target region. Comparison of the intensity of the hybridization signal from the probe for the target region with control probe signal from analysis of normal RNA (*e.g.,* a non-amplified portion of the same or related cell, tissue, organ, *etc*.) provides an estimate of the relative copy number of the target nucleic acid. Alternatively, other methods well known in the art to detect RNA can be used, such that higher or lower expression relative to an appropriate control (*e.g.,* a non-amplified portion of the same or related cell tissue, organ, etc.) provides an estimate of the relative copy number of the target nucleic acid.

An alternative means for determining genomic copy number is *in situ* hybridization *(e.g.,* Angerer (1987) Meth. Enzymol 152: 649). Generally, *in situ* hybridization comprises the following steps: (1) fixation of tissue or biological structure to be analyzed; (2) prehybridization treatment of the biological structure to increase accessibility of target DNA, and to reduce nonspecific binding; (3) hybridization of the mixture of nucleic acids to the nucleic acid in the biological structure or tissue; (4) post-hybridization washes to remove nucleic acid fragments not bound in the hybridization and (5) detection of the hybridized nucleic acid fragments. The reagent used in each of these steps and the conditions for use vary depending on the particular application. In a typical *in situ* hybridization assay, cells are fixed to a solid support, typically a glass slide. If a nucleic acid is to be probed, the cells are typically denatured with heat or alkali. The cells are then contacted with a hybridization solution at a moderate temperature to permit annealing of labeled probes specific to the nucleic acid sequence encoding the protein. The targets (*e.g.,* cells) are then typically washed at a predetermined stringency or at an increasing stringency until an appropriate signal to noise ratio is obtained. The probes are typically labeled, *e.g.,* with radioisotopes or fluorescent reporters. In one embodiment, probes are sufficiently long so as to specifically hybridize with the target nucleic acid(s) under stringent conditions. Probes generally range in length from about 200 bases to about 1000 bases. In some applications it is necessary to block the hybridization capacity of repetitive sequences. Thus, in some embodiments, tRNA, human genomic DNA, or Cot-I DNA is used to block non-specific hybridization.

An alternative means for determining genomic copy number is comparative genomic hybridization. In general, genomic DNA is isolated from normal reference cells, as well as from test cells (*e.g.,* tumor cells) and amplified, if necessary. The two nucleic acids are differentially labeled and then hybridized *in situ* to metaphase chromosomes of a reference cell. The repetitive sequences in both the reference and test DNAs are either removed or their hybridization capacity is reduced by some means, for example by prehybridization with appropriate blocking nucleic acids and/or including such blocking nucleic acid sequences for said repetitive sequences during said hybridization. The bound, labeled DNA sequences are then rendered in a visualizable form, if necessary. Chromosomal regions in the test cells which are at increased or decreased copy number can be identified by detecting regions where the ratio of signal from the two DNAs is altered. For example, those regions that have decreased in copy number in the test cells will show relatively lower signal from the test DNA than the reference compared to other regions of the genome. Regions that have been increased in copy number in the test cells will show relatively higher signal from the test DNA. Where there are chromosomal deletions or multiplications, differences in the ratio of the signals from the two labels will be detected and the ratio will provide a measure of the copy number. In another embodiment of CGH, array CGH (aCGH), the immobilized chromosome element is replaced with a collection of solid support bound target nucleic acids on an array, allowing for a large or complete percentage of the genome to be represented in the collection of solid support bound targets. Target nucleic acids may comprise cDNAs, genomic DNAs, oligonucleotides (*e.g.,* to detect single nucleotide polymorphisms) and the like. Array-based CGH may also be performed with single-color labeling (as opposed to labeling the control and the possible tumor sample with two different dyes and mixing them prior to hybridization, which will yield a ratio due to competitive hybridization of probes on the arrays). In single color CGH, the control is labeled and hybridized to one array and absolute signals are read, and the possible tumor sample is labeled and hybridized to a second array (with identical content) and absolute signals are read. Copy number difference is calculated based on absolute signals from the two arrays. Methods of preparing immobilized chromosomes or arrays and performing comparative genomic hybridization are well known in the art (see, *e.g.,* U.S. Pat. Nos: 6,335,167; 6,197,501; 5,830,645; and 5,665,549 and Albertson (1984) EMBO J. 3: 1227-1234; Pinkel (1988) Proc. Natl. Acad. Sci. USA 85: 9138-9142; EPO Pub. No. 430,402; Methods in Molecular Biology, Vol. 33: In situ Hybridization Protocols, Choo, ed., Humana Press, Totowa, N.J. (1994), *etc.*) In another embodiment, the hybridization protocol of Pinkel, et al. (1998) Nature Genetics 20: 207-211, or of Kallioniemi (1992) Proc. Natl Acad Sci USA 89:5321-5325 (1992) is used.

In still another embodiment, amplification-based assays can be used to measure copy number. In such amplification-based assays, the nucleic acid sequences act as a template in an amplification reaction (*e.g.*, Polymerase Chain Reaction (PCR). In a quantitative amplification, the amount of amplification product will be proportional to the amount of template in the original sample. Comparison to appropriate controls, e.g. healthy tissue, provides a measure of the copy number.

Methods of "quantitative" amplification are well known to those of skill in the art. For example, quantitative PCR involves simultaneously co-amplifying a known quantity of a control sequence using the same primers. This provides an internal standard that may be used to calibrate the PCR reaction. Detailed protocols for quantitative PCR are provided in Innis, et al. (1990) PCR Protocols, A Guide to Methods and Applications, Academic Press, Inc. N.Y.). Measurement of DNA copy number at microsatellite loci using quantitative PCR analysis is described in Ginzonger, et al. (2000) Cancer Research 60:5405-5409. The known nucleic acid sequence for the genes is sufficient to enable one of skill in the art to routinely select primers to amplify any portion of the gene. Fluorogenic quantitative PCR may also be used in the methods of the present invention. In fluorogenic quantitative PCR, quantitation is based on amount of fluorescence signals, *e.g.,* TaqMan and SYBR green.

Other suitable amplification methods include, but are not limited to, ligase chain reaction (LCR) (see Wu and Wallace (1989) Genomics 4: 560, Landegren, et al. (1988) Science 241:1077, and Barringer et al. (1990) Gene 89: 117), transcription amplification (Kwoh, et al. (1989) Proc. Natl. Acad. Sci. USA 86: 1173), self-sustained sequence replication (Guatelli, et al. (1990) Proc. Nat. Acad. Sci. USA 87: 1874), dot PCR, and linker adapter PCR, *etc.*

Loss of heterozygosity (LOH) and major copy proportion (MCP) mapping (Wang, Z.C., et al. (2004) Cancer Res 64(1):64-71; Seymour, A. B., et al. (1994) Cancer Res 54, 2761-4; Hahn, S. A., et al. (1995) Cancer Res 55, 4670-5; Kimura, M., et al. (1996) Genes Chromosomes Cancer 17, 88-93; Li et al., (2008) MBC Bioinform. 9, 204-219) may also be used to identify regions of amplification or deletion.

### b. Methods for Detection of Biomarker Nucleic Acid Expression

Biomarker expression may be assessed by any of a wide variety of well known methods for detecting expression of a transcribed molecule or protein. Non-limiting examples of such methods include immunological methods for detection of secreted, cell-surface, cytoplasmic, or nuclear proteins, protein purification methods, protein function or activity assays, nucleic acid hybridization methods, nucleic acid reverse transcription methods, and nucleic acid amplification methods.

In preferred embodiments, activity of a particular gene is characterized by a measure of gene transcript (*e.g.* mRNA), by a measure of the quantity of translated protein, or by a measure of gene product activity. Marker expression can be monitored in a variety of ways, including by detecting mRNA levels, protein levels, or protein activity, any of which can be measured using standard techniques. Detection can involve quantification of the level of gene expression (*e.g.,* genomic DNA, cDNA, mRNA, protein, or enzyme activity), or, alternatively, can be a qualitative assessment of the level of gene expression, in particular in comparison with a control level. The type of level being detected will be clear from the context.

In another embodiment, detecting or determining expression levels of a biomarker and functionally similar homologs thereof, including a fragment or genetic alteration thereof (*e.g.,* in regulatory or promoter regions thereof) comprises detecting or determining RNA levels for the marker of interest. In one embodiment, one or more cells from the subject to be tested are obtained and RNA is isolated from the cells. In a preferred embodiment, a sample of breast tissue cells is obtained from the subject.

In one embodiment, RNA is obtained from a single cell. For example, a cell can be isolated from a tissue sample by laser capture microdissection (LCM). Using this technique, a cell can be isolated from a tissue section, including a stained tissue section, thereby assuring that the desired cell is isolated (see, *e.g.,* Bonner et al. (1997) Science 278: 1481; Emmert-Buck et al. (1996) Science 274:998; Fend et al. (1999) Am. J. Path. 154: 61 and Murakami et al. (2000) Kidney Int. 58:1346). For example, Murakami et al., *supra,* describe isolation of a cell from a previously immunostained tissue section.

It is also be possible to obtain cells from a subject and culture the cells *in vitro,* such as to obtain a larger population of cells from which RNA can be extracted. Methods for establishing cultures of non-transformed cells, i.e., primary cell cultures, are known in the art.

When isolating RNA from tissue samples or cells from individuals, it may be important to prevent any further changes in gene expression after the tissue or cells has been removed from the subject. Changes in expression levels are known to change rapidly following perturbations, *e.g.,* heat shock or activation with lipopolysaccharide (LPS) or other reagents. In addition, the RNA in the tissue and cells may quickly become degraded. Accordingly, in a preferred embodiment, the tissue or cells obtained from a subject is snap frozen as soon as possible.

RNA can be extracted from the tissue sample by a variety of methods, e.g., the guanidium thiocyanate lysis followed by CsCI centrifugation (Chirgwin et al., 1979, Biochemistry 18:5294-5299). RNA from single cells can be obtained as described in methods for preparing cDNA libraries from single cells, such as those described in Dulac, C. (1998) Curr. Top. Dev. Biol. 36, 245 and Jena et al. (1996) J. Immunol. Methods 190:199. Care to avoid RNA degradation must be taken, e.g., by inclusion of RNAsin.

The RNA sample can then be enriched in particular species. In one embodiment, poly(A)+ RNA is isolated from the RNA sample. In general, such purification takes advantage of the poly-A tails on mRNA. In particular and as noted above, poly-T oligonucleotides may be immobilized within on a solid support to serve as affinity ligands for mRNA. Kits for this purpose are commercially available, e.g., the MessageMaker kit (Life Technologies, Grand Island, NY).

In a preferred embodiment, the RNA population is enriched in marker sequences. Enrichment can be undertaken, *e.g.,* by primer-specific cDNA synthesis, or multiple rounds of linear amplification based on cDNA synthesis and template-directed *in vitro* transcription (*see, e.g.,* Wang et al. (1989) PNAS 86, 9717; Dulac et al., *supra,* and Jena et al., *supra*).

The population of RNA, enriched or not in particular species or sequences, can further be amplified. As defined herein, an "amplification process" is designed to strengthen, increase, or augment a molecule within the RNA. For example, where RNA is mRNA, an amplification process such as RT-PCR can be utilized to amplify the mRNA, such that a signal is detectable or detection is enhanced. Such an amplification process is beneficial particularly when the biological, tissue, or tumor sample is of a small size or volume.

Various amplification and detection methods can be used. For example, it is within the scope of the present invention to reverse transcribe mRNA into cDNA followed by polymerase chain reaction (RT-PCR); or, to use a single enzyme for both steps as described in U.S. Pat. No. 5,322,770, or reverse transcribe mRNA into cDNA followed by symmetric gap ligase chain reaction (RT-AGLCR) as described by R. L. Marshall, et al., PCR Methods and Applications 4: 80-84 (1994). Real time PCR may also be used.

Other known amplification methods which can be utilized herein include but are not limited to the so-called "NASBA" or "3SR" technique described in PNAS USA 87: 1874-1878 (1990) and also described in Nature 350 (No. 6313): 91-92 (1991); Q-beta amplification as described in published European Patent Application (EPA) No. 4544610; strand displacement amplification (as described in G. T. Walker et al., Clin. Chem. 42: 9-13 (1996) and European Patent Application No. 684315; target mediated amplification, as described by PCT Publication WO9322461; PCR; ligase chain reaction (LCR) (see, *e.g.,* Wu and Wallace, Genomics 4, 560 (1989), Landegren et al., Science 241, 1077 (1988)); self-sustained sequence replication (SSR) (see, *e.g.,* Guatelli et al., Proc. Nat. Acad. Sci. USA, 87, 1874 (1990)); and transcription amplification (see, *e.g.,* Kwoh et al., Proc. Natl. Acad. Sci. USA 86, 1173 (1989)).

Many techniques are known in the state of the art for determining absolute and relative levels of gene expression, commonly used techniques suitable for use in the present invention include Northern analysis, RNase protection assays (RPA), microarrays and PCR-based techniques, such as quantitative PCR and differential display PCR. For example, Northern blotting involves running a preparation of RNA on a denaturing agarose gel, and transferring it to a suitable support, such as activated cellulose, nitrocellulose or glass or nylon membranes. Radiolabeled cDNA or RNA is then hybridized to the preparation, washed and analyzed by autoradiography.

*In situ* hybridization visualization may also be employed, wherein a radioactively labeled antisense RNA probe is hybridized with a thin section of a biopsy sample, washed, cleaved with RNase and exposed to a sensitive emulsion for autoradiography. The samples may be stained with hematoxylin to demonstrate the histological composition of the sample, and dark field imaging with a suitable light filter shows the developed emulsion. Nonradioactive labels such as digoxigenin may also be used.

Alternatively, mRNA expression can be detected on a DNA array, chip or a microarray. Labeled nucleic acids of a test sample obtained from a subject may be hybridized to a solid surface comprising biomarker DNA. Positive hybridization signal is obtained with the sample containing biomarker transcripts. Methods of preparing DNA arrays and their use are well known in the art (see, *e.g.,* U.S. Pat. Nos: 6,618,6796; 6,379,897; 6,664,377; 6,451,536; 548,257; U.S. 20030157485 and Schena et al. (1995) Science 20, 467-470; Gerhold et al. (1999) Trends In Biochem. Sci. 24, 168-173; and Lennon et al. (2000) Drug Discovery Today 5, 59-65). Serial Analysis of Gene Expression (SAGE) can also be performed (See for example U.S. Patent Application 20030215858).

To monitor mRNA levels, for example, mRNA is extracted from the biological sample to be tested, reverse transcribed, and fluorescently-labeled cDNA probes are generated. The microarrays capable of hybridizing to marker cDNA are then probed with the labeled cDNA probes, the slides scanned and fluorescence intensity measured. This intensity correlates with the hybridization intensity and expression levels.

Types of probes that can be used in the methods described herein include cDNA, riboprobes, synthetic oligonucleotides and genomic probes. The type of probe used will generally be dictated by the particular situation, such as riboprobes for *in situ* hybridization, and cDNA for Northern blotting, for example. In one embodiment, the probe is directed to nucleotide regions unique to the RNA. The probes may be as short as is required to differentially recognize marker mRNA transcripts, and may be as short as, for example, 15 bases; however, probes of at least 17, 18, 19 or 20 or more bases can be used. In one embodiment, the primers and probes hybridize specifically under stringent conditions to a DNA fragment having the nucleotide sequence corresponding to the marker. As herein used, the term "stringent conditions" means hybridization will occur only if there is at least 95% identity in nucleotide sequences. In another embodiment, hybridization under "stringent conditions" occurs when there is at least 97% identity between the sequences.

The form of labeling of the probes may be any that is appropriate, such as the use of radioisotopes, for example, ³²P and ³⁵S. Labeling with radioisotopes may be achieved, whether the probe is synthesized chemically or biologically, by the use of suitably labeled bases.

In one embodiment, the biological sample contains polypeptide molecules from the test subject. Alternatively, the biological sample can contain mRNA molecules from the test subject or genomic DNA molecules from the test subject.

In another embodiment, the methods further involve obtaining a control biological sample from a control subject, contacting the control sample with a compound or agent capable of detecting marker polypeptide, mRNA, genomic DNA, or fragments thereof, such that the presence of the marker polypeptide, mRNA, genomic DNA, or fragments thereof, is detected in the biological sample, and comparing the presence of the marker polypeptide, mRNA, genomic DNA, or fragments thereof, in the control sample with the presence of the marker polypeptide, mRNA, genomic DNA, or fragments thereof in the test sample.

### c. Methods for Detection of Biomarker Protein Expression

The activity or level of a biomarker protein can be detected and/or quantified by detecting or quantifying the expressed polypeptide. The polypeptide can be detected and quantified by any of a number of means well known to those of skill in the art. Aberrant levels of polypeptide expression of the polypeptides encoded by a biomarker nucleic acid and functionally similar homologs thereof, including a fragment or genetic alteration thereof (*e.g.,* in regulatory or promoter regions thereof) are associated with the likelihood of response of a cancer to an anti-immune checkpoint therapy. Any method known in the art for detecting polypeptides can be used. Such methods include, but are not limited to, immunodiffusion, immunoelectrophoresis, radioimmunoassay (RIA), enzyme-linked immunosorbent assays (ELISAs), immunofluorescent assays, Western blotting, binder-ligand assays, immunohistochemical techniques, agglutination, complement assays, high performance liquid chromatography (HPLC), thin layer chromatography (TLC), hyperdiffusion chromatography, and the like (*e.g.,* Basic and Clinical Immunology, Sites and Terr, eds., Appleton and Lange, Norwalk, Conn. pp 217-262,). Preferred are binder-ligand immunoassay methods including reacting antibodies with an epitope or epitopes and competitively displacing a labeled polypeptide or derivative thereof.

For example, ELISA and RIA procedures may be conducted such that a desired biomarker protein standard is labeled (with a radioisotope such as ¹²⁵I or ³⁵S, or an assayable enzyme, such as horseradish peroxidase or alkaline phosphatase), and, together with the unlabelled sample, brought into contact with the corresponding antibody, whereon a second antibody is used to bind the first, and radioactivity or the immobilized enzyme assayed (competitive assay). Alternatively, the biomarker protein in the sample is allowed to react with the corresponding immobilized antibody, radioisotope- or enzyme-labeled anti-biomarker proteinantibody is allowed to react with the system, and radioactivity or the enzyme assayed (ELISA-sandwich assay). Other conventional methods may also be employed as suitable.

The above techniques may be conducted essentially as a "one-step" or "two-step" assay. A "one-step" assay involves contacting antigen with immobilized antibody and, without washing, contacting the mixture with labeled antibody. A "two-step" assay involves washing before contacting, the mixture with labeled antibody. Other conventional methods may also be employed as suitable.

In one embodiment, a method for measuring biomarker protein levels comprises the steps of: contacting a biological specimen with an antibody or variant (e.g., fragment) thereof which selectively binds the biomarker protein, and detecting whether said antibody or variant thereof is bound to said sample and thereby measuring the levels of the biomarker protein.

Enzymatic and radiolabeling of biomarker protein and/or the antibodies may be effected by conventional means. Such means will generally include covalent linking of the enzyme to the antigen or the antibody in question, such as by glutaraldehyde, specifically so as not to adversely affect the activity of the enzyme, by which is meant that the enzyme must still be capable of interacting with its substrate, although it is not necessary for all of the enzyme to be active, provided that enough remains active to permit the assay to be effected. Indeed, some techniques for binding enzyme are non-specific (such as using formaldehyde), and will only yield a proportion of active enzyme.

It is usually desirable to immobilize one component of the assay system on a support, thereby allowing other components of the system to be brought into contact with the component and readily removed without laborious and time-consuming labor. It is possible for a second phase to be immobilized away from the first, but one phase is usually sufficient.

It is possible to immobilize the enzyme itself on a support, but if solid-phase enzyme is required, then this is generally best achieved by binding to antibody and affixing the antibody to a support, models and systems for which are well-known in the art. Simple polyethylene may provide a suitable support.

Enzymes employable for labeling are not particularly limited, but may be selected from the members of the oxidase group, for example. These catalyze production of hydrogen peroxide by reaction with their substrates, and glucose oxidase is often used for its good stability, ease of availability and cheapness, as well as the ready availability of its substrate (glucose). Activity of the oxidase may be assayed by measuring the concentration of hydrogen peroxide formed after reaction of the enzyme-labeled antibody with the substrate under controlled conditions well-known in the art.

Other techniques may be used to detect biomarker protein according to a practitioner's preference based upon the present disclosure. One such technique is Western blotting (Towbin et at., Proc. Nat. Acad. Sci. 76:4350 (1979)), wherein a suitably treated sample is run on an SDS-PAGE gel before being transferred to a solid support, such as a nitrocellulose filter. Anti-biomarker protein antibodies (unlabeled) are then brought into contact with the support and assayed by a secondary immunological reagent, such as labeled protein A or anti-immunoglobulin (suitable labels including ¹²⁵I, horseradish peroxidase and alkaline phosphatase). Chromatographic detection may also be used.

Immunohistochemistry may be used to detect expression of biomarker protein, e.g., in a biopsy sample. A suitable antibody is brought into contact with, for example, a thin layer of cells, washed, and then contacted with a second, labeled antibody. Labeling may be by fluorescent markers, enzymes, such as peroxidase, avidin, or radiolabelling. The assay is scored visually, using microscopy.

Anti-biomarker protein antibodies, such as intrabodies, may also be used for imaging purposes, for example, to detect the presence of biomarker protein in cells and tissues of a subject. Suitable labels include radioisotopes, iodine (¹²⁵I, ¹²¹I), carbon (¹⁴C), sulphur (³⁵S), tritium (³H), indium (¹¹²In), and technetium (⁹⁹mTc), fluorescent labels, such as fluorescein and rhodamine, and biotin.

For *in vivo* imaging purposes, antibodies are not detectable, as such, from outside the body, and so must be labeled, or otherwise modified, to permit detection. Markers for this purpose may be any that do not substantially interfere with the antibody binding, but which allow external detection. Suitable markers may include those that may be detected by X-radiography, NMR or MRI. For X-radiographic techniques, suitable markers include any radioisotope that emits detectable radiation but that is not overtly harmful to the subject, such as barium or cesium, for example. Suitable markers for NMR and MRI generally include those with a detectable characteristic spin, such as deuterium, which may be incorporated into the antibody by suitable labeling of nutrients for the relevant hybridoma, for example.

The size of the subject, and the imaging system used, will determine the quantity of imaging moiety needed to produce diagnostic images. In the case of a radioisotope moiety, for a human subject, the quantity of radioactivity injected will normally range from about 5 to 20 millicuries of technetium-99. The labeled antibody or antibody fragment will then preferentially accumulate at the location of cells which contain biomarker protein. The labeled antibody or antibody fragment can then be detected using known techniques.

Antibodies that may be used to detect biomarker protein include any antibody, whether natural or synthetic, full length or a fragment thereof, monoclonal or polyclonal, that binds sufficiently strongly and specifically to the biomarker protein to be detected. An antibody may have a K_{d} of at most about 10⁻⁶M, 10⁻⁷M, 10⁻⁸M, 10⁻⁹M, 10⁻¹⁰M, 10⁻¹¹M, 10⁻¹²M. The phrase "specifically binds" refers to binding of, for example, an antibody to an epitope or antigen or antigenic determinant in such a manner that binding can be displaced or competed with a second preparation of identical or similar epitope, antigen or antigenic determinant. An antibody may bind preferentially to the biomarker protein relative to other proteins, such as related proteins.

Antibodies are commercially available or may be prepared according to methods known in the art.

Antibodies and derivatives thereof that may be used encompass polyclonal or monoclonal antibodies, chimeric, human, humanized, primatized (CDR-grafted), veneered or single-chain antibodies as well as functional fragments, *i.e.,* biomarker protein binding fragments, of antibodies. For example, antibody fragments capable of binding to a biomarker protein or portions thereof, including, but not limited to, Fv, Fab, Fab' and F(ab') 2 fragments can be used. Such fragments can be produced by enzymatic cleavage or by recombinant techniques. For example, papain or pepsin cleavage can generate Fab or F(ab') 2 fragments, respectively. Other proteases with the requisite substrate specificity can also be used to generate Fab or F(ab') 2 fragments. Antibodies can also be produced in a variety of truncated forms using antibody genes in which one or more stop codons have been introduced upstream of the natural stop site. For example, a chimeric gene encoding a F(ab') 2 heavy chain portion can be designed to include DNA sequences encoding the CH, domain and hinge region of the heavy chain.

Synthetic and engineered antibodies are described in, *e.g.,* Cabilly et al., U.S. Pat. No. 4,816,567 Cabilly et al., European Patent No. 0,125,023 B1; Boss et al., U.S. Pat. No. 4,816,397; Boss et al., European Patent No. 0,120,694 B1; Neuberger, M. S. et al., WO 86/01533; Neuberger, M. S. et al., European Patent No. 0,194,276 B1; Winter, U.S. Pat. No. 5,225,539; Winter, European Patent No. 0,239,400 B1; Queen et al., European Patent No. 0451216 B1; and Padlan, E. A. et al., EP 0519596 A1. See also, Newman, R. et al., BioTechnology, 10: 1455-1460 (1992), regarding primatized antibody, and Ladner et al., U.S. Pat. No. 4,946,778 and Bird, R. E. et al., Science, 242: 423-426 (1988)) regarding single-chain antibodies. Antibodies produced from a library, e.g., phage display library, may also be used.

In some embodiments, agents that specifically bind to a biomarker protein other than antibodies are used, such as peptides. Peptides that specifically bind to a biomarker protein can be identified by any means known in the art. For example, specific peptide binders of a biomarker protein can be screened for using peptide phage display libraries.

### d. Methods for Detection of Biomarker Structural Alterations

The following illustrative methods can be used to identify the presence of a structural alteration in a biomarker nucleic acid and/or biomarker polypeptide molecule in order to, for example, identify Ang-2 proteins that are both overexpressed and functional.

In certain embodiments, detection of the alteration involves the use of a probe/primer in a polymerase chain reaction (PCR) (see, *e.g.,* U.S. Pat. Nos. 4,683,195 and 4,683,202), such as anchor PCR or RACE PCR, or, alternatively, in a ligation chain reaction (LCR) (see, *e.g.,* Landegran et al. (1988) Science 241:1077-1080; and Nakazawa et al. (1994) Proc. Natl. Acad. Sci. USA 91:360-364), the latter of which can be particularly useful for detecting point mutations in a biomarker nucleic acid such as a biomarker gene (see Abravaya et al. (1995) Nucleic Acids Res. 23:675-682). This method can include the steps of collecting a sample of cells from a subject, isolating nucleic acid (e.g., genomic, mRNA or both) from the cells of the sample, contacting the nucleic acid sample with one or more primers which specifically hybridize to a biomarker gene under conditions such that hybridization and amplification of the biomarker gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J. C. et al. (1990) Proc. Natl. Acad. Sci. USA 87:1874-1878), transcriptional amplification system (Kwoh, D. Y. et al. (1989) Proc. Natl. Acad. Sci. USA 86:1173-1177), Q-Beta Replicase (Lizardi, P. M. et al. (1988) Bio-Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a biomarker nucleic acid from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Pat. No. 5,498,531) can be used to score for the presence of specific mutations by development or loss of a ribozyme cleavage site.

In other embodiments, genetic mutations in biomarker nucleic acid can be identified by hybridizing a sample and control nucleic acids, e.g., DNA or RNA, to high density arrays containing hundreds or thousands of oligonucleotide probes (Cronin, M. T. et al. (1996) Hum. Mutat. 7:244-255; Kozal, M. J. et al. (1996) Nat. Med. 2:753-759). For example, biomarker genetic mutations can be identified in two dimensional arrays containing light-generated DNA probes as described in Cronin *et al.* (1996) supra. Briefly, a first hybridization array of probes can be used to scan through long stretches of DNA in a sample and control to identify base changes between the sequences by making linear arrays of sequential, overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene. Such biomarker genetic mutations can be identified in a variety of contexts, including, for example, germline and somatic mutations.

In yet another embodiment, any of a variety of sequencing reactions known in the art can be used to directly sequence a biomarker gene and detect mutations by comparing the sequence of the sample biomarker with the corresponding wild-type (control) sequence. Examples of sequencing reactions include those based on techniques developed by Maxam and Gilbert (1977) Proc. Natl. Acad. Sci. USA 74:560 or Sanger (1977) Proc. Natl. Acad Sci. USA 74:5463. It is also contemplated that any of a variety of automated sequencing procedures can be utilized when performing the diagnostic assays (Naeve (1995) Biotechniques 19:448-53), including sequencing by mass spectrometry (see, *e.g.,* PCT International Publication No. WO 94/16101; Cohen et al. (1996) Adv. Chromatogr. 36:127-162; and Griffin et al. (1993) Appl. Biochem. Biotechnol. 38:147-159).

Other methods for detecting mutations in a biomarker gene include methods in which protection from cleavage agents is used to detect mismatched bases in RNA/RNA or RNA/DNA heteroduplexes (Myers et al. (1985) Science 230:1242). In general, the art technique of "mismatch cleavage" starts by providing heteroduplexes formed by hybridizing (labeled) RNA or DNA containing the wild-type biomarker sequence with potentially mutant RNA or DNA obtained from a tissue sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with SI nuclease to enzymatically digest the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al. (1988) Proc. Natl. Acad. Sci. USA 85:4397 and Saleeba et al. (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in biomarker cDNAs obtained from samples of cells. For example, the mutY enzyme of *E. coli* cleaves A at G/A mismatches and the thymidine DNA glycosylase from HeLa cells cleaves T at G/T mismatches (Hsu et al. (1994) Carcinogenesis 15:1657-1662). According to an exemplary embodiment, a probe based on a biomarker sequence, *e.g.,* a wild-type biomarker treated with a DNA mismatch repair enzyme, and the cleavage products, if any, can be detected from electrophoresis protocols or the like (*e.g.,* U.S. Pat. No. 5,459,039.)

In other embodiments, alterations in electrophoretic mobility can be used to identify mutations in biomarker genes. For example, single strand conformation polymorphism (SSCP) may be used to detect differences in electrophoretic mobility between mutant and wild type nucleic acids (Orita et al. (1989) Proc Natl. Acad. Sci USA 86:2766; see also Cotton (1993) Mutat. Res. 285:125-144 and Hayashi (1992) Genet. Anal. Tech. Appl. 9:73-79). Single-stranded DNA fragments of sample and control biomarker nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet. 7:5)*.*

In yet another embodiment the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to ensure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) Biophys. Chem. 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) Nature 324:163; Saiki et al. (1989) Proc. Natl. Acad. Sci. USA 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) Nucleic Acids Res. 17:2437-2448) or at the extreme 3' end of one primer where, under appropriate conditions, mismatch can prevent, or reduce polymerase extension (Prossner (1993) Tibtech 11:238). In addition it may be desirable to introduce a novel restriction site in the region of the mutation to create cleavage-based detection (Gasparini et al. (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

### 3. Anti-Cancer Therapies

The efficacy of anti-immune checkpoint therapy is predicted according to biomarker amount and/or activity associated with a cancer in a subject according to the methods described herein. In one embodiment, such anti-immune checkpoint therapy or combinations of therapies (*e.g.,* anti-CTLA-4 antibodies in combination with bevacizumab) can be administered once a subject is indicated as being a likely responder to anti-immune checkpoint therapy. In another embodiment, such anti-immune checkpoint therapy can be avoided once a subject is indicated as not being a likely responder to anti-immune checkpoint therapy and an alternative treatment regimen, such as targeted and/or untargeted anti-cancer therapies can be administered. Combination therapies are also contemplated and can comprise, for example, one or more chemotherapeutic agents and radiation, one or more chemotherapeutic agents and immunotherapy, or one or more chemotherapeutic agents, radiation and chemotherapy, each combination of which can be with anti-immune checkpoint therapy.

The term "targeted therapy" refers to administration of agents that selectively interact with a chosen biomolecule to thereby treat cancer. For example, anti-Ang-2 agents, such as therapeutic monoclonal blocking antibodies, which are well-known in the art and described above, can be used to target tumor microenvironments and cells expressing unwanted Ang-2. Similarly, bevacizumab (Avastin^{®}) is a humanized monoclonal antibody that targets vascular endothelial growth factor (see, for example, U.S. Pat. Publ. 2013/0121999, WO 2013/083499, and Presta et al. (1997) Cancer Res. 57:4593-4599).

Immunotherapy is one form of targeted therapy that may comprise, for example, the use of cancer vaccines and/or sensitized antigen presenting cells. For example, an oncolytic virus is a virus that is able to infect and lyse cancer cells, while leaving normal cells unharmed, making them potentially useful in cancer therapy. Replication of oncolytic viruses both facilitates tumor cell destruction and also produces dose amplification at the tumor site. They may also act as vectors for anticancer genes, allowing them to be specifically delivered to the tumor site. The immunotherapy can involve passive immunity for short-term protection of a host, achieved by the administration of pre-formed antibody directed against a cancer antigen or disease antigen (*e.g.,* administration of a monoclonal antibody, optionally linked to a chemotherapeutic agent or toxin, to a tumor antigen). Immunotherapy can also focus on using the cytotoxic lymphocyte-recognized epitopes of cancer cell lines. Alternatively, antisense polynucleotides, ribozymes, RNA interference molecules, triple helix polynucleotides and the like, can be used to selectively modulate biomolecules that are linked to the initiation, progression, and/or pathology of a tumor or cancer.

The term "untargeted therapy" referes to administration of agents that do not selectively interact with a chosen biomolecule yet treat cancer. Representative examples of untargeted therapies include, without limitation, chemotherapy, gene therapy, and radiation therapy.

In one embodiment, chemotherapy is used. Chemotherapy includes the administration of a chemotherapeutic agent. Such a chemotherapeutic agent may be, but is not limited to, those selected from among the following groups of compounds: platinum compounds, cytotoxic antibiotics, antimetabolities, anti-mitotic agents, alkylating agents, arsenic compounds, DNA topoisomerase inhibitors, taxanes, nucleoside analogues, plant alkaloids, and toxins; and synthetic derivatives thereof. Exemplary compounds include, but are not limited to, alkylating agents: cisplatin, treosulfan, and trofosfamide; plant alkaloids: vinblastine, paclitaxel, docetaxol; DNA topoisomerase inhibitors: teniposide, crisnatol, and mitomycin; anti-folates: methotrexate, mycophenolic acid, and hydroxyurea; pyrimidine analogs: 5-fluorouracil, doxifluridine, and cytosine arabinoside; purine analogs: mercaptopurine and thioguanine; DNA antimetabolites: 2'-deoxy-5-fluorouridine, aphidicolin glycinate, and pyrazoloimidazole; and antimitotic agents: halichondrin, colchicine, and rhizoxin. Compositions comprising one or more chemotherapeutic agents (e.g., FLAG, CHOP) may also be used. FLAG comprises fludarabine, cytosine arabinoside (Ara-C) and G-CSF. CHOP comprises cyclophosphamide, vincristine, doxorubicin, and prednisone. In another embodiments, PARP (*e.g.,* PARP-1 and/or PARP-2) inhibitors are used and such inhibitors are well known in the art (*e.g.*, Olaparib, ABT-888, BSI-201, BGP-15 (N-Gene Research Laboratories, Inc.); INO-1001 (Inotek Pharmaceuticals Inc.); PJ34 (Soriano *et al.,* 2001; Pacher *et al.,* 2002b); 3-aminobenzamide (Trevigen); 4-amino-1,8-naphthalimide; (Trevigen); 6(5H)-phenanthridinone (Trevigen); benzamide (U.S. Pat. Re. 36,397); and NU1025 (Bowman et al.). The mechanism of action is generally related to the ability of PARP inhibitors to bind PARP and decrease its activity. PARP catalyzes the conversion of .beta.-nicotinamide adenine dinucleotide (NAD+) into nicotinamide and poly-ADP-ribose (PAR). Both poly (ADP-ribose) and PARP have been linked to regulation of transcription, cell proliferation, genomic stability, and carcinogenesis (Bouchard V. J. et.al. Experimental Hematology, Volume 31, Number 6, June 2003, pp. 446-454(9); Herceg Z.; Wang Z.-Q. Mutation Research/Fundamental and Molecular Mechanisms of Mutagenesis, Volume 477, Number 1, 2 Jun. 2001, pp. 97-110(14)). Poly(ADP-ribose) polymerase 1 (PARP1) is a key molecule in the repair of DNA single-strand breaks (SSBs) (de Murcia J. et al. 1997. Proc Natl Acad Sci USA 94:7303-7307; Schreiber V, Dantzer F, Ame J C, de Murcia G (2006) Nat Rev Mol Cell Biol 7:517-528; Wang Z Q, et al. (1997) Genes Dev 11:2347-2358). Knockout of SSB repair by inhibition of PARP1 function induces DNA double-strand breaks (DSBs) that can trigger synthetic lethality in cancer cells with defective homology-directed DSB repair (Bryant H E, et al. (2005) Nature 434:913-917; Farmer H, et al. (2005) Nature 434:917-921). The foregoing examples of chemotherapeutic agents are illustrative, and are not intended to be limiting.

In another embodiment, radiation therapy is used. The radiation used in radiation therapy can be ionizing radiation. Radiation therapy can also be gamma rays, X-rays, or proton beams. Examples of radiation therapy include, but are not limited to, external-beam radiation therapy, interstitial implantation of radioisotopes (1-125, palladium, iridium), radioisotopes such as strontium-89, thoracic radiation therapy, intraperitoneal P-32 radiation therapy, and/or total abdominal and pelvic radiation therapy. For a general overview of radiation therapy, see Hellman, Chapter 16: Principles of Cancer Management: Radiation Therapy, 6th edition, 2001, DeVita et al., eds., J. B. Lippencott Company, Philadelphia. The radiation therapy can be administered as external beam radiation or teletherapy wherein the radiation is directed from a remote source. The radiation treatment can also be administered as internal therapy or brachytherapy wherein a radioactive source is placed inside the body close to cancer cells or a tumor mass. Also encompassed is the use of photodynamic therapy comprising the administration of photosensitizers, such as hematoporphyrin and its derivatives, Vertoporfin (BPD-MA), phthalocyanine, photosensitizer Pc4, demethoxy-hypocrellin A; and 2BA-2-DMHA.

In another embodiment, hormone therapy is used. Hormonal therapeutic treatments can comprise, for example, hormonal agonists, hormonal antagonists (e.g., flutamide, bicalutamide, tamoxifen, raloxifene, leuprolide acetate (LUPRON), LH-RH antagonists), inhibitors of hormone biosynthesis and processing, and steroids (*e.g.,* dexamethasone, retinoids, deltoids, betamethasone, cortisol, cortisone, prednisone, dehydrotestosterone, glucocorticoids, mineralocorticoids, estrogen, testosterone, progestins), vitamin A derivatives (*e*.*g*., all-trans retinoic acid (ATRA)); vitamin D3 analogs; antigestagens (*e*.*g*., mifepristone, onapristone), or antiandrogens (*e.g.,* cyproterone acetate).

In another embodiment, hyperthermia, a procedure in which body tissue is exposed to high temperatures (up to 106°F.) is used. Heat may help shrink tumors by damaging cells or depriving them of substances they need to live. Hyperthermia therapy can be local, regional, and whole-body hyperthermia, using external and internal heating devices. Hyperthermia is almost always used with other forms of therapy (*e.g.,* radiation therapy, chemotherapy, and biological therapy) to try to increase their effectiveness. Local hyperthermia refers to heat that is applied to a very small area, such as a tumor. The area may be heated externally with high-frequency waves aimed at a tumor from a device outside the body. To achieve internal heating, one of several types of sterile probes may be used, including thin, heated wires or hollow tubes filled with warm water; implanted microwave antennae; and radiofrequency electrodes. In regional hyperthermia, an organ or a limb is heated. Magnets and devices that produce high energy are placed over the region to be heated. In another approach, called perfusion, some of the patient's blood is removed, heated, and then pumped (perfused) into the region that is to be heated internally. Whole-body heating is used to treat metastatic cancer that has spread throughout the body. It can be accomplished using warm-water blankets, hot wax, inductive coils (like those in electric blankets), or thermal chambers (similar to large incubators). Hyperthermia does not cause any marked increase in radiation side effects or complications. Heat applied directly to the skin, however, can cause discomfort or even significant local pain in about half the patients treated. It can also cause blisters, which generally heal rapidly.

In still another embodiment, photodynamic therapy (also called PDT, photoradiation therapy, phototherapy, or photochemotherapy) is used for the treatment of some types of cancer. It is based on the discovery that certain chemicals known as photosensitizing agents can kill one-celled organisms when the organisms are exposed to a particular type of light. PDT destroys cancer cells through the use of a fixed-frequency laser light in combination with a photosensitizing agent. In PDT, the photosensitizing agent is injected into the bloodstream and absorbed by cells all over the body. The agent remains in cancer cells for a longer time than it does in normal cells. When the treated cancer cells are exposed to laser light, the photosensitizing agent absorbs the light and produces an active form of oxygen that destroys the treated cancer cells. Light exposure must be timed carefully so that it occurs when most of the photosensitizing agent has left healthy cells but is still present in the cancer cells. The laser light used in PDT can be directed through a fiber-optic (a very thin glass strand). The fiber-optic is placed close to the cancer to deliver the proper amount of light. The fiber-optic can be directed through a bronchoscope into the lungs for the treatment of lung cancer or through an endoscope into the esophagus for the treatment of esophageal cancer. An advantage of PDT is that it causes minimal damage to healthy tissue. However, because the laser light currently in use cannot pass through more than about 3 centimeters of tissue (a little more than one and an eighth inch), PDT is mainly used to treat tumors on or just under the skin or on the lining of internal organs. Photodynamic therapy makes the skin and eyes sensitive to light for 6 weeks or more after treatment. Patients are advised to avoid direct sunlight and bright indoor light for at least 6 weeks. If patients must go outdoors, they need to wear protective clothing, including sunglasses. Other temporary side effects of PDT are related to the treatment of specific areas and can include coughing, trouble swallowing, abdominal pain, and painful breathing or shortness of breath. In December 1995, the U.S. Food and Drug Administration (FDA) approved a photosensitizing agent called porfimer sodium, or Photofrin^{®}, to relieve symptoms of esophageal cancer that is causing an obstruction and for esophageal cancer that cannot be satisfactorily treated with lasers alone. In January 1998, the FDA approved porfimer sodium for the treatment of early nonsmall cell lung cancer in patients for whom the usual treatments for lung cancer are not appropriate. The National Cancer Institute and other institutions are supporting clinical trials (research studies) to evaluate the use of photodynamic therapy for several types of cancer, including cancers of the bladder, brain, larynx, and oral cavity.

In yet another embodiment, laser therapy is used to harness high-intensity light to destroy cancer cells. This technique is often used to relieve symptoms of cancer such as bleeding or obstruction, especially when the cancer cannot be cured by other treatments. It may also be used to treat cancer by shrinking or destroying tumors. The term "laser" stands for light amplification by stimulated emission of radiation. Ordinary light, such as that from a light bulb, has many wavelengths and spreads in all directions. Laser light, on the other hand, has a specific wavelength and is focused in a narrow beam. This type of high-intensity light contains a lot of energy. Lasers are very powerful and may be used to cut through steel or to shape diamonds. Lasers also can be used for very precise surgical work, such as repairing a damaged retina in the eye or cutting through tissue (in place of a scalpel). Although there are several different kinds of lasers, only three kinds have gained wide use in medicine: Carbon dioxide (CO₂) laser--This type of laser can remove thin layers from the skin's surface without penetrating the deeper layers. This technique is particularly useful in treating tumors that have not spread deep into the skin and certain precancerous conditions. As an alternative to traditional scalpel surgery, the CO₂ laser is also able to cut the skin. The laser is used in this way to remove skin cancers. Neodymium:yttrium-aluminum-garnet (Nd:YAG) laser-- Light from this laser can penetrate deeper into tissue than light from the other types of lasers, and it can cause blood to clot quickly. It can be carried through optical fibers to less accessible parts of the body. This type of laser is sometimes used to treat throat cancers. Argon laser--This laser can pass through only superficial layers of tissue and is therefore useful in dermatology and in eye surgery. It also is used with light-sensitive dyes to treat tumors in a procedure known as photodynamic therapy (PDT). Lasers have several advantages over standard surgical tools, including: Lasers are more precise than scalpels. Tissue near an incision is protected, since there is little contact with surrounding skin or other tissue. The heat produced by lasers sterilizes the surgery site, thus reducing the risk of infection. Less operating time may be needed because the precision of the laser allows for a smaller incision. Healing time is often shortened; since laser heat seals blood vessels, there is less bleeding, swelling, or scarring. Laser surgery may be less complicated. For example, with fiber optics, laser light can be directed to parts of the body without making a large incision. More procedures may be done on an outpatient basis. Lasers can be used in two ways to treat cancer: by shrinking or destroying a tumor with heat, or by activating a chemical--known as a photosensitizing agent--that destroys cancer cells. In PDT, a photosensitizing agent is retained in cancer cells and can be stimulated by light to cause a reaction that kills cancer cells. CO₂ and Nd:YAG lasers are used to shrink or destroy tumors. They may be used with endoscopes, tubes that allow physicians to see into certain areas of the body, such as the bladder. The light from some lasers can be transmitted through a flexible endoscope fitted with fiber optics. This allows physicians to see and work in parts of the body that could not otherwise be reached except by surgery and therefore allows very precise aiming of the laser beam. Lasers also may be used with low-power microscopes, giving the doctor a clear view of the site being treated. Used with other instruments, laser systems can produce a cutting area as small as 200 microns in diameter--less than the width of a very fine thread. Lasers are used to treat many types of cancer. Laser surgery is a standard treatment for certain stages of glottis (vocal cord), cervical, skin, lung, vaginal, vulvar, and penile cancers. In addition to its use to destroy the cancer, laser surgery is also used to help relieve symptoms caused by cancer (palliative care). For example, lasers may be used to shrink or destroy a tumor that is blocking a patient's trachea (windpipe), making it easier to breathe. It is also sometimes used for palliation in colorectal and anal cancer. Laser-induced interstitial thermotherapy (LITT) is one of the most recent developments in laser therapy. LITT uses the same idea as a cancer treatment called hyperthermia; that heat may help shrink tumors by damaging cells or depriving them of substances they need to live. In this treatment, lasers are directed to interstitial areas (areas between organs) in the body. The laser light then raises the temperature of the tumor, which damages or destroys cancer cells.

The duration and/or dose of treatment with anti-immune checkpoint therapies may vary according to the particular anti-immune checkpoint agent or combination thereof. An appropriate treatment time for a particular cancer therapeutic agent will be appreciated by the skilled artisan. The present invention contemplates the continued assessment of optimal treatment schedules for each cancer therapeutic agent, where the phenotype of the cancer of the subject as determined by the methods of the present invention is a factor in determining optimal treatment doses and schedules.

Any means for the introduction of a polynucleotide into mammals, human or non-human, or cells thereof may be adapted to the practice of this invention for the delivery of the various constructs of the present invention into the intended recipient. In one embodiment of the present invention, the DNA constructs are delivered to cells by transfection, *i*.*e*., by delivery of "naked" DNA or in a complex with a colloidal dispersion system. A colloidal system includes macromolecule complexes, nanocapsules, microspheres, beads, and lipid-based systems including oil-in-water emulsions, micelles, mixed micelles, and liposomes. The preferred colloidal system of this invention is a lipid-complexed or liposome-formulated DNA. In the former approach, prior to formulation of DNA, e.g., with lipid, a plasmid containing a transgene bearing the desired DNA constructs may first be experimentally optimized for expression (*e.g.*, inclusion of an intron in the 5' untranslated region and elimination of unnecessary sequences (Felgner, et al., Ann NY Acad Sci 126-139, 1995). Formulation of DNA, *e.g.* with various lipid or liposome materials, may then be effected using known methods and materials and delivered to the recipient mammal. See, *e.g.,* Canonico et al, Am J Respir Cell Mol Biol 10:24-29, 1994; Tsan et al, Am J Physiol 268; Alton et al., Nat Genet. 5: 135-142, 1993 and U.S. patent No. 5,679,647 by Carson et al.

The targeting of liposomes can be classified based on anatomical and mechanistic factors. Anatomical classification is based on the level of selectivity, for example, organ-specific, cell-specific, and organelle-specific. Mechanistic targeting can be distinguished based upon whether it is passive or active. Passive targeting utilizes the natural tendency of liposomes to distribute to cells of the reticulo-endothelial system (RES) in organs, which contain sinusoidal capillaries. Active targeting, on the other hand, involves alteration of the liposome by coupling the liposome to a specific ligand such as a monoclonal antibody, sugar, glycolipid, or protein, or by changing the composition or size of the liposome in order to achieve targeting to organs and cell types other than the naturally occurring sites of localization.

The surface of the targeted delivery system may be modified in a variety of ways. In the case of a liposomal targeted delivery system, lipid groups can be incorporated into the lipid bilayer of the liposome in order to maintain the targeting ligand in stable association with the liposomal bilayer. Various linking groups can be used for joining the lipid chains to the targeting ligand. Naked DNA or DNA associated with a delivery vehicle, *e.g.,* liposomes, can be administered to several sites in a subject (see below).

Nucleic acids can be delivered in any desired vector. These include viral or non-viral vectors, including adenovirus vectors, adeno-associated virus vectors, retrovirus vectors, lentivirus vectors, and plasmid vectors. Exemplary types of viruses include HSV (herpes simplex virus), AAV (adeno associated virus), HIV (human immunodeficiency virus), BIV (bovine immunodeficiency virus), and MLV (murine leukemia virus). Nucleic acids can be administered in any desired format that provides sufficiently efficient delivery levels, including in virus particles, in liposomes, in nanoparticles, and complexed to polymers.

The nucleic acids encoding a protein or nucleic acid of interest may be in a plasmid or viral vector, or other vector as is known in the art. Such vectors are well known and any can be selected for a particular application. In one embodiment of the present invention, the gene delivery vehicle comprises a promoter and a demethylase coding sequence. Preferred promoters are tissue-specific promoters and promoters which are activated by cellular proliferation, such as the thymidine kinase and thymidylate synthase promoters. Other preferred promoters include promoters which are activatable by infection with a virus, such as the α- and β-interferon promoters, and promoters which are activatable by a hormone, such as estrogen. Other promoters which can be used include the Moloney virus LTR, the CMV promoter, and the mouse albumin promoter. A promoter may be constitutive or inducible.

In another embodiment, naked polynucleotide molecules are used as gene delivery vehicles, as described in WO 90/11092 and U.S. Patent 5,580,859. Such gene delivery vehicles can be either growth factor DNA or RNA and, in certain embodiments, are linked to killed adenovirus. Curiel et al., Hum. Gene. Ther. 3:147-154, 1992. Other vehicles which can optionally be used include DNA-ligand (Wu et al., J. Biol. Chem. 264:16985-16987, 1989), lipid-DNA combinations (Felgner et al., Proc. Natl. Acad. Sci. USA 84:7413 7417, 1989), liposomes (Wang et al., Proc. Natl. Acad. Sci. 84:7851-7855, 1987) and microprojectiles (Williams et al., Proc. Natl. Acad. Sci. 88:2726-2730, 1991).

A gene delivery vehicle can optionally comprise viral sequences such as a viral origin of replication or packaging signal. These viral sequences can be selected from viruses such as astrovirus, coronavirus, orthomyxovirus, papovavirus, paramyxovirus, parvovirus, picornavirus, poxvirus, retrovirus, togavirus or adenovirus. In a preferred embodiment, the growth factor gene delivery vehicle is a recombinant retroviral vector. Recombinant retroviruses and various uses thereof have been described in numerous references including, for example, Mann et al., Cell 33:153, 1983, Cane and Mulligan, Proc. Nat'l. Acad. USA 81:6349, 1984, Miller et al., Human Gene Therapy 1:5-14, 1990, U.S. Patent Nos. 4,405,712, 4,861,719, and 4,980,289, and PCT Application Nos. WO 89/02,468, WO 89/05,349, and WO 90/02,806. Numerous retroviral gene delivery vehicles can be utilized in the present invention, including for example those described in EP 0,415,731; WO 90/07936; WO 94/03622; WO 93/25698; WO 93/25234; U.S. Patent No. 5,219,740; WO 9311230; WO 9310218; Vile and Hart, Cancer Res. 53:3860-3864, 1993; Vile and Hart, Cancer Res. 53:962-967, 1993; Ram et al., Cancer Res. 53:83-88, 1993; Takamiya et al., J. Neurosci. Res. 33:493-503, 1992; Baba et al., J. Neurosurg. 79:729-735, 1993 (U.S. Patent No. 4,777,127, GB 2,200,651, EP 0,345,242 and WO91/02805).

Other viral vector systems that can be used to deliver a polynucleotide of the present invention have been derived from herpes virus, e.g., Herpes Simplex Virus (U.S. Patent No. 5,631,236 by Woo et al., issued May 20, 1997 and WO 00/08191 by Neurovex), vaccinia virus (Ridgeway (1988) Ridgeway, "Mammalian expression vectors," In: Rodriguez R L, Denhardt D T, ed. Vectors: A survey of molecular cloning vectors and their uses. Stoneham: Butterworth,; Baichwal and Sugden (1986) "Vectors for gene transfer derived from animal DNA viruses: Transient and stable expression of transferred genes," In: Kucherlapati R, ed. Gene transfer. New York: Plenum Press; Coupar et al. (1988) Gene, 68:1-10), and several RNA viruses. Preferred viruses include an alphavirus, a poxivirus, an arena virus, a vaccinia virus, a polio virus, and the like. They offer several attractive features for various mammalian cells (Friedmann (1989) Science, 244:1275-1281; Ridgeway, 1988, supra; Baichwal and Sugden, 1986, supra; Coupar et al., 1988; Horwich et al.(1990) J.Virol., 64:642-650).

In other embodiments, target DNA in the genome can be manipulated using well-known methods in the art. For example, the target DNA in the genome can be manipulated by deletion, insertion, and/or mutation are retroviral insertion, artificial chromosome techniques, gene insertion, random insertion with tissue specific promoters, gene targeting, transposable elements and/or any other method for introducing foreign DNA or producing modified DNA/modified nuclear DNA. Other modification techniques include deleting DNA sequences from a genome and/or altering nuclear DNA sequences. Nuclear DNA sequences, for example, may be altered by site-directed mutagenesis.

In other embodiments, recombinant biomarker polypeptides, and fragments thereof, can be administered to subjects. In some embodiments, fusion proteins can be constructed and administered which have enhanced biological properties. In addition, the biomarker polypeptides, and fragment thereof, can be modified according to well-known pharmacological methods in the art (*e*.*g*., pegylation, glycosylation, oligomerization, *etc.*) in order to further enhance desirable biological activities, such as increased bioavailability and decreased proteolytic degradation.

### 4. Clincal Efficacy

Clinical efficacy can be measured by any method known in the art. For example, the response to a therapy, such as anti-immune checkpoint therapies, relates to any response of the cancer, *e.g.,* a tumor, to the therapy, preferably to a change in tumor mass and/or volume after initiation of neoadjuvant or adjuvant chemotherapy. Tumor response may be assessed in a neoadjuvant or adjuvant situation where the size of a tumor after systemic intervention can be compared to the initial size and dimensions as measured by CT, PET, mammogram, ultrasound or palpation and the cellularity of a tumor can be estimated histologically and compared to the cellularity of a tumor biopsy taken before initiation of treatment. Response may also be assessed by caliper measurement or pathological examination of the tumor after biopsy or surgical resection. Response may be recorded in a quantitative fashion like percentage change in tumor volume or cellularity or using a semi-quantitative scoring system such as residual cancer burden (Symmans et al., J. Clin. Oncol. (2007) 25:4414-4422) or Miller-Payne score (Ogston et al., (2003) Breast (Edinburgh, Scotland) 12:320-327) in a qualitative fashion like "pathological complete response" (pCR), "clinical complete remission" (cCR), "clinical partial remission" (cPR), "clinical stable disease" (cSD), "clinical progressive disease" (cPD) or other qualitative criteria. Assessment of tumor response may be performed early after the onset of neoadjuvant or adjuvant therapy, e.g., after a few hours, days, weeks or preferably after a few months. A typical endpoint for response assessment is upon termination of neoadjuvant chemotherapy or upon surgical removal of residual tumor cells and/or the tumor bed.

In some embodiments, clinical efficacy of the therapeutic treatments described herein may be determined by measuring the clinical benefit rate (CBR). The clinical benefit rate is measured by determining the sum of the percentage of patients who are in complete remission (CR), the number of patients who are in partial remission (PR) and the number of patients having stable disease (SD) at a time point at least 6 months out from the end of therapy. The shorthand for this formula is CBR=CR+PR+SD over 6 months. In some embodiments, the CBR for a particular anti-immune checkpoint therapeutic regimen is at least 25%, 30%, 35%, 40%, 45%, 50%, 55%, 60%, 65%, 70%, 75%, 80%, 85%, or more.

Additional criteria for evaluating the response to anti-immune checkpoint therapies are related to "survival," which includes all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point (e.g., time of diagnosis or start of treatment) and end point (*e.g.,* death, recurrence or metastasis). In addition, criteria for efficacy of treatment can be expanded to include response to chemotherapy, probability of survival, probability of metastasis within a given time period, and probability of tumor recurrence.

For example, in order to determine appropriate threshold values, a particular anti-immune checkpoint therapeutic regimen can be administered to a population of subjects and the outcome can be correlated to biomarker measurements that were determined prior to administration of any anti-immune checkpoint therapy. The outcome measurement may be pathologic response to therapy given in the neoadjuvant setting. Alternatively, outcome measures, such as overall survival and disease-free survival can be monitored over a period of time for subjects following anti-immune checkpoint therapy for whom biomarker measurement values are known. In certain embodiments, the same doses of anti-immune checkpoint agents are administered to each subject. In related embodiments, the doses administered are standard doses known in the art for anti-immune checkpoint agents. The period of time for which subjects are monitored can vary. For example, subjects may be monitored for at least 2, 4, 6, 8, 10, 12, 14, 16, 18, 20, 25, 30, 35, 40, 45, 50, 55, or 60 months. Biomarker measurement threshold values that correlate to outcome of an anti-immune checkpoint therapy can be determined using methods such as those described in the Examples section.

### 5. Further Uses and Methods of the Present invention

The compositions described herein can be used in a variety of diagnostic, prognostic, and therapeutic applications. In any method described herein, such as a diagnostic method, prognostic method, therapeutic method, or combination thereof, all steps of the method can be performed by a single actor or, alternatively, by more than one actor. For example, diagnosis can be performed directly by the actor providing therapeutic treatment. Alternatively, a person providing a therapeutic agent can request that a diagnostic assay be performed. The diagnostician and/or the therapeutic interventionist can interpret the diagnostic assay results to determine a therapeutic strategy. Similarly, such alternative processes can apply to other assays, such as prognostic assays.

### a. Screening Methods

One aspect of the present invention relates to screening assays, including non-cell based assays. In one embodiment, the assays provide a method for identifying whether a cancer is likely to respond to anti-immune checkpoint therapy and/or whether an agent can inhibit the growth of or kill a cancer cell that is unlikely to respond to anti-immune checkpoint therapy.

In one embodiment, the present invention relates to assays for screening test agents which bind to, or modulate the biological activity of, at least one biomarker listed in Table 1. In one embodiment, a method for identifying such an agent entails determining the ability of the agent to modulate, e.g. inhibit, the at least one biomarker listed in Table 1.

In one embodiment, an assay is a cell-free or cell-based assay, comprising contacting at least one biomarker listed in Table 1, with a test agent, and determining the ability of the test agent to modulate (*e.g.* inhibit) the enzymatic activity of the biomarker, such as by measuring direct binding of substrates or by measuring indirect parameters as described below.

For example, in a direct binding assay, biomarker protein (or their respective target polypeptides or molecules) can be coupled with a radioisotope or enzymatic label such that binding can be determined by detecting the labeled protein or molecule in a complex. For example, the targets can be labeled with ¹²⁵I, ³⁵S, ¹⁴C, or ³H, either directly or indirectly, and the radioisotope detected by direct counting of radioemmission or by scintillation counting. Alternatively, the targets can be enzymatically labeled with, for example, horseradish peroxidase, alkaline phosphatase, or luciferase, and the enzymatic label detected by determination of conversion of an appropriate substrate to product. Determining the interaction between biomarker and substrate can also be accomplished using standard binding or enzymatic analysis assays. In one or more embodiments of the above described assay methods, it may be desirable to immobilize polypeptides or molecules to facilitate separation of complexed from uncomplexed forms of one or both of the proteins or molecules, as well as to accommodate automation of the assay.

Binding of a test agent to a target can be accomplished in any vessel suitable for containing the reactants. Non-limiting examples of such vessels include microtiter plates, test tubes, and micro-centrifuge tubes. Immobilized forms of the antibodies of the present invention can also include antibodies bound to a solid phase like a porous, microporous (with an average pore diameter less than about one micron) or macroporous (with an average pore diameter of more than about 10 microns) material, such as a membrane, cellulose, nitrocellulose, or glass fibers; a bead, such as that made of agarose or polyacrylamide or latex; or a surface of a dish, plate, or well, such as one made of polystyrene.

In an alternative embodiment, determining the ability of the agent to modulate the interaction between the biomarker and a substrate or a biomarker and its natural binding partner can be accomplished by determining the ability of the test agent to modulate the activity of a polypeptide or other product that functions downstream or upstream of its position within the signaling pathway (e.g., feedback loops). Such feedback loops are well-known in the art (see, for example, Chen and Guillemin (2009) Int. J. Tryptophan Res. 2:1-19).

The present invention further pertains to novel agents identified by the above-described screening assays. Accordingly, it is within the scope of this invention to further use an agent identified as described herein in an appropriate animal model. For example, an agent identified as described herein can be used in an animal model to determine the efficacy, toxicity, or side effects of treatment with such an agent. Alternatively, an antibody identified as described herein can be used in an animal model to determine the mechanism of action of such an agent.

### b. Predictive Medicine

The present invention also pertains to the field of predictive medicine in which diagnostic assays, prognostic assays, and monitoring clinical trials are used for prognostic (predictive) purposes to thereby treat an individual prophylactically. Accordingly, one aspect of the present invention relates to diagnostic assays for determining the amount and/or activity level of a biomarker listed in Table 1 in the context of a biological sample (*e.g.,* blood, serum, cells, or tissue) to thereby determine whether an individual afflicted with a cancer is likely to respond to anti-immune checkpoint therapy, whether in an original or recurrent cancer. Such assays can be used for prognostic or predictive purpose to thereby prophylactically treat an individual prior to the onset or after recurrence of a disorder characterized by or associated with biomarker polypeptide, nucleic acid expression or activity. The skilled artisan will appreciate that any method can use one or more (e.g., combinations) of biomarkers listed in Table 1.

Another aspect of the present invention pertains to monitoring the influence of agents (*e.g.,* drugs, compounds, and small nucleic acid-based molecules) on the expression or activity of a biomarker listed in Table 1. These and other agents are described in further detail in the following sections.

The skilled artisan will also appreciated that, in certain embodiments, the methods of the present invention implement a computer program and computer system. For example, a computer program can be used to perform the algorithms described herein. A computer system can also store and manipulate data generated by the methods of the present invention which comprises a plurality of biomarker signal changes/profiles which can be used by a computer system in implementing the methods of this invention. In certain embodiments, a computer system receives biomarker expression data; (ii) stores the data; and (iii) compares the data in any number of ways described herein (e.g., analysis relative to appropriate controls) to determine the state of informative biomarkers from cancerous or precancerous tissue. In other embodiments, a computer system (i) compares the determined expression biomarker level to a threshold value; and (ii) outputs an indication of whether said biomarker level is significantly modulated (e.g., above or below) the threshold value, or a phenotype based on said indication.

In certain embodiments, such computer systems are also considered part of the present invention. Numerous types of computer systems can be used to implement the analytic methods of this invention according to knowledge possessed by a skilled artisan in the bioinformatics and/or computer arts. Several software components can be loaded into memory during operation of such a computer system. The software components can comprise both software components that are standard in the art and components that are special to the present invention (*e.g.,* dCHIP software described in Lin et al. (2004) Bioinformatics 20, 1233-1240; radial basis machine learning algorithms (RBM) known in the art).

The methods of the present invention can also be programmed or modeled in mathematical software packages that allow symbolic entry of equations and high-level specification of processing, including specific algorithms to be used, thereby freeing a user of the need to procedurally program individual equations and algorithms. Such packages include, *e.g.,* Matlab from Mathworks (Natick, Mass.), Mathematica from Wolfram Research (Champaign, III.) or S-Plus from MathSoft (Seattle, Wash.).

In certain embodiments, the computer comprises a database for storage of biomarker data. Such stored profiles can be accessed and used to perform comparisons of interest at a later point in time. For example, biomarker expression profiles of a sample derived from the non-cancerous tissue of a subject and/or profiles generated from population-based distributions of informative loci of interest in relevant populations of the same species can be stored and later compared to that of a sample derived from the cancerous tissue of the subject or tissue suspected of being cancerous of the subject.

In addition to the exemplary program structures and computer systems described herein, other, alternative program structures and computer systems will be readily apparent to the skilled artisan. Such alternative systems, which do not depart from the above described computer system and programs structures either in spirit or in scope, are therefore intended to be comprehended within the accompanying claims.

### c. Diagnostic Assays

The present invention provides, in part, methods, systems, and code for accurately classifying whether a biological sample is associated with a cancer that is likely to respond to anti-immune checkpoint therapy. In some embodiments, the present invention is useful for classifying a sample (*e.g.,* from a subject) as associated with or at risk for responding to or not responding to anti-immune checkpoint therapy using a statistical algorithm and/or empirical data (*e.g.,* the amount or activity of a biomarker listed in Table 1).

An exemplary method for detecting the amount or activity of a biomarker listed in Table 1, and thus useful for classifying whether a sample is likely or unlikely to respond to anti-immune checkpoint therapy involves obtaining a biological sample from a test subject and contacting the biological sample with an agent, such as a protein-binding agent like an antibody or antigen-binding fragment thereof, or a nucleic acid-binding agent like an oligonucleotide, capable of detecting the amount or activity of the biomarker in the biological sample. In some embodiments, at least one antibody or antigen-binding fragment thereof is used, wherein two, three, four, five, six, seven, eight, nine, ten, or more such antibodies or antibody fragments can be used in combination (*e.g.,* in sandwich ELISAs) or in serial. In certain instances, the statistical algorithm is a single learning statistical classifier system. For example, a single learning statistical classifier system can be used to classify a sample as a based upon a prediction or probability value and the presence or level of the biomarker. The use of a single learning statistical classifier system typically classifies the sample as, for example, a likely anti-immune checkpoint therapy responder or progressor sample with a sensitivity, specificity, positive predictive value, negative predictive value, and/or overall accuracy of at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

Other suitable statistical algorithms are well known to those of skill in the art. For example, learning statistical classifier systems include a machine learning algorithmic technique capable of adapting to complex data sets (e.g., panel of markers of interest) and making decisions based upon such data sets. In some embodiments, a single learning statistical classifier system such as a classification tree (e.g., random forest) is used. In other embodiments, a combination of 2, 3, 4, 5, 6, 7, 8, 9, 10, or more learning statistical classifier systems are used, preferably in tandem. Examples of learning statistical classifier systems include, but are not limited to, those using inductive learning (*e.g.,* decision/classification trees such as random forests, classification and regression trees (C&RT), boosted trees, etc.), Probably Approximately Correct (PAC) learning, connectionist learning (*e.g.,* neural networks (NN), artificial neural networks (ANN), neuro fuzzy networks (NFN), network structures, perceptrons such as multi-layer perceptrons, multi-layer feed-forward networks, applications of neural networks, Bayesian learning in belief networks, etc.), reinforcement learning (*e.g.,* passive learning in a known environment such as naive learning, adaptive dynamic learning, and temporal difference learning, passive learning in an unknown environment, active learning in an unknown environment, learning action-value functions, applications of reinforcement learning, etc.), and genetic algorithms and evolutionary programming. Other learning statistical classifier systems include support vector machines (*e.g.,* Kernel methods), multivariate adaptive regression splines (MARS), Levenberg-Marquardt algorithms, Gauss-Newton algorithms, mixtures of Gaussians, gradient descent algorithms, and learning vector quantization (LVQ). In certain embodiments, the method of the present invention further comprises sending the sample classification results to a clinician, *e.g.,* an oncologist.

In another embodiment, the diagnosis of a subject is followed by administering to the individual a therapeutically effective amount of a defined treatment based upon the diagnosis.

In one embodiment, the methods further involve obtaining a control biological sample (*e.g.,* biological sample from a subject who does not have a cancer or whose cancer is susceptible to anti-immune checkpoint therapy), a biological sample from the subject during remission, or a biological sample from the subject during treatment for developing a cancer progressing despite anti-immune checkpoint therapy.

### d. Prognostic Assays

The diagnostic methods described herein can furthermore be utilized to identify subjects having or at risk of developing a cancer that is likely or unlikely to be responsive to anti-immune checkpoint therapy. The assays described herein, such as the preceding diagnostic assays or the following assays, can be utilized to identify a subject having or at risk of developing a disorder associated with a misregulation of the amount or activity of at least one biomarker described in Table 1, such as in cancer. Alternatively, the prognostic assays can be utilized to identify a subject having or at risk for developing a disorder associated with a misregulation of the at least one biomarker described in Table 1, such as in cancer. Furthermore, the prognostic assays described herein can be used to determine whether a subject can be administered an agent (*e.g.,* an agonist, antagonist, peptidomimetic, polypeptide, peptide, nucleic acid, small molecule, or other drug candidate) to treat a disease or disorder associated with the aberrant biomarker expression or activity.

### e. Treatment Methods

The compositions described herein (including dual binding antibodies and derivatives and conjugates thereof) can be used in a variety of *in vitro* and *in vivo* therapeutic applications using the formulations and/or combinations described herein. In one embodiment, anti-immune checkpoint agents can be used to treat cancers determined to be responsive thereto. For example, antibodies that block the interaction between PD-L1, PD-L2, and/or CTLA-4 and their receptors (*e.g.,* PD-L1 binding to PD-1, PD-L2 binding to PD-1, and the like) can be used to treat cancer in subjects identified as likely responding thereto.

### 6. Pharmaceutical Compositions

In another aspect, the present invention provides pharmaceutically acceptable compositions which comprise a therapeutically-effective amount of an agent that modulates (*e.g.,* decreases) biomarker expression and/or activity, formulated together with one or more pharmaceutically acceptable carriers (additives) and/or diluents. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets, boluses, powders, granules, pastes; (2) parenteral administration, for example, by subcutaneous, intramuscular or intravenous injection as, for example, a sterile solution or suspension; (3) topical application, for example, as a cream, ointment or spray applied to the skin; (4) intravaginally or intrarectally, for example, as a pessary, cream or foam; or (5) aerosol, for example, as an aqueous aerosol, liposomal preparation or solid particles containing the compound.

The phrase "therapeutically-effective amount" as used herein means that amount of an agent that modulates (*e.g.,* inhibits) biomarker expression and/or activity, or expression and/or activity of the complex, or composition comprising an agent that modulates (*e.g.,* inhibits) biomarker expression and/or activity, or expression and/or activity of the complex, which is effective for producing some desired therapeutic effect, *e.g.,* cancer treatment, at a reasonable benefit/risk ratio.

The phrase "pharmaceutically acceptable" is employed herein to refer to those agents, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, solvent or encapsulating material, involved in carrying or transporting the subject chemical from one organ, or portion of the body, to another organ, or portion of the body. Each carrier must be "acceptable" in the sense of being compatible with the other ingredients of the formulation and not injurious to the subject. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The term "pharmaceutically-acceptable salts" refers to the relatively non-toxic, inorganic and organic acid addition salts of the agents that modulates (e.g., inhibits) biomarker expression and/or activity, or expression and/or activity of the complex encompassed by the present invention. These salts can be prepared in situ during the final isolation and purification of the respiration uncoupling agents, or by separately reacting a purified respiration uncoupling agent in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like (See, for example, Berge et al. (1977) "Pharmaceutical Salts", J. Pharm. Sci. 66: 1-19).

In other cases, the agents useful in the methods of the present invention may contain one or more acidic functional groups and, thus, are capable of forming pharmaceutically-acceptable salts with pharmaceutically-acceptable bases. The term "pharmaceutically-acceptable salts" in these instances refers to the relatively non-toxic, inorganic and organic base addition salts of agents that modulates (e.g., inhibits) biomarker expression and/or activity, or expression and/or activity of the complex. These salts can likewise be prepared in situ during the final isolation and purification of the respiration uncoupling agents, or by separately reacting the purified respiration uncoupling agent in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like (see, for example, Berge *et al.*, *supra*)*.*

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations useful in the methods of the present invention include those suitable for oral, nasal, topical (including buccal and sublingual), rectal, vaginal, aerosol and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient, which can be combined with a carrier material to produce a single dosage form will generally be that amount of the compound which produces a therapeutic effect. Generally, out of one hundred per cent, this amount will range from about 1 per cent to about ninety-nine percent of active ingredient, preferably from about 5 per cent to about 70 per cent, most preferably from about 10 per cent to about 30 per cent.

Methods of preparing these formulations or compositions include the step of bringing into association an agent that modulates (e.g., inhibits) biomarker expression and/or activity, with the carrier and, optionally, one or more accessory ingredients. In general, the formulations are prepared by uniformly and intimately bringing into association a respiration uncoupling agent with liquid carriers, or finely divided solid carriers, or both, and then, if necessary, shaping the product.

Formulations suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a respiration uncoupling agent as an active ingredient. A compound may also be administered as a bolus, electuary or paste.

In solid dosage forms for oral administration (capsules, tablets, pills, dragees, powders, granules and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered peptide or peptidomimetic moistened with an inert liquid diluent.

Tablets, and other solid dosage forms, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions, which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions, which can be used include polymeric substances and waxes. The active ingredient can also be in micro-encapsulated form, if appropriate, with one or more of the above-described excipients.

Liquid dosage forms for oral administration include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active agent may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations for rectal or vaginal administration may be presented as a suppository, which may be prepared by mixing one or more respiration uncoupling agents with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active agent.

Formulations which are suitable for vaginal administration also include pessaries, tampons, creams, gels, pastes, foams or spray formulations containing such carriers as are known in the art to be appropriate.

Dosage forms for the topical or transdermal administration of an agent that modulates (e.g., inhibits) biomarker expression and/or activity include powders, sprays, ointments, pastes, creams, lotions, gels, solutions, patches and inhalants. The active component may be mixed under sterile conditions with a pharmaceutically-acceptable carrier, and with any preservatives, buffers, or propellants which may be required.

The ointments, pastes, creams and gels may contain, in addition to a respiration uncoupling agent, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to an agent that modulates (e.g., inhibits) biomarker expression and/or activity, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

The agent that modulates (e.g., inhibits) biomarker expression and/or activity, can be alternatively administered by aerosol. This is accomplished by preparing an aqueous aerosol, liposomal preparation or solid particles containing the compound. A nonaqueous (e.g., fluorocarbon propellant) suspension could be used. Sonic nebulizers are preferred because they minimize exposing the agent to shear, which can result in degradation of the compound.

Ordinarily, an aqueous aerosol is made by formulating an aqueous solution or suspension of the agent together with conventional pharmaceutically acceptable carriers and stabilizers. The carriers and stabilizers vary with the requirements of the particular compound, but typically include nonionic surfactants (Tweens, Pluronics, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

Transdermal patches have the added advantage of providing controlled delivery of a respiration uncoupling agent to the body. Such dosage forms can be made by dissolving or dispersing the agent in the proper medium. Absorption enhancers can also be used to increase the flux of the peptidomimetic across the skin. The rate of such flux can be controlled by either providing a rate controlling membrane or dispersing the peptidomimetic in a polymer matrix or gel.

Ophthalmic formulations, eye ointments, powders, solutions and the like, are also contemplated as being within the scope of this invention.

Pharmaceutical compositions of this invention suitable for parenteral administration comprise one or more respiration uncoupling agents in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the present invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of an agent that modulates (e.g., inhibits) biomarker expression and/or activity, in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions, which are compatible with body tissue.

When the respiration uncoupling agents of the present invention are administered as pharmaceuticals, to humans and animals, they can be given per se or as a pharmaceutical composition containing, for example, 0.1 to 99.5% (more preferably, 0.5 to 90%) of active ingredient in combination with a pharmaceutically acceptable carrier.

Actual dosage levels of the active ingredients in the pharmaceutical compositions of this invention may be determined by the methods of the present invention so as to obtain an amount of the active ingredient, which is effective to achieve the desired therapeutic response for a particular subject, composition, and mode of administration, without being toxic to the subject.

The nucleic acid molecules of the present invention can be inserted into vectors and used as gene therapy vectors. Gene therapy vectors can be delivered to a subject by, for example, intravenous injection, local administration (see U.S. Pat. No. 5,328,470) or by stereotactic injection (see *e.g.*, Chen et al. (1994) Proc. Natl. Acad. Sci. USA 91:3054 3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g*., retroviral vectors, the pharmaceutical preparation can include one or more cells which produce the gene delivery system.

The present invention also encompasses kits for detecting and/or modulating biomarkers described herein. A kit of the present invention may also include instructional materials disclosing or describing the use of the kit or an antibody of the disclosed invention in a method of the disclosed invention as provided herein. A kit may also include additional components to facilitate the particular application for which the kit is designed. For example, a kit may additionally contain means of detecting the label (*e.g*., enzyme substrates for enzymatic labels, filter sets to detect fluorescent labels, appropriate secondary labels such as a sheep anti-mouse-HRP, *etc.*) and reagents necessary for controls (*e.g*., control biological samples or standards). A kit may additionally include buffers and other reagents recognized for use in a method of the disclosed invention. Non-limiting examples include agents to reduce non-specific binding, such as a carrier protein or a detergent.

### Exemplification

This invention is further illustrated by the following examples, which should not be construed as limiting.

### Example 1: Materials and Methods for Examples 2-4

### a. Patients

The patients with metastatic melanoma enrolled in the phase 1 trial of Ipi or Ipi-Bev have been described in Hodi et al. (2014) Cancer Immunol. Res. 2:632-642. Additional ipilimumab samples were obtained from expanded access trials during the FDA approval process, as well as from post-approval commercial use. Pre-treatment and on-treatment (1-2 months after the initiation of treatment) serum samples were collected as described below from metastatic patients receiving immune therapy (i.e., ipilimumab (Ipi) alone or ipilimumab plus bevacizumab (Ipi-Bev)). Angiopoietin-2 (Ang-2) levels in the pre- and post-treatment serum samples were determined using Luminex^{®} assays described below. Correlation analysis of the pre- and post-treatment Ang-2 levels and the change of Ang-2 as function of therapy with clinical response and survival of the patients were also determined.

### b. Serum collections

Blood samples collected in Red Top tubes were centrifuged at 1,000x g for 15 min. at room temperature (RT). The supernatant (serum) was collected and stored < -20°C in aliquots.

### c. Ang-2 measurement

Serum Ang-2 was measured using Human Angiogenesis/Growth Factor Magnetic Bead Panel 1 kits (Millipore, Billerica, MA) and Magnetic Luminex Screening Assay kits (R&D Systems, Minneapolis, MN). The instructions provided by the manufacturers were followed exactly.

### d. Statistical analysis

The algorithm of Contal-O'Quigley was used to estimate the optimal division point of pre-treatment Ang-2. This algorithm divides the sample into high and low based on each possible value of pre-treatment Ang-2 and assesses overall survival based on the resulting two categories. The division point with the largest log-rank statistic was declared to be the "best" division point. Overall survival was defined as the time from trial enrollment to death from any cause. The survival distribution was summarized using the method of Kaplan-Meier. Confidence intervals were estimated using log(-log(survival)) methodology. Conditional landmark analysis was used to explore the relationship between 3.1-month fold-change in Ang-2 and survival. This method minimizes the potential for guarantee-time bias. Patients who were alive and had pre-treatment and subsequent Ang-2 measurements within 3.1 months were followed forward in time. Cox proportion hazards models were used to describe the relationship between Ang-2 categories and response or survival. Cox models were stratified by trial (Ipi, Ipi-Bev, and PD-1) to allow for differences between trials in the baseline hazard of death. Hazard ratios are shown with 95% confidence intervals. Statistical significance of Cox model results are based on the Wald test. In some cases, GraphPad Prism 6 software was also used for statistical analysis of association of Ang-2 with survival and comparison of Ang-2 levels. The association of pre-treatment serum Ang-2 or Ang-2 fold change and clinical response and the association of immune therapy and serum Ang-2 change were evaluated using the Fisher's exact test. The correlation between immune therapy and serum Ang-2 fold change were examined using the Kruskal-Wallis rank test. The differences in migration of macrophages were evaluated using unpaired, two-tailed *t* test. P < 0.05 was considered statistically significant for all comparisons.

GraphPad Prism 6 software was used for statistical analysis of association of Ang-2 with survival and comparison of Ang-2 levels.

### e. Immunohistochemical analysis

For IHC staining of Ang-2, CD163, and CD206, 5-µm-thick paraffin-embedded sections were pre-baked at 60°C for 1 hour, deparaffinized, and rehydrated. Antigen retrieval was induced by heating sections in citrate buffer (pH 6.0, Invitrogen, Carlsbad, CA) for 30 minutes using a steamer. After cooling for 30 minutes, sections were treated with peroxidase block (DAKO, Carpinteria, CA) for 5 minutes, followed by application of a serum-free protein block (DAKO) for 20 minutes. Slides were then incubated overnight at 4°C with primary antibodies against Ang-2 (1:25, sc-74403, Santa Cruz, Dallas, Texas) or CD163 (1:200, 10D6, NeoMarkers, Fremont, CA) diluted in Da Vinci Green Diluent (Biocare Medical, Concord, CA). For secondary reagents, EnVision^{™} anti-mouse HRP-labeled polymer (DAKO) was applied for 30 minutes to sections for CD163 staining. Ang-2 sections were incubated with Novocastra Post Primary (Leica Biosystems, Buffalo Grove, IL) for 30 minutes, followed by Novolink^{™} Polymer (Leica) for 30 minutes. Sections were then developed with diaminobenzidine (DAKO), counterstained with hematoxylin, dehydrated, and mounted. CD68 (PG-M1, DAKO) staining was performed using an automated staining system (Bond^{™} III, Leica Biosystems) following the manufacturer's protocols for the Bond^{™} Polymer Refine detection system (Leica Biosystems). Heat-induced antigen retrieval was performed using ER1 solution (pH 6.0) (Leica Biosystems) for 30 minutes. Anti-CD68 antibody was diluted 1:200 in Da Vinci Green Diluent and incubated for 30 minutes. Slides were removed from the autostainer to be dehydrated and mounted. Ang-2 expression was observed in cytoplasm of tumor cells and endothelia of small blood vessels.

### f. Generation, treatment, and migration of monocyte derived macrophages

Monocytes were isolated form frozen human PBMC of healthy donors using the adhesion method. The attached monocytes were treated with M-CSF to differentiate into macrophages. To induce M1 and M2 polarization, macrophages were treated with IFNy (100 ng/ml) plus LPS (20 ng/ml), M-CSF (100 ng/ml), IL-4 (10 or 20 ng/ml), or IL-10 (10 or 20 ng/ml) for 2 days. In some experiments, Ang-2 (300 ng/ml; R&D Systems) was added to the macrophages to examine its effect on PD-L1 expression. PD-L1expression on macrophages was detected by staining the cells with PE-conjugated anti-human PD-L1 and FACS analysis. FACS data were analyzed using FlowJo software. Migration was performed on Transwell 6.5 mm inserts with 8.0 µm polycarbonate membrane. M1 and M2 polarized macrophages were detached and resuspended in R-PS1 medium R-PS1 medium (RPMI1640 containing 50 µg/ml penicillin, 100 µg/ml streptomycin and 1% FBS) and 100 µl (0.5 - 1 × 10⁵) of cells were added onto each insert. The inserts were placed onto the lower chambers containing 600 µl of R-PS1 with BSA or 300 ng/ml recombinant Ang-2 and incubated for 4-5 hours at 37°C and 5% CO2. Cells that did not migrate were carefully removed using cotton swabs from the upside of the inserts and transmigrated cells were fixed in methanol for and stained in PBS containing 0.1% Triton X-100 and 20 µg/ml propidium iodide (PI). Migrated cells were photographed from 5 randomly selected fields for each insert under a fluorescence microscope and counted using imaged software (National Institutes of Health).

### g. Culture and treatment of tumor associated endothelial cells and melanoma cells

Melanoma tumor samples were obtained from patients on Dana-Farber/Harvard Cancer Center Institutional Review Board-approved protocols. Tumor-associated endothelial cells (TEC) were isolated using Dynabeads^{®} CD31 Endothelial Cells according to manufacturer's instructions (Life Technologies, Grand Island, NY) and confirmed by surface expression of CD31 and tube formation. TEC were cultured in EGM-2 (Lonza). Melanoma cell lines, K008, M028, M34 and A375, were cultured in DMEM supplemented with 50 µg/ml penicillin, 100 µg/ml streptomycin and 1% FBS. In some experiments, TEC and melanoma cells were cultured in a hypoxic chamber with 1% O₂. To examine the effect of VEGF and Bev on Ang-2 expression, TEC were incubated with VEGF (100 ng/ml) and/or Bev (25 µg/ml) in EGM-2 or serum and angiogenesis factor reduced EBM/EGM-2 medium and melanoma cells were cultured in DMEM containing 1% FBS respectively.

### Example 2: Therapy with ipilimumab (Ipi) alone or in combination with other anti-cancer therapeutics alters circulating Ang-2 levels in metastatic melanoma patients

Ang-2 levels were analyzed in pre-treatment serum samples collected at the time of registration of patients, as well as from the first post-treatment serum samples collected at 1-3.1 months after the start of treatment. The median baseline Ang-2 levels were 2554 (697 - 12578), 2183 (505 - 12061) and 2038 (1083-5163) pg/ml for Ipi alone, Ipi-Bev and PD-1 blockade treated patients, respectively. The post-treatment median Ang-2 levels were 2797 (646-44790), 2465 (565-11672) and 2686 (1006-8982) pg/ml for Ipi alone, Ipi-Bev, and PD-1 blockade patients, respectively. There was a median increase of 12.0 and 10% in the circulating Ang-2 levels of Ipi and PD-1 blockade patients, while a median reduction of 17.2% in the circulating Ang-2 levels of Ipi-Bev patients as function of treatment was observed. The mean Ang-2 fold change of Ipi-Bev patients was statistically smaller than that of Ipi and PD-1 blockade patients (Figure 1A; p = 0.0004). ).

For the particular analysis described herein, a change in Ang-2 by 25% (i.e., post-treatment/pre-treatment ratio ≥ 1.25 or ≤ 0.75) was considered as being significant. By this cutoff, 9.3, 31.7, 37.5% of Ipi-Bev, lpi alone and PD-1 blockade patients had increased, while 37.2, 14.6, and 8.3% of Ipi-Bev, Ipi, and PD-1 blockade patients had decreased circulating Ang-2 as function of treatment (Figures 1B and 1C). Thus, therapy with Ipi alone or PD-1 blockade increased circulating Ang-2 in a significant portion of the patients, while Ipi-Bev had an opposite effect. The post-treatment circulating Ang-2 levels were associated with the pre-treatment levels in all three groups of patients (Figure 2).

### Example 3: Increased circulating Ang-2 is associated with PD or SD

Association analysis found that progressive disease (PD) patients were associated with higher pre- and post-treatment circulating Ang-2 levels compared to CR and PR patients receiving Ipi-Bev or PD-1 blockade, although a similar association was not observed in patients treated with Ipi alone (Figures 3A-3C). However, among the 41 Ipi alone patients, 13 had increased circulating Ang-2 as function of treatment and 10 of them had PD and 2 had SD (Figure 4A). Patients with PD had higher post-/pre-treatment ratios compared to those with CR, PR or SD (Figure 4B). Among the 24 PD-1 blockade-treated patients, 9 of them displayed increased serum Ang-2 and 6 of them had PD and 3 had SD, while none of them achieved PR or CR (Figure 4C). PD and SD patients of this cohort had larger fold changes than PR patients (Figure 4D). Among the 43 Ipi-Bev patients, 4 had increased post-treatment Ang-2 levels, 3 had PD, and one had SD (Figure 4E). In this group, Ang-2 fold changes are comparable among the CR/PR, SD and PD patients (Figure 4F). These results indicate that an increase in circulating Ang-2 levels is associated with poor response to Ipi based immunotherapy.

### Example 4: Increased circulating Ang-2 or increased pre-treatment serum Ang-2 is associated with decreased survival and/or lower response rate

Association analysis found that an increase in circulating Ang-2 by 25% or more was associated with decreased survival in lpi alone- and PD-1 blockade-treated patients (Figure 5A and 5B). In lpi-Bev-treated patients, an increase in Ang-2 was also associated with a trend toward shortened survival (Figure 5C). As described above, one of the Ipi-Bev patients with increased circulating Ang-2 had SD. Interestingly, Ipi-Bev therapy was found to induce potent humoral immune response to Ang-2 and robustly increased circulating anti-Ang-2 antibody levels in this patient. These finding indicate neutralization of Ang-2 by its antibody. When this patient was not considered, an increase in circulating Ang-2 by 25% or more was strongly associated with shortened survival in Ipi-Bev patients (Figure 5D). Furthermore, when Ipi-Bev patients were combined with Ipi- or Ipi and PD-1 blockade-treated patients, an increase in circulating Ang-2 was significantly associated with decreased survival (Figures 5E-5F).

Similarly, association analysis also found that an increase in pre-treatment serum Ang-2 was associated with decreased survival (Figure 6). A high pre-treatment serum Ang-2 level (e.g., > 3,175 pg/mL) was strongly associated with shortened survival in subjects treated with immune checkpoint blockers (Figures 6A-6E). High pre-treatment serum Ang-2 levels was associated with reduced response rate in PD-1 blockade- and Ipi-Bev blockade-treated patients (Figures 7A-7B). Furthermore, an increase in circulating Ang-2 by 25% or more was significantly associated with reduced response rate in PD-1 blockade-treated patients (Figure 7C) and all patients combined (Figure 7D).

Analysis of the pooled patients of all three immune checkpoint therapy found that high pre-treatment serum Ang-2 level followed by a therapy-induced increase was a strong predictor for poor overall survival and progressive disease (Figures 8A-8C). Patients with high pre-treatment Ang-2 levels and large fold changes (e.g., ≥ 1.25-fold change) had the worst overall survival, while those with low pre-treatment Ang-2 and small fold changes (e.g., < 1.25-fold change) had the best survival (Figure 8A). Patients with high pre-treatment Ang-2 and small fold changes, or low pre-treatment and large fold changes, had median survivals in between. None (0%) of the 11 patients with high pre-treatment Ang-2 and large fold change achieved PR/CR, as compared with 16 (26.2%) of the 61 patients with low pre-treatment Ang-2 and small fold change who did achieve PR/CR (Figure 8B). On the other hand, 10 (90.9%) of the 11 patients with high pre-treatment Ang-2 and large fold change had PD, as compared with 29.5% of the patients with low pre-treatment Ang-2 and small fold change (Figure 8C). The difference in progression rate between these two categories was statistically significant (P = 0.0002). Patients with high pre-treatment Ang-2 and small Ang-2 fold change also had a lower progression rate (38.1%) compared to those with high pre-treatment Ang-2 and large fold change (P = 0.0075). These observations indicate that high pre-treatment Ang-2 level followed by therapy-induced increase is a strong predictor for PD and poor survival.

The effects of Ipi and Ipi-Bev treatment on Ang-2 expression and macrophage infiltration in melanoma were also analyzed using immunohistochemistry. Figure 9A shows a representative Ipi-treated melanoma in which Ang-2 expression was significantly increased after treatment along with corresponding macrophage infiltration determined using CD68 (as a biomarker for macrophages) and CD163 (as a biomarker M2 macrophages). Similarly, increased tumor vascular Ang-2 expression was associated with increased infiltration of CD68+ and CD163+ macrophages in a representative lpi-Bev-treated melanoma (Figure 9B). By contrast, decreased macrophage infiltration was seen in representative melanoma samples from Ipi-Bev-treated samples showing reduced tumor vascular Ang-2 expression after treatment (Figure 9C). Overall, tumor CD68⁺ and/or CD163⁺ macrophage infiltration correlated with tumor vascular Ang-2 expression (Figures 9D-9E). It is believed that Bev (anti-VEGF) reduces Ang-2 expression in tumor associated endothelial cells (TEC) by blocking VEGF-induced Ang-2 expression. This is supported by *in vitro* findings that Bev reduced Ang-2 expression in TEC (Figure 10A) and, specifically, VEGF enhanced Ang-2 expression in TEC under normoxic and hypoxic conditions and Bev blocked VEGF induced Ang-2 expression (Figure 10B). It was further determined from *in vitro* studies that hypoxia increased Ang-2 expression, while VEGF appeared to have little effect in melanoma cells (Figure 10C).

The association between tumor vascular Ang-2 expression and tumor macrophage infiltration indicates that Ang-2 acts as a chemoattractant for monocytes/macrophages. For example, monocytes derived from healthy donors were differentiated and activated into M1-like macrophages with IFNy/LPS or M2-like macrophages with M-CSF, IL-4 or IL-10. Ang-2 promoted migration of M-CSF, IL-4 and IL-10 activated M2-like macrophages but had little effect on migration of IFNy/LPS activated M1-like macrophages (Figures 11A-11B). The association of serum Ang-2 level and clinical outcomes to immune checkpoint therapy indicates that Ang-2 plays a role in immune regulation, presumably via mechanisms involving PD-1/PD-L1. Indeed, *in vitro* studies found that Ang-2 significantly enhanced PD-L1 expression on M-CSF, IL-10 or IL-4 activated M2-like macrophages (Figures 12A-12C).

Taken together, these results indicate that circulating Ang-2 was detected in the pre- and post-treatment samples of all the patients and high pre-treatment serum Ang-2 (e.g., > 3,175 pg/ml) and/or an increase in circulating Ang-2 level by 25% or more as a function of PD-1 blockade, Ipi, or Ipi-Bev treatment was found to be correlated with clinical outcomes. Patients with high pre-treatment circulating Ang-2, Ang-2 fold change ≥ 1.25, or both, as a function of therapy, had an association with shortened overall survival and/or reduced response rate. Among the 108 PD-1 blockade-, Ipi-, and Ipi-Bev-treated patients, 11 patients had high pre-treatment serum Ang-2 and increased circulating Ang-2 (fold change ≥ 1.25) and 10 of them had PD, while none of them achieved PR or CR. Thus, the combination of high pre-treatment and an increase in circulating Ang-2 is a strong indicator for disease progression and poor survival.

High pre-treatment circulating Ang-2 and/or early increases in circulating Ang-2 as function of PD-1 blockade and lpi based immune therapy, either Ipi alone or in combination with anti-VEGF blockade, Bev, were associated with poor clinical outcomes in advanced melanoma patients. Patients with high pre-treatment circulating Ang-2 had worse survival. Patients with an increase by ≥ 25% in circulating Ang-2 as function of PD-1 blockade-, lpi blockade-, or Ipi-Bev blockade-therapy had reduced response rate and worse survival compared to those with decreased or unaltered circulating Ang-2. Furthermore, high pre-treatment circulating Ang-2 followed by an increase is associated with increased progression rate and poor survival. These findings indicate that high pre-treatment circulating Ang-2, an increase in Ang-2 or their combination can be used as an early predictive biomarker for PD and decreased survival for melanoma patients receiving PD-1 and CTLA-4 based immune therapy. As circulating Ang-2 can be easily and accurately measured by well-established methods and the increase occurs as early as 1-3 months after the initial of treatment, Ang-2 provides a safe, convenient, and cost-effective biomarker that could be used in clinical practice to identify those patients who are resistance to and may not benefit from anti-immune checkpoint therapies, such as anti-PD-1 and anti-CTLA-4-based immune therapy, in the early phase of treatment.

The association of high pre-treatment circulating Ang-2 and increase in circulating Ang-2 with PD and worsened clinical outcomes indicates that increased Ang-2 is a mechanism for resistance to PD-1 and CTLA-4 based immune therapy. Such a mechanism is further supported by the findings that tumor macrophage infiltration was positively associated with tumor vascular Ang-2 expression and that Ang-2 promoted PD-L1 expression on and migration of protumoral, proangiogenic and immunosuppressive M2 macrophages. This is also supported by the fact that Ang-2 increase occurred prior to the clinical observation of PD. Furthermore, elevated levels of circulating Ang-2 are associated with poor prognosis for many types of cancers. Circulating Ang2 has also been identified as a biomarker for progression and metastasis in melanoma and has been found to be expressed by tumor-associated endothelial cells and melanoma cells (Helfrich et al. (2009) Clin. Cancer Res. 15:1384-1392). siRNA-mediated silencing of Ang-2 has been identified to strongly reduce invasive and migratory capacity of melanoma cells. Thus, the results described herein indicate that the addition of anti-Ang-2 therapeutics to PD-1 and lpi-based immune therapy (PD-1 blockade, Ipi, or in combination) improves the clinical efficacy of PD-1 blockade or Ipi.

The results described herein also indicate that higher portions of PD-1 blockade and lpi patients exhibited increased circulating Ang-2 than Ipi-Bev patients as function of treatment. It is believed that this observation is related to the distinct effect of these treatments on tumor vasculature. Ang-2 has been shown to be upregulated by hypoxia, VEGF, and PEG2. In agreement, *in vitro* studies found that hypoxia enhanced Ang-2 expression in melanoma cells and VEGF induced Ang-2 expression in melanoma associated endothelial cells and Bev blocked VEGF induced Ang-2 expression in these cells. Vessel destruction (vasculopathy) has been found in tumors of melanoma patients receiving lpi or Ipi-GVAX and increased Ang-2 expression was observed in post-treatment tumors of a portion of Ipi patients (Hodi et al. (2003) Proc. Natl. Acad. Sci. U.S.A. 100:4712-4717). Based on the results described herein, it is believed that lpi therapy makes the tumor microenvironments more hypoxic and increases Ang-2 expression and secretion, resulting in elevation of circulating Ang-2. Interestingly, vasculopathy was not found in tumors of melanoma patients treated with Ipi-Bev, consistent with vessel normalization by Bev. Normalization of tumor vessels could make the tumor microenvironments less hypoxic and downregulate Ang-2 expression. In agreement, endothelial Ang-2 expression was found to be reduced in the post-treatment tumor biopsies of Ipi-Bev patients compared to the paired pre-treatment samples.

## Claims

1. A method of identifying the likelihood of a cancer in a subject to be responsive to an anti-immune checkpoint therapy, wherein the anti-immune checkpoint therapy comprises a therapy targeting CTLA-4 or PD-1, the method comprising:
a) using a subject sample from a patient having cancer;
b) measuring the amount or activity of human Ang2 in the subject sample; and
c) comparing said amount or activity of human Ang2 in a control sample,
wherein a significantly increased amount or activity of human Ang2 in the subject sample relative to the control sample identifies the cancer as being less likely to be responsive to the anti-immune checkpoint therapy and wherein a decreased amount or activity of human Ang2 in the subject sample relative to the control sample identifies the cancer as being more likely to be responsive to the anti-immune checkpoint therapy.

2. A method of identifying the likelihood of a cancer in a subject to be responsive to anti-immune checkpoint therapy, wherein the anti-immune checkpoint therapy comprises a therapy targeting CTLA-4 or PD-1, the method comprising:
a) using a subject sample from a patient having cancer, wherein the subject sample comprises nucleic acid molecules from the subject;
b) determining the copy number of human Ang2 in the sample; and
c) comparing said copy number to that of a control sample, wherein an increased copy number of human Ang2 in the subject sample relative to the control sample identifies the cancer as being less likely to be responsive to the anti-immune checkpoint therapy and wherein a decreased copy number of human Ang2 in the subject sample relative to the control sample identifies the cancer as being more likely to be responsive to the anti-immune checkpoint therapy.

3. The method of claim 1 or 2, further comprising recommending, prescribing, or administering anti-immune checkpoint therapy if the cancer is determined likely to be responsive to anti-immune checkpoint therapy or administering anti-cancer therapy other than anti-immune checkpoint therapy if the cancer is determined be less likely to be responsive to anti-immune checkpoint therapy,
preferably wherein the anti-cancer therapy is selected from the group consisting of targeted therapy, chemotherapy, radiation therapy, and/or hormonal therapy.

4. The method of any one of claims 1-3, wherein the control sample is determined from a cancerous or non-cancerous sample from either the subject or a member of the same species to which the patient belongs,
preferably wherein the control sample is a cancerous or non-cancerous sample from the patient obtained from an earlier point in time than the subject sample, optionally wherein the control sample is obtained before the patient has received anti-immune checkpoint therapy and the subject sample is obtained after the patient has received anti-immune checkpoint therapy, or/and
wherein the control sample comprises cells or does not comprise cells, or/and
wherein the control sample comprises cancer cells known to be responsive or non-responsive to the anti-immune checkpoint therapy.

5. A method of assessing the efficacy of an anti-immune checkpoint agent for treating a cancer in a subject that is unlikely to be responsive to anti-immune checkpoint therapy, wherein the anti-immune checkpoint therapy comprises a therapy targeting CTLA-4 or PD-1, comprising:
a) detecting in a first subject sample and maintained in the presence of the agent the amount or activity of human Ang2;
b) detecting the amount or activity of human Ang2 in a second subject sample and maintained in the absence of the agent; and
c) comparing the amount or activity of human Ang2 from steps a) and b), wherein a significantly increased amount or activity of human Ang2 in the first subject sample relative to at least one subsequent subject sample, indicates that the agent treats the cancer in the subject.

6. An *in vitro* or *ex vivo* method of assessing the efficacy of an anti-immune checkpoint agent for treating a cancer in a subject or prognosing progression of a cancer in a subject, wherein the anti-immune checkpoint therapy comprises a therapy targeting CTLA-4 or PD-1, comprising:
a) detecting in a subject sample at a first point in time the amount or activity of human Ang2;
b) repeating step a) during at least one subsequent point in time after administration of the agent; and
c) comparing the expression and/or activity detected in steps a) and b), wherein a significantly increased amount or activity of human Ang2_in the first subject sample relative to at least one subsequent subject sample, indicates that the cancer is unlikely to progress or that the agent treats the cancer in the subject,
preferably wherein between the first point in time and the subsequent point in time, the subject has undergone treatment, completed treatment, and/or is in remission for the cancer, or
wherein the first and/or at least one subsequent subject sample has been obtained from an animal model of the cancer, or
wherein the first and/or at least one subsequent subject sample is a portion of a single subject sample or pooled samples obtained from the subject.

7. The method or assay of any one of claims 1-6, wherein the subject sample and/or the control sample has not been contacted with any melanoma treatment or inhibitor of an immune checkpoint, or/and
wherein the subject has not been administered any melanoma treatment or inhibitor of an immune checkpoint, or/and
further comprising recommending, prescribing, or administering at least one additional anti-cancer therapeutic agent, optionally wherein the at least one additional anti-cancer therapeutic agent is bevacizumab and/or an anti-Ang-2 therapeutic agent, or/and
wherein the subject sample is selected from the group consisting of serum, whole blood, plasma, urine, cells, cell lines, and biopsies.

8. The method or assay of any one of claims 1-7, wherein the amount of human Ang2 is detected using a reagent which specifically binds with the protein,
preferably wherein the reagent is selected from the group consisting of an antibody, an antibody derivative, and an antibody fragment.

9. The method or assay of any one of claims 1-7, wherein human Ang2 is assessed by detecting the presence in the subject sample of a transcribed polynucleotide or portion thereof,
preferably wherein the transcribed polynucleotide is an mRNA or a cDNA, or/and
wherein the step of detecting further comprises amplifying the transcribed polynucleotide, or/and
wherein the transcribed polynucleotide is detected by identifying a nucleic acid that anneals with the biomarker nucleic acid, or a portion thereof, under stringent hybridization conditions, or/and
wherein human Ang-2 is a fragment thereof, or/and
wherein the anti-immune checkpoint therapy comprises at least one antibody selected from the group consisting of anti-CTLA-4 antibodies, anti-PD-1 antibodies, anti-PD-L1 antibodies, anti-PD-L2 antibodies, and combinations thereof,
preferably wherein the anti-immune checkpoint therapy comprises ipilimumab.

10. The method or assay of any one of claims 1-9, wherein the human Ang-2 protein comprises the amino acid sequence of SEQ ID NO: 2, SEQ ID NO: 4, or SEQ ID NO: 6.

11. The method or assay of any one of claims 1-10, wherein the cancer is a solid tumor.

12. The method or assay of claim 11, wherein the tumor is melanoma, non-small cell lung cancer (SCLC), or renal cell cancer.

13. The method or assay of claim 12, wherein the melanoma is metastatic melanoma.

14. The method or assay of any one of claims 1-13, wherein the anti-immune checkpoint therapy further comprises a therapy targeting VEGF.

15. The method or assay of any one of claims 1-14, wherein the subject is a mammal or an animal model of cancer, preferably wherein the mammal is a human.

## Patentansprüche

1. Verfahren zur Identifizierung der Wahrscheinlichkeit, dass ein Krebs in einem Probanden auf eine Anti-Immun-Checkpoint-Therapie anspricht, wobei die Anti-Immun-Checkpoint-Therapie eine Therapie umfasst, die auf CTLA-4 oder PD-1 abzielt, wobei das Verfahren die Schritte umfasst:
a) Verwenden einer Probandenprobe eines Patienten mit Krebs;
b) Messen der Menge oder Aktivität von menschlichem Ang2 in der Probandenprobe; und
c) Vergleichen der Menge oder Aktivität von menschlichem Ang2 in einer Kontrollprobe, wobei eine signifikant erhöhte Menge oder Aktivität von menschlichem Ang2 in der Probandenprobe im Vergleich zur Kontrollprobe auf eine geringere Wahrscheinlichkeit des Ansprechens des Krebses auf die Anti-Immun-Checkpoint-Therapie hinweist und wobei eine verringerte Menge oder Aktivität von menschlichem Ang2 in der Probandenprobe im Vergleich zur Kontrollprobe auf eine höhere Wahrscheinlichkeit des Ansprechens des Krebses auf die Anti-Immun-Checkpoint-Therapie hinweist.

2. Verfahren zur Identifizierung der Wahrscheinlichkeit, dass ein Krebs in einem Probanden auf eine Anti-Immun-Checkpoint-Therapie anspricht, wobei die Anti-Immun-Checkpoint-Therapie eine Therapie umfasst, die auf CTLA-4 oder PD-1 abzielt, wobei das Verfahren die Schritte umfasst:
a) Verwenden einer Probandenprobe eines Patienten mit Krebs, wobei die Probandenprobe Nukleinsäuremoleküle des Probanden umfasst;
b) Bestimmen der Kopienzahl von menschlichem Ang2 in der Probe; und
c) Vergleichen der Kopienzahl mit der einer Kontrollprobe, wobei eine erhöhte Kopienzahl von menschlichem Ang2 in der Probandenprobe im Vergleich zur Kontrollprobe auf eine geringere Wahrscheinlichkeit des Ansprechens des Krebses auf die Anti-Immun-Checkpoint-Therapie hinweist und wobei eine verringerte Kopienzahl von menschlichem Ang2 in der Probandenprobe im Vergleich zur Kontrollprobe auf eine höhere Wahrscheinlichkeit des Ansprechens des Krebses auf die Anti-Immun-Checkpoint-Therapie hinweist.

3. Verfahren nach Anspruch 1 oder 2, ferner umfassend das Empfehlen, Verschreiben oder Verabreichen einer Anti-Immun-Checkpoint-Therapie, wenn festgestellt wird, dass der Krebs wahrscheinlich auf eine Anti-Immun-Checkpoint-Therapie anspricht, oder das Verabreichen einer anderen Anti-Krebs-Therapie als einer Anti-Immun-Checkpoint-Therapie, wenn festgestellt wird, dass der Krebs weniger wahrscheinlich auf eine Anti-Immun-Checkpoint-Therapie anspricht, vorzugsweise wobei die Anti-Krebs-Therapie aus der Gruppe ausgewählt ist, die aus einer zielgerichteten Therapie, Chemotherapie, Strahlentherapie und/oder Hormontherapie besteht.

4. Verfahren nach einem der Ansprüche 1 bis 3, wobei die Kontrollprobe aus einer Krebs- oder Nicht-Krebs-Probe des Probanden oder eines Mitglieds der gleichen Spezies, zu welcher der Patient gehört, bestimmt wird,
vorzugsweise wobei es sich bei der Kontrollprobe um eine Krebs- oder Nicht-Krebs-Probe des Patienten handelt, die zu einem früheren Zeitpunkt als die Probandenprobe gewonnen wurde, wobei die Kontrollprobe gegebenenfalls gewonnen wird, bevor der Patient eine Anti-Immun-Checkpoint-Therapie erhalten hat, und die Probandenprobe gewonnen wird, nachdem der Patient eine Anti-Immun-Checkpoint-Therapie erhalten hat, oder/und wobei die Kontrollprobe Zellen umfasst oder keine Zellen umfasst, oder/und wobei die Kontrollprobe Krebszellen umfasst, von denen bekannt ist, dass sie auf die Anti-Immun-Checkpoint-Therapie ansprechen oder nicht ansprechen.

5. Verfahren zur Bewertung der Wirksamkeit eines Anti-Immun-Checkpoint-Mittels zur Behandlung von Krebs bei einem Probanden, bei dem es unwahrscheinlich ist, dass er auf eine Anti-Immun-Checkpoint-Therapie anspricht, wobei die Anti-Immun-Checkpoint-Therapie eine Therapie umfasst, die auf CTLA-4 oder PD-1 abzielt, umfassend die Schritte:
a) Nachweis der Menge oder Aktivität von menschlichem Ang2 in einer ersten Probandenprobe, die in Gegenwart des Mittels aufrechterhalten wird;
b) Nachweis der Menge oder Aktivität von menschlichem Ang2 in einer zweiten Probandenprobe, die in Abwesenheit des Mittels aufrechterhalten wird; und
c) Vergleichen der Menge oder Aktivität von menschlichem Ang2 aus den Schritten a) und b), wobei eine signifikant erhöhte Menge oder Aktivität von menschlichem Ang2 in der ersten Probandenprobe relativ zu mindestens einer nachfolgenden Probandenprobe darauf hinweist, dass das Mittel den Krebs in dem Probanden behandelt.

6. *In vitro-* oder *ex vivo*-Verfahren zur Bewertung der Wirksamkeit eines Anti-Immun-Checkpoint-Mittels zur Behandlung von Krebs in einem Probanden oder zur Prognose des Fortschreitens von Krebs in einem Probanden, wobei die Anti-Immun-Checkpoint-Therapie eine auf CTLA-4 oder PD-1 abzielende Therapie umfasst, umfassend die Schritte:
a) Nachweis der Menge oder Aktivität von menschlichem Ang2 in einer Probandenprobe zu einem ersten Zeitpunkt;
b) Wiederholung von Schritt a) während mindestens eines späteren Zeitpunkts nach Verabreichung des Mittels; und
c) Vergleichen der in den Schritten a) und b) nachgewiesenen Expression und/oder Aktivität, wobei eine signifikant erhöhte Menge oder Aktivität von menschlichem Ang2 in der ersten Probandenprobe im Vergleich zu mindestens einer nachfolgenden Probandenprobe darauf hinweist, dass ein Fortschreiten des Krebses unwahrscheinlich ist oder dass das Mittel den Krebs in dem Probanden behandelt,
vorzugsweise wobei zwischen dem ersten Zeitpunkt und dem späteren Zeitpunkt der Proband sich einer Behandlung unterzogen hat, die Behandlung abgeschlossen hat und/oder eine Remission des Krebses erreicht hat, oder
wobei die erste und/oder mindestens eine nachfolgende Probandenprobe aus einem Tiermodell des Krebses gewonnen wurde, oder
wobei die erste und/oder mindestens eine nachfolgende Probandenprobe ein Teil einer einzelnen Probandenprobe oder von dem Probanden erhaltener Sammelproben ist.

7. Verfahren oder Assay nach einem der Ansprüche 1 bis 6, wobei die Probandenprobe und/oder die Kontrollprobe nicht mit einer Melanombehandlung oder einem Inhibitor eines Immun-Checkpoints in Kontakt gebracht worden ist, oder/und
wobei dem Probanden keine Melanombehandlung oder kein Inhibitor eines Immun-Checkpoints verabreicht wurde, oder/und
ferner umfassend das Empfehlen, Verschreiben oder Verabreichen von mindestens einem zusätzlichen Anti-Krebs-Therapeutikum, wobei es sich bei dem mindestens einen zusätzlichen Anti-Krebs-Therapeutikum gegebenenfalls um Bevacizumab und/oder ein Anti-Ang-2-Therapeutikum handelt, oder/und
wobei die Probandenprobe aus der Gruppe ausgewählt ist, die aus Serum, Vollblut, Plasma, Urin, Zellen, Zelllinien und Biopsien besteht.

8. Verfahren oder Assay nach einem der Ansprüche 1 bis 7, wobei die Menge an menschlichem Ang2 unter Verwendung eines Reagens nachgewiesen wird, das spezifisch an das Protein bindet,
vorzugsweise wobei das Reagens ausgewählt ist aus der Gruppe bestehend aus einem Antikörper, einem Antikörperderivat und einem Antikörperfragment.

9. Verfahren oder Assay nach einem der Ansprüche 1 bis 7, wobei menschliches Ang2 durch Nachweis des Vorhandenseins eines transkribierten Polynukleotids oder eines Teils davon in der Probandenprobe bestimmt wird,
vorzugsweise wobei das transkribierte Polynukleotid eine mRNA oder eine cDNA ist, oder/und wobei der Schritt des Nachweises ferner die Amplifikation des transkribierten Polynukleotids umfasst, oder/und
wobei das transkribierte Polynukleotid durch Identifizierung einer Nukleinsäure, die sich an der Biomarker-Nukleinsäure oder einem Teil davon unter stringenten Hybridisierungsbedingungen anlagert, nachgewiesen wird, oder/und
wobei menschliches Ang-2 ein Fragment davon ist, oder/und
wobei die Anti-Immun-Checkpoint-Therapie mindestens einen Antikörper umfasst, der ausgewählt ist aus der Gruppe bestehend aus Anti-CTLA-4-Antikörpern, Anti-PD-1-Antikörpern, Anti-PD-L1-Antikörpern, Anti-PD-L2-Antikörpern und Kombinationen davon, vorzugsweise wobei die Anti-Immun-Checkpoint-Therapie Ipilimumab umfasst.

10. Verfahren oder Assay nach einem der Ansprüche 1 bis 9, wobei das menschliche Ang-2-Protein die Aminosäuresequenz der SEQ ID NO: 2, SEQ ID NO: 4 oder SEQ ID NO: 6 umfasst.

11. Verfahren oder Assay nach einem der Ansprüche 1 bis 10, wobei der Krebs ein solider Tumor ist.

12. Verfahren oder Assay nach Anspruch 11, wobei der Tumor ein Melanom, nichtkleinzelliger Lungenkrebs (SCLC) oder Nierenzellkrebs ist.

13. Verfahren oder Assay nach Anspruch 12, wobei das Melanom ein metastasierendes Melanom ist.

14. Verfahren oder Assay nach einem der Ansprüche 1 bis 13, wobei die Anti-Immun-Checkpoint-Therapie ferner eine auf VEGF abzielende Therapie umfasst.

15. Verfahren oder Assay nach einem der Ansprüche 1 bis 14, wobei der Proband ein Säugetier oder ein Tiermodell für Krebs ist, vorzugsweise wobei das Säugetier ein Mensch ist.

## Revendications

1. Procédé d'identification de la probabilité qu'un cancer chez un sujet réponde à une thérapie de point de contrôle anti-immunitaire, dans lequel la thérapie de point de contrôle anti-immunitaire comprend une thérapie ciblant CTLA-4 ou PD-1, le procédé comprenant les étapes de:
a) utiliser un échantillon sujet provenant d'un patient atteint d'un cancer;
b) mesurer la quantité ou l'activité de l'Ang2 humain dans l'échantillon sujet; et
c) comparer ladite quantité ou activité de l'Ang2 humain dans un échantillon de contrôle, une quantité ou activité significativement augmentée de l'Ang2 humain dans l'échantillon sujet par rapport à l'échantillon de contrôle identifiant le cancer comme étant moins susceptible de répondre à la thérapie de point de contrôle anti-immunitaire et une quantité ou activité diminuée de l'Ang2 humain dans l'échantillon sujet par rapport à l'échantillon de contrôle identifiant le cancer comme étant plus susceptible de répondre à la thérapie de point de contrôle anti-immunitaire.

2. Procédé d'identification de la probabilité qu'un cancer chez un sujet réponde à une thérapie de point de contrôle anti-immunitaire, dans lequel la thérapie de point de contrôle anti-immunitaire comprend une thérapie ciblant CTLA-4 ou PD-1, le procédé comprenant les étapes de:
a) utiliser un échantillon sujet provenant d'un patient atteint d'un cancer, dans lequel l'échantillon sujet comprend des molécules d'acide nucléique du sujet;
b) déterminer le nombre de copies de l'Ang2 humain dans l'échantillon; et
c) comparer ledit nombre de copies à celui d'un échantillon de contrôle, un nombre de copies augmenté de l'Ang2 humain dans l'échantillon sujet par rapport à l'échantillon de contrôle identifiant le cancer comme étant moins susceptible de répondre à la thérapie de point de contrôle anti-immunitaire et un nombre de copies diminué de l'Ang2 humain dans l'échantillon sujet par rapport à l'échantillon de contrôle identifiant le cancer comme étant plus susceptible de répondre à la thérapie de point de contrôle anti-immunitaire.

3. Procédé selon la revendication 1 ou 2, comprenant en outre la recommandation, la prescription ou l'administration d'une thérapie de point de contrôle anti-immunitaire si le cancer est jugé susceptible de répondre à la thérapie de point de contrôle anti-immunitaire ou l'administration d'une thérapie anticancéreuse autre que la thérapie de point de contrôle anti-immunitaire si le cancer est jugé moins susceptible de répondre à la thérapie de point de contrôle anti-immunitaire,
de préférence dans lequel la thérapie anticancéreuse est choisie dans le groupe constitué de la thérapie ciblée, de la chimiothérapie, de la radiothérapie, et/ou de l'hormonothérapie.

4. Procédé selon l'une quelconque des revendications 1 à 3, dans lequel l'échantillon de contrôle est déterminé à partir d'un échantillon non cancéreux provenant du sujet ou d'un membre de la même espèce à laquelle appartient le patient, ou
de préférence dans lequel l'échantillon de contrôle est un échantillon cancéreux ou non cancéreux du patient obtenu à un moment antérieur à celui de l'échantillon sujet, éventuellement dans lequel l'échantillon de contrôle est obtenu avant que le patient ne reçoive une thérapie de point de contrôle anti-immunitaire et l'échantillon sujet est obtenu après que le patient a reçu une thérapie de point de contrôle anti-immunitaire, ou/et dans lequel l'échantillon de contrôle comprend des cellules ou ne comprend pas de cellules, ou/et
dans lequel l'échantillon de contrôle comprend des cellules cancéreuses dont on sait qu'elles répondent ou ne répondent pas à la thérapie de point de contrôle anti-immunitaire.

5. Procédé d'évaluation de l'efficacité d'un agent de point de contrôle anti-immunitaire pour traiter un cancer chez un sujet qui n'est pas susceptible de répondre à une thérapie de point de contrôle anti-immunitaire, dans lequel la thérapie de point de contrôle anti-immunitaire comprend une thérapie ciblant CTLA-4 ou PD-1, le procédé comprenant les étapes de:
a) détecter, dans un premier échantillon sujet maintenu en présence de l'agent, la quantité ou l'activité de l'Ang2 humain;
b) détecter la quantité ou l'activité de l'Ang2 humain dans un second échantillon sujet et maintenu en l'absence de l'agent; et
c) comparer la quantité ou l'activité de l'Ang2 humain des étapes a) et b), une quantité ou activité significativement augmentée de l'Ang2 humain dans le premier échantillon sujet par rapport à au moins un échantillon sujet ultérieur indiquant que l'agent traite le cancer chez le sujet.

6. Procédé *in vitro* ou *ex vivo* d'évaluation de l'efficacité d'un agent de point de contrôle anti-immunitaire pour traiter un cancer chez un sujet ou pronostiquer la progression d'un cancer chez un sujet, dans lequel la thérapie de point de contrôle anti-immunitaire comprend une thérapie ciblant CTLA-4 ou PD-1, le procédé comprenant les étapes de:
a) détecter dans un échantillon sujet à un premier moment la quantité ou l'activité d'Ang2 humain;
b) répéter l'étape a) pendant au moins un moment ultérieur après l'administration de l'agent; et
c) comparer l'expression et/ou l'activité détectées aux étapes a) et b), une quantité ou activité significativement augmentée de l'Ang2 humain dans le premier échantillon sujet par rapport à au moins un échantillon sujet ultérieur indiquant que le cancer est peu susceptible d'évoluer ou que l'agent traite le cancer chez le sujet.
de préférence dans lequel, entre le premier moment et le moment ultérieur, le sujet a subi un traitement, a terminé le traitement et/ou est en rémission du cancer, ou
dans lequel le premier échantillon sujet et/ou au moins un échantillon sujet ultérieur ont été obtenus à partir d'un modèle animal du cancer, ou
dans lequel le premier échantillon sujet et/ou au moins un échantillon sujet ultérieur est une partie d'un échantillon unique du sujet ou d'échantillons groupés obtenus à partir du sujet.

7. Procédé ou essai selon l'une quelconque des revendications 1 à 6, dans lequel l'échantillon sujet et/ou l'échantillon de contrôle n'ont pas été mis en contact avec un traitement de mélanome ou un inhibiteur d'un point de contrôle immunitaire, ou/et dans lequel le sujet n'a pas été mis en contact avec un traitement de mélanome ou un inhibiteur d'un point de contrôle immunitaire, ou/et
comprenant en outre la recommandation, la prescription ou l'administration d'au moins un agent thérapeutique anticancéreux supplémentaire, éventuellement dans lequel l'au moins un agent thérapeutique anticancéreux supplémentaire est le bevacizumab et/ou un agent thérapeutique anti-Ang-2, ou/et
l'échantillon sujet est choisi dans le groupe constitué par le sérum, le sang total, le plasma, l'urine, les cellules, les lignées cellulaires et les biopsies.

8. Procédé ou essai selon l'une quelconque des revendications 1 à 7, dans lequel la quantité d'Ang2 humain est détectée à l'aide d'un réactif qui se lie spécifiquement à la protéine,
de préférence dans lequel le réactif est choisi dans le groupe constitué par un anticorps, un dérivé d'anticorps et un fragment d'anticorps.

9. Procédé ou essai selon l'une quelconque des revendications 1 à 7, dans lequel l'Ang2 humain est évalué en détectant la présence dans l'échantillon sujet d'un polynucléotide transcrit ou d'une partie de ce polynucléotide,
de préférence dans lequel le polynucléotide transcrit est un ARNm ou un ADNc, ou/et dans lequel l'étape de détection comprend en outre l'amplification du
polynucléotide transcrit, ou/et
dans lequel le polynucléotide transcrit est détecté par l'identification d'un acide nucléique qui s'ancre avec l'acide nucléique du biomarqueur, ou une partie de celui-ci, dans des conditions d'hybridation rigoureuses, ou/et
dans lequel l'Ang-2 humain est un fragment de celui-ci, ou/et
dans lequel la thérapie de point de contrôle anti-immunitaire comprend au moins un anticorps choisi dans le groupe constitué par les anticorps anti-CTLA-4, les anticorps anti-PD-1, les anticorps anti-PD-L1, les anticorps anti-PD-L2, et des combinaisons de ceux-ci,
de préférence dans lequel la thérapie de point de contrôle anti-immunitaire comprend l'ipilimumab.

10. Procédé ou essai selon l'une quelconque des revendications 1 à 9, dans lequel la protéine Ang-2 humaine comprend la séquence d'acides aminés de SEQ ID NO : 2, SEQ ID NO:4, ou SEQ ID NO:6.

11. Procédé ou essai selon l'une quelconque des revendications 1 à 10, dans lequel le cancer est une tumeur solide.

12. Procédé ou essai selon la revendication 11, dans lequel la tumeur est un mélanome, un cancer du poumon non à petites cellules (SCLC) ou un cancer des cellules rénales.

13. Procédé ou essai selon la revendication 12, dans lequel le mélanome est un mélanome métastatique.

14. Procédé ou essai selon l'une quelconque des revendications 1 à 13, dans lequel la thérapie de point de contrôle anti-immunitaire comprend en outre une thérapie ciblant le VEGF.

15. Procédé ou essai selon l'une quelconque des revendications 1 à 14, dans lequel le sujet est un mammifère ou un modèle animal de cancer, de préférence dans lequel le mammifère est un humain.
